(19) **Europäisches Patentamt / European Patent Office / Office européen des brevets**

(11) **EP 4 752 754 A1**

(12) **EUROPEAN PATENT APPLICATION**

(43) Date of publication:
03.06.2026 Bulletin 2026/23

(21) Application number: 25218816.4

(22) Date of filing: 26.11.2025

(51) International Patent Classification (IPC):
*G06F 16/906* (2019.01)

(52) Cooperative Patent Classification (CPC):
**G06F 16/906**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: 27.11.2024 US 202463725998 P

(71) Applicant: **Tempus AI, Inc.**
**Chicago, IL 60654-2282 (US)**

(72) Inventors:
• **Fields, Paul**
**Chicago / IL, 60654 (US)**

• **Franch Exposito, Sebastia**
**Chicago / IL, 60654 (US)**
• **Jain, Prerna**
**Chicago / IL, 60654 (US)**
• **Macera, Lisa Sandra**
**Chicago / IL, 60654 (US)**
• **Pojman, Nicholas**
**Chicago / IL, 60654 (US)**
• **Scherrer, Emilie**
**Chicago / IL, 60654 (US)**

(74) Representative: **Grund, Martin**
**Grund Intellectual Property Group**
**Patentanwälte und Solicitor PartG mbB**
**Steinsdorfstraße 2**
**80538 München (DE)**

(54) **SYSTEMS AND METHODS FOR MACHINE-LEARNING-BASED EMBEDDING AND
CLUSTERING OF DOCUMENTS WITH SUBSEQUENT ACTION RANKING AND NOTIFICATION**

(57) This application describes, amongst other things, example methods and systems using machine-learning components having knowledge of a plurality of numerical embeddings corresponding to a plurality of documents, where the plurality of numerical embeddings relates to a set of similarity clusters. An example method includes obtaining a constraint indicating a period of time and a type of subject matter and, using the constraint, obtaining a set of documents and corresponding meta-data. A set of numerical embeddings are generated for the set of documents. A set of updated similarity clusters are generated by updating the set of similarity clusters to incorporate the set of numerical embeddings. A set of potential actions are identified based on links between entity information and the set of updated similarity clusters. The set of potential actions are ranked, and a notification is provided indicating top-ranked actions.

Platform
100

FIG. 1

EP 4 752 754 A1

**Description**

**PRIORITY AND RELATED APPLICATIONS**

This application claims priority to U.S. Provisional Patent Application No. 63/725,998 filed November 27, 2024, which is hereby incorporated by reference in its entirety.

**TECHNICAL FIELD**

This application relates generally to employing artificial intelligence and machine learning, including but not limited to systems and methods for embedding documents and relevant metadata to identify and initiate entity actions.

**BACKGROUND**

Biomedical research and clinical decision-making are supported by a variety of document sources, such as conference abstracts, peer-reviewed journal articles, clinical trial protocols, real-world data reports, molecular profiles, imaging annotations, patient registries, and electronic health records. These sources are collected, processed, and interpreted across multidisciplinary teams and data pipelines. Due to the heterogeneity and scale of these sources, coupled with the need to extract salient themes (such as emerging biomarkers, therapeutic targets, and patient cohorts) researchers and clinicians face significant burdens. Manual aggregation and review of hundreds or thousands of documents can require extensive human resources and protracted timelines. Basic data management systems and computational tools may assist with text parsing and keyword searches; however, they lack the depth required to uncover latent thematic structures. As a result, the research community continues to seek more efficient approaches for transforming raw text and metadata into analysis-ready insights. Accordingly, there remains a need for streamlined, scalable systems that can embed diverse documents, dynamically cluster related content, rank follow-up actions, and deliver personalized notifications to accelerate biomedical research workflows.

**SUMMARY**

Existing digital research assistants and platforms offer capabilities such as document retrieval, summarization, and basic query handling, yet they operate in isolation from one another and depend on extensive user configuration and interaction. Many platforms target single modalities, such as text, without integrating multimodal information like molecular profiles, imaging annotations, and real-world outcomes. Moreover, processing techniques provided by conventional analytics tools are often limited to static, one-time batch processing, inhibiting timely identification of evolving research trends. Additionally, conventional systems typically depend on keyword matching or term-frequency heuristics that miss semantically equivalent concepts, synonyms, and context-dependent meanings, leading to low recall and noisy results. Their clustering approaches are likewise constrained, often using fixed vocabularies and static topic models that fail to capture latent relationships across modalities, temporal evolution within a corpus, or the influence of metadata such as study design, biomarkers, or sponsor. As a result, superficially similar documents are grouped together while clinically or scientifically related materials expressed with different terminology remain fragmented and overlooked, limiting downstream actionability and obscuring emerging themes that would otherwise be detectable with embedding-based, multi-modal, and continuously updating pipelines.

With conventional systems, users are often required to manually prioritize potential next steps and often lack tailored action recommendations that align with their professional interests and institutional focus. When large corpora are ingested, conventional pipelines can surface thousands of putative "next actions." Because these candidates are generated without robust user modeling or linkage to an institution's capabilities, most are tangential to the user's role, therapeutic focus, data access, or compliance constraints. In practice, this deluge forces users to triage lengthy, undifferentiated action lists, masking the few high-value items under a litany of low-relevance suggestions. Absent personalization signals, such as active projects, preferred modalities, available datasets, approved workflows, or strategic priorities, these actions cannot be effectively ranked or filtered, resulting in alert fatigue, missed opportunities, and substantial time lost to manual curation.

Notifications and alerts are typically generic, requiring users to sift through irrelevant updates to identify items of importance. Conventional notification systems embody an inefficient man-machine interface that burdens users with irrelevant and unimportant notifications, context switching, and manual reconciliation.

In contrast, as discussed in greater detail below, some of the disclosed systems employ embedding-based retrieval and multimodal fusion to represent heterogeneous inputs (such as unstructured text, imaging annotations, molecular profiles, tabular outcomes, trial results, test results, and associated metadata) in a shared embedding space, enabling similarity matching across modalities and languages without reliance on keyword rules. Similarity structures may be maintained in continuously updated cluster graphs that support temporal modeling, stream ingestion, and online learning to reflect evolving research themes and emergent biomarkers. Clustering may be metadata-aware, incorporating study design, sponsor, cohort attributes, and assay type as conditioning signals, and using adaptive algorithms that split/merge topics as new evidence arrives, rather than relying on static bag-of-words or fixed topic models.

Personalization may be achieved through explicit entity modeling that encodes active projects, therapeutic focus, approved workflows, available datasets, compliance constraints, and role-based data. Candidate actions may be scored with multiple criteria, e.g., that account for institutional capabilities, data availability, estimated effort and cost, regulatory feasibility, and expected impact and return. The candidate actions may be further refined by reinforcement or active learning from user feedback. The disclosed systems may support multilingual ingestion and ontology mapping to normalize synonyms and cross-vendor terminology, integrate cohort and clinical trial matchers to contextualize clusters against real-world patient populations and ongoing studies, and/or provide explainable rationales for recommended actions. Together, these techniques reduce noise, improve recall and precision, enable timely detection of trend shifts, and deliver high-value, user-relevant action recommendations with auditable lineage and privacy-aware controls.

Collectively, the techniques described herein materially lower cognitive burden by curating a smaller, higher-signal set of items that are aligned to an entity's active projects and interests. The man-machine interface can be improved through stateful, bidirectional interactions that capture preferences and feedback and adaptive triage that ranks actions by impact, effort , result, and dependency. Accordingly, users spend less time parsing and reconciling information across systems, experience fewer context switches, and can advance from insight to action within a single, guided workflow thereby improving accuracy and efficiency in the process and interface.

In accordance with some embodiments, a method includes obtaining, at a machine-learning component, a constraint indicating a period of time and a type of subject matter. The machine-learning component has knowledge of a plurality of numerical embeddings corresponding to a plurality of documents, and the plurality of numerical embeddings relates to a set of similarity clusters. The method also includes obtaining, using the constraint, a set of documents and corresponding metadata, where at least a subset of the set of documents is not included in the plurality of documents. The method further includes generating a set of numerical embeddings for the set of documents based on information contained within the set of documents and the corresponding metadata, and generating a set of updated similarity clusters by updating the set of similarity clusters to incorporate the set of numerical embeddings. The method also includes obtaining, for an entity, entity information comprising a set of one or more workflows and one or more datasets and identifying a set of potential actions based on links between the entity information and the set of updated similarity clusters. The method further includes generating a ranked set of potential actions by ranking the set of potential actions according to one or more criteria, and providing a notification to the entity indicating a set of top-ranked potential actions from the ranked set of potential actions.

In accordance with some embodiments, a computing system is provided, such as a cloud computing system, a server system, a personal computer system, and/or other type of electronic device. The computing system includes control circuitry and memory storing one or more sets of instructions. The one or more sets of instructions include instructions for performing any of the methods described herein. In accordance with some embodiments, a non-transitory computer-readable storage medium is provided. The non-transitory computer-readable storage medium stores one or more sets of instructions for execution by a computing system. The one or more sets of instructions include instructions for performing any of the methods described herein.

Thus, devices and systems are disclosed with methods for machine-learning-based embedding and clustering of documents with subsequent action ranking and notification. Such methods, devices, and systems may complement or replace conventional methods, devices, and systems for machine-learning-based embedding and clustering of documents with subsequent action ranking and notification.

The features and advantages described in the specification are not necessarily all inclusive and, in particular, some additional features and advantages will be apparent to one of ordinary skill in the art in view of the drawings, specification, and claims provided in this disclosure. Moreover, it should be noted that the language used in the specification has been principally selected for readability and instructional purposes and has not necessarily been selected to delineate or circumscribe the subject matter described herein.

## BRIEF DESCRIPTION OF THE DRAWINGS

So that the present disclosure can be understood in greater detail, a more particular description can be had by reference to the features of various embodiments, some of which are illustrated in the appended drawings. The appended drawings, however, merely illustrate pertinent features of the present disclosure and are therefore not necessarily to be considered limiting, for the description can admit to other effective features as the person of skill in this art will appreciate upon reading this disclosure.

Figure 1 is a block diagram illustrating an example platform in accordance with some embodiments.

Figures 2A and 2B are block diagrams illustrating an example client device in accordance with some embodiments.

Figure 3 is a block diagram illustrating an example server system in accordance with some embodiments.

Figure 4 is a block diagram illustrating example databases in accordance with some embodiments.

Figures 5A and 5B illustrate example processes for data importation and vectorization in accordance with some embodiments.

Figure 6 illustrates an example architecture for deploying agents in accordance with some embodiments.

Figures 7A and 7B illustrate example user interfaces for interacting with a digital assistant in accordance with some embodiments.

Figure 8A is a flow diagram illustrating an example method for generating draft publications in accordance with some embodiments.

Figure 8B is a flow diagram illustrating an example method for identifying patient cohorts in accordance with some embodiments.

Figure 8C is a flow diagram illustrating an example method for identifying research gaps in accordance with some embodiments.

Figure 8D is a flow diagram illustrating an example method for generating draft publications in accordance with some embodiments.

Figure 8E is a flow diagram illustrating an example method for generating ranked sets of potential actions in accordance with some embodiments.

Figures 9A and 9B illustrate example document visualizations in accordance with some embodiments.

In accordance with common practice, the various features illustrated in the drawings are not necessarily drawn to scale, and like reference numerals can be used to denote like features throughout the specification and figures.

## DETAILED DESCRIPTION

The present disclosure describes, among other things, an embedding-driven pipeline that ingests heterogeneous research documents/files, embeds them into a shared vector space, maintains metadata-aware, temporally adaptive similarity clusters, and ranks downstream "next actions" using multi-criteria scoring aligned to an entity's capabilities, datasets, and constraints. By replacing manual review, keyword retrieval, and/or static topic models with embeddings, normalization, similarity clustering, and reinforcement from user feedback, the system can materially reduce noise, improve recall and precision across modalities, detect emergent trends in near real time, and surface a compact, high-signal set of actionable recommendations with auditable provenance. This system lowers the cognitive burden on users (e.g., via stateful, bidirectional interactions and in-context execution).

In some embodiments, a platform (a computing system) includes one or more processors, memory, and programs executable to implement the foregoing pipeline, including components for one or more of: (i) ingestion (and de-identification) of (multimodal) inputs; (ii) chunking and embedding generation; (iii) a vector index and/or cluster graph that supports updates, split/merge operations, and metadata conditioning; (iv) an ontology and synonym mapper; (v) retrieval-augmented generation with provenance tracking and guardrails; (vi) an entity-modeling subsystem that encodes workflows, datasets, capabilities, and access controls; (vii) an action generator that links clusters to entity resources; and (viii) a ranking engine that scores candidate actions by criteria such as impact, feasibility, effort, return, and cost. A notification and interaction layer may be included to provide notifications. The interaction layer may be configured to provide stateful, in-context, and auditable user experiences, capture feedback to refine future rankings, and/or expose APIs for integration with external trial registries, cohort builders, and EHR systems. When executed on the platform, these modules can cooperatively perform continuous ingestion, clustering, action identification, multi-criteria ranking, and delivery of personalized, executable recommendations.

In some embodiments, the platform acts as an operating system for implementing task-specific orchestrations for performing specific tasks. The platform may include one or more of the following example components. For example, a genetic sequencing component with downstream molecular bioinformatics may operate to call out relevant biomarkers in DNA, RNA, or their derivatives for a specimen (e.g., a tumor biopsy) that is sequenced and reported back to an ordering physician. As another example, a pathology imaging component may operate on cellular and/or slide level images to identify relevant biomarkers from cells within an imaged specimen. As another example, a radiological imaging component may operate on larger images of the body through various radiology imaging technologies to identify the presence or longitudinal progression of tumors. Other examples include identifying various disease states using cardiology, neurology, and/or endocrinology imaging components. Each of these components may include, or communicate with, a corresponding agent to identify and/or report information relevant to a user query or request.

As an example, an agent may be configured by a user using a user interface (e.g., a console of a web or desktop application) and deployed to various environments (e.g., a research environment, an alpha environment, a beta environment, a client environment, and/or a production environment). Each environment may be linked to different sources, have different permissions, and/or have different authorized users. In some embodiments, precision medicine principles are employed in customizing the user interfaces, such as modifications based on a set of subjects (e.g., patients) associated with the user of the application. For example, the user (or an immediate family member of the user) may be one of the subjects. An environment may be defined by access to data sources and/or users. The agent configuration may be stored in a control plane. The control plane may be configured to control how data is managed, routed, and/or processed. The agents themselves may execute in the appropriate workload planes (e.g., data planes), and the workload planes may not have access to the control plane. The control plane may supervise/direct each workload plane, while the workload planes are configured to manipulate and/or transport data.

In this example, the agent builder in the control plane is configured to push configurations into the various environments. For example, this synchronization may be fast enough that a user can configure an agent and immediately evaluate the configuration in the interactive console in a working environment. An example architecture includes two components: an agent builder in a control plane that hosts the user interface (UI) for configuring agents, and an agent host in a workload plane that hosts the UI and API for interacting with deployed agents. When an agent configuration is changed or an agent version is deployed, the agent builder may inform the agent host in each environment so that the updated agent can be deployed. For example, this may be via a pubsub message to the agent-config topic or via a simple HTTP request. In some embodiments, the agent builder utilizes a cognitive architecture that includes memory modules and action spaces. For example, the cognitive architecture organizes agents along three dimensions: their information storage (e.g., divided into working and long-term memories); their action space (e.g., divided into internal and external actions); and their decision-making procedure (e.g., structured as an interactive loop with planning and execution).

As another example, after deployment, an agent may receive a user query (e.g., requesting information about clinical trials), generate a structured application programming interface (API) call, use the generated API call to query a remote server to retrieve a relevant result, and reformat the relevant information to return to the user. In some embodiments, each action is performed by a different agent builder block component (also sometimes referred to as a builder block, block, or node). In some embodiments, the agent is configured for multiple types of tasks. In these embodiments, the agent may identify the intent of a user's query (e.g., to search for clinical trials or identify adverse events) and respond accordingly. In some embodiments, the agent is configured for only one type of task (e.g., is a task-specific agent). In some of these embodiments, the agent does not identify an intent of the user (e.g., the agent may assume the intent). In some embodiments, the agent receives the intent from a different component or system. The agent may also interface with other agents to obtain additional information for the user query (such as patient records or relevant guidelines). In some embodiments, the agent includes a pretrained language model (e.g., trained on a particular domain and/or using particular databases). In some embodiments, the agent queries an unstructured database (e.g., in addition, or alternatively, to generating the API call).

In some embodiments, an end-to-end (multimodal) model is trained to perform the foregoing operations across modalities and tasks without delegating to separate components or agents. In some embodiments, the model ingests heterogeneous inputs (e.g., text, molecular profiles, imaging data, tabular outcomes, and associated metadata), performs representation learning via shared cross-modal encoders with modality-specific adapters, generates embeddings in a common latent space, and executes temporally aware clustering through an internal memory and attention mechanism (e.g., that supports streaming similarity updates). For example, the same model may implement ontology normalization, retrieval-augmented reasoning, action generation, and ranking using a multi-objective head (e.g., optimized for impact, feasibility, effort, cost, and/or constraints under a joint loss).

The platform, or components thereof, may be used in conjunction with any medical field (e.g., to assist physicians in the treatment of any associated disease state therein), such as on oncology, endocrinology (e.g., diabetes), neurology, mental health (e.g., depression and related pharmacogenetics), and cardiovascular disease. For example, the platform may also include a cardiology-based component (e.g., comprising one or more agents) that operates on electrocardiogram (ECG) data to identify patients having an elevated risk for cardiovascular disease. As another example, the platform may include a data curation component (e.g., comprising one or more agents) that obtains raw (e.g., unstructured) data and structures it into a common and useful format as a repository (e.g., a multimodal database) of clinical data from which other bioinformatics, analytics, agents, models, and/or components may operate. As another example, the platform may be configured to search within the clinical data to identify cohorts of related patients and/or generate insights and/or analytics. As another example, the platform may be configured to monitor an electronic health record (EHR) to identify care gaps and/or reminders to physicians to act with a respective patient. In this way, the platform may serve as a docket manager that identifies issues/events the corresponding physicians did not manually docket, e.g., to ensure patients and other subjects get timely care. The platform may also be configured to track and/or catalog relevant therapies (e.g., on label and/or off label use) for a set of disease states. The platform may also track and/or catalog relevant clinical trials (e.g., in multiple countries and/or from multiple authorities) for a set of disease states. In some embodiments, the platform is configured to interact with patients/subjects directly.

As discussed below, the platform may include an AI-enabled assistive user interface (which may sometimes be described herein as a clinical assistant or digital assistant) that provides access to patient and research insights. The AI-enabled assistive user interface may use one or more task-specific orchestrations that each include language models, an end-to-end mode, and/or other types of machine learning.

In some embodiments, the platform includes a hub component that allows physicians to order, track, and view test results, and export patient data. In some embodiments the hub component provides insights into research focus, cohort building, genomic alterations, treatment implications, as well as clinical trial matching. The hub component may be used in conjunction with the AI-enabled clinical assistant to allow physicians, experts, researchers, and other types of users to interact using conversational language including natural language inputs, follow-up questions, and remarks. The platform may also include a peer-to-peer messaging component for physicians, experts, and other types of users to share knowledge, insight, and/or perspective on medical fields such as molecular oncology (e.g., as it pertains to patient care). The messaging component may be used in conjunction with the AI-enabled clinical assistant to engage in, and optionally learn from, the conversations on the messaging component. For example, the AI-enabled clinical assistant may be invoked in conversation to provide insights and/or data for a particular topic or conversation. The platform may also include an EHR interface component (e.g., comprising one or more agents) configured to allow physicians, and optionally other qualified users, to view, edit, and/search an EHR. The EHR interface component may be communicatively coupled with one or more services and/or databases to obtain updated information and reports (e.g., via push notifications). The EHR interface component may be used in conjunction with the AI-enabled clinical assistant to search, edit, summarize, tag, and/or reform an EHR. The platform may also include a research analytical component (e.g., comprising one or more agents) that provides de-identified patient/clinical data and insights. For example, the platform may provide insights derived from providing available data and/or newly-ingested data to a machine-learning model (e.g., the insights are output by the model in response to providing the data).

In some embodiments, the platform further includes data connectors that interface with third-party databases and services to ingest multi-modal, largely unstructured information (such as conference abstracts and PubMed articles, clinical trial registries, payer claims repositories, lab and genomics portals, imaging archives (e.g., PACS), device telemetry, and pharmacovigilance feeds) via authenticated APIs and/or secure file transfer. The ingested materials may be normalized through schema harmonization and ontology mapping, de-identified where appropriate, and partitioned into modality-specific chunks that are processed by cross-modal encoders with modality adapters to generate numerical embeddings in a shared latent space. These embeddings capture semantics across text, imaging annotations, molecular profiles, tabular outcomes, and conversation logs, and may be inserted into a vector index with role-based access controls, provenance tags, and time stamps to support similarity search, temporally aware clustering, and streaming updates. By projecting heterogeneous, cross-vendor data into a common embedding space, the hub component and clinical assistant can perform retrieval, reasoning, cohort matching, and trial linking across modalities and sources, yielding unified insights even when upstream data formats and taxonomies differ.

Reference will now be made to embodiments, examples of which are illustrated in the accompanying drawings. In the following description, numerous specific details are set forth in order to provide an understanding of the various described embodiments. However, it will be apparent to one of ordinary skill in the art that the various described embodiments may be practiced without these specific details. In other instances, well-known methods, procedures, components, circuits, and networks have not been described in detail so as not to unnecessarily obscure aspects of the embodiments.

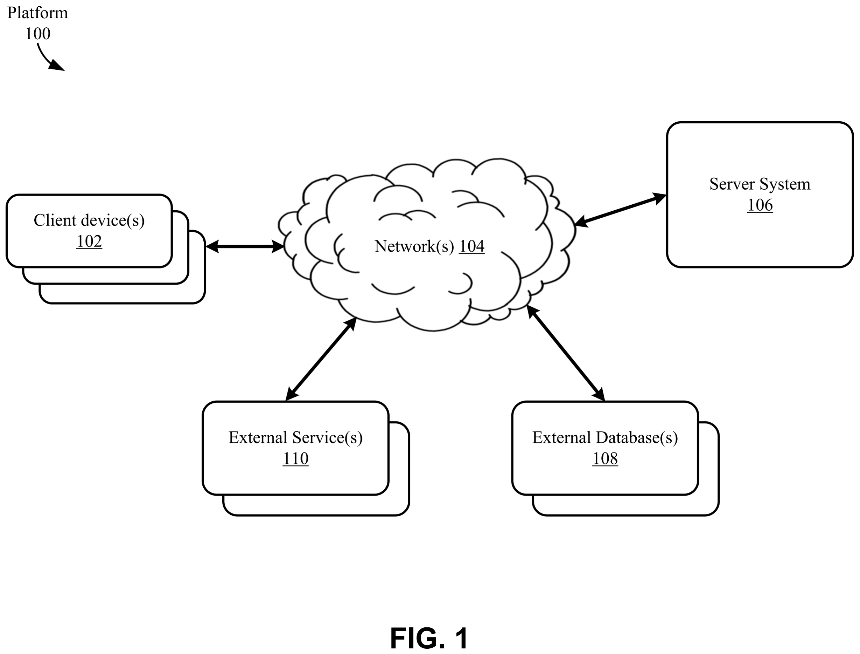

Figure 1 is a block diagram illustrating a platform 100 in accordance with some embodiments. In some embodiments, the platform 100 is an AI platform (e.g., the AI platform discussed previously). The platform 100 includes one or more client devices 102 communicatively coupled to a server system 106 via one or more networks 104. In accordance with some embodiments, the platform 100 further includes, or communicates with, one or more external services 110 and one or more external databases 108. In some embodiments, the one or more networks 104 include public communication networks, private communication networks, or a combination of both public and private communication networks. For example, the one or more networks 104 can be any network (or combination of networks) such as the Internet, other wide area networks (WAN), local area networks (LAN), virtual private networks (VPN), metropolitan area networks (MAN), peer-to-peer networks, and/or ad-hoc connections. In some embodiments, the platform 100 includes only a subset of the components shown in Figure 1. For example, the platform 100 may include only one of: a client device 102 or a server system 106.

In some embodiments, a client device 102 is associated with one or more users. In some embodiments, each user is separately authenticated (e.g., assigned distinct/unique authentication tokens). In some embodiments, a client device 102 is a personal computer, mobile electronic device, wearable computing device, laptop computer, tablet computer, mobile phone, feature phone, smart phone, a speaker, television (TV), and/or any other electronic device capable of interacting with a user (e.g., an electronic device having an I/O interface). The client device(s) 102 may communicatively couple to other components of the platform 100 wirelessly and/or through a wired connection (e.g., directly through an interface, such as an HDMI interface).

In some embodiments, the client device(s) 102 send and receive information, such as documents, queries, and/or results, through network(s) 104. For example, the client device(s) 102 may send a query or request to the server system 106, the external service(s) 110, and/or the external database(s) 108 through network(s) 104. As another example, the client device(s) 102 may receive results and other responses from the server system 106, the external service(s) 110, and/or the external database(s) 108 through network(s) 104. In some embodiments, two or more client devices 102 communicate with one another (e.g., resending and responding to queries and requests). The two or more client devices 102 may communicate via the network(s) 104 or directly (e.g., via a wired connection or through a peer-to-peer wireless connection).

In some embodiments, the server system 106 includes multiple electronic devices communicatively coupled to one another. In some embodiments, the multiple electronic devices are collocated (e.g., in a datacenter), while in other embodiments, the multiple electronic devices are geographically separated from one another. In some embodiments, the server system 106 stores and provides clinical and/or patient data. In some embodiments, the server system 106 trains, publishes, and/or utilities one or more agents and/or language models. In some embodiments, the server system 106 receives and responds to queries and requests from the client device(s) 102 using the one or more agents and/or language models. In some embodiments, the server system 106 includes multiple nodes and/or clusters configured to manage different types of tasks and/or handle requests and queries from different geographical locations.

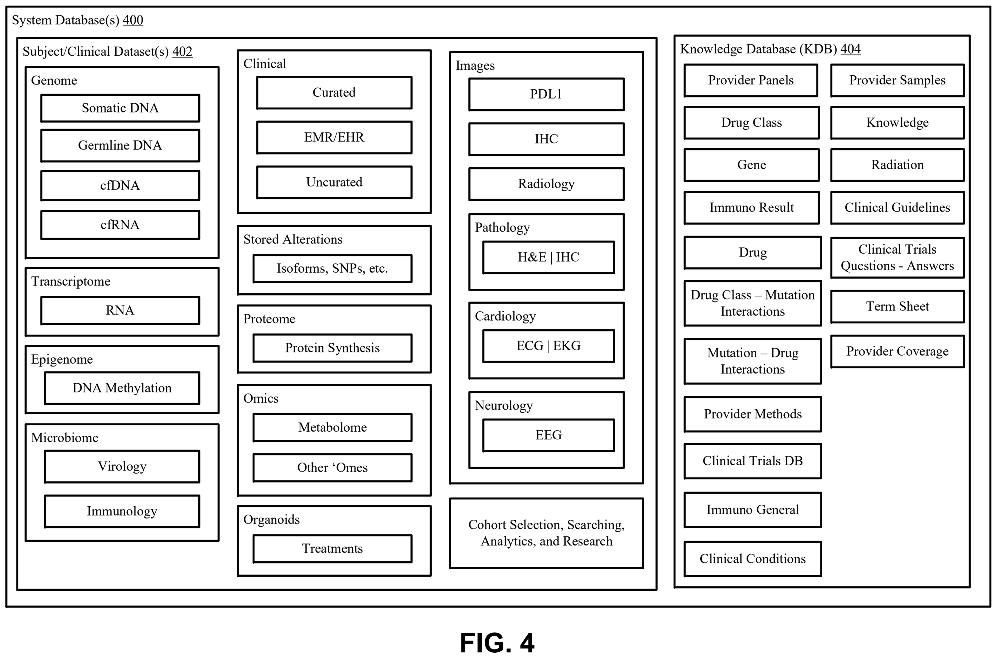

In some embodiments, the client device(s) 102 and/or the server system 106 communicate with the external service(s) 110 and/or the external database(s) 108 via an application programming interface (API). In some embodiments, the external service(s) 110 and/or the external database(s) 108 are maintained/operated by a third party to the platform 100. In some embodiments, the external service(s) 110 include agents, location services, time services, web-enabled services, and/or services that access information stored external to the platform 100. In some embodiments, the external database(s) 108 include one or more medical databases, clinical databases, subject databases, research databases, and/or general knowledge databases. In some embodiments, the external database(s) 108 comprise one or more of the databases shown in Figure 4. In some embodiments, the external database(s) 108 comprise one or more user databases (e.g., patient databases maintained by a third-party user of the platform 100).

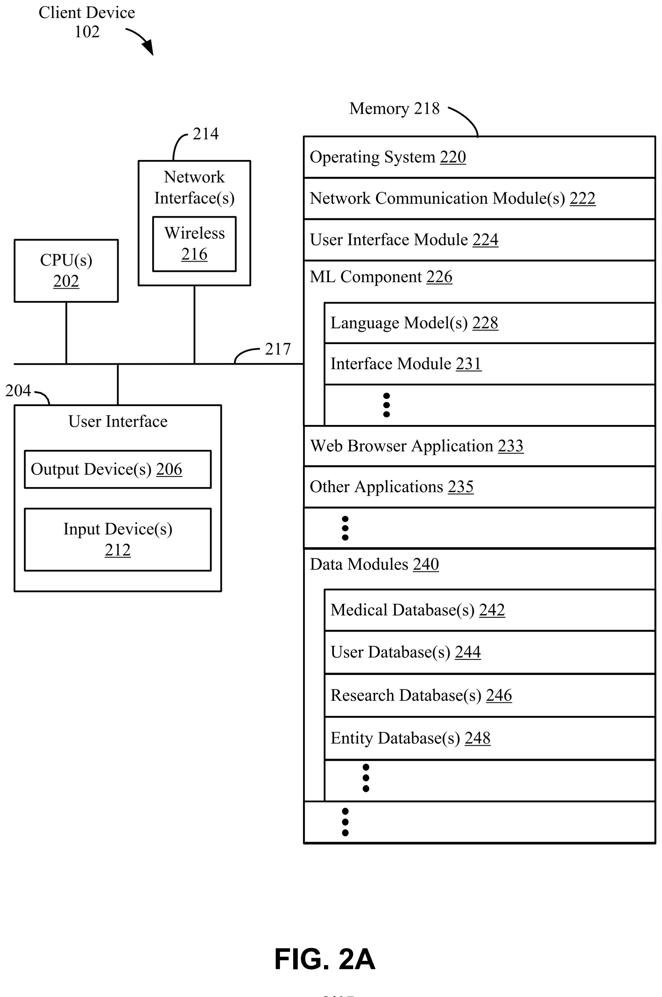

Figure 2A is a block diagram illustrating a client device 102 in accordance with some embodiments. The client device 102 includes one or more central processing units (CPUs) 202, a user interface 204, one or more network (or other communications) interfaces 214, memory 218, and one or more communication buses 217 for interconnecting these components. In some embodiments, the client device 102 includes a processor or other control circuitry (e.g., in addition, or alternatively, to the CPUs 202). For example, the client device 102 may include one or more GPUs and/or DPUs (e.g., for performing machine learning tasks). The communication buses 217 optionally include circuitry (sometimes called a chipset) that interconnects and controls communications between system components. Optionally, the client device 102 includes a location-detection component, such as a global navigation satellite system (GNSS) (e.g., GPS (global positioning system), GLONASS, Galileo, BeiDou) or other geo-location receiver, and/or location-detection software for determining the location of the client device 102.

In some embodiments, the client device 102 includes one or more sensors including, but not limited to, accelerometers, gyroscopes, compasses, magnetometer, light sensors, near field communication transceivers, barometers, humidity sensors, temperature sensors, proximity sensors, range finders, and/or other sensors/devices for sensing and measuring various environmental conditions.

The user interface 204 includes output device(s) 206 and input device(s) 212. In some embodiments, the input device(s) 212 include a keyboard, mouse, a track pad, and/or a touchscreen. In some embodiments, the user interface 204 includes a display device that includes a touch-sensitive surface, in which case the display device is a touch-sensitive display. In client devices that have a touch-sensitive display, a physical keyboard is optional (e.g., a soft keyboard may be displayed when keyboard entry is needed). In some embodiments, the output device(s) 206 include a speaker and/or a connection port for connecting to speakers, earphones, headphones, or other external listening devices. In some embodiments, the input device(s) 212 include a microphone and/or voice recognition device to capture audio (e.g., speech from a user).

In some embodiments, the one or more network interfaces 214 include wireless and/or wired interfaces for receiving data from and/or transmitting data to other client devices 102, the server system 106, and/or other devices or systems. The data communications may be conducted using any of a variety of custom or standard wireless protocols (e.g., NFC, RFID, IEEE 802.15.4, Wi-Fi, ZigBee, 6LoWPAN, Thread, Z-Wave, Bluetooth, ISA100.11a, WirelessHART, MiWi, etc.). Furthermore, the data communications may be conducted using any of a variety of custom or standard wired protocols (e.g., USB, Firewire, Ethernet, etc.). For example, the one or more network interfaces 214 may include a wireless interface 216 for enabling wireless data communications with other client devices 102, systems, and/or other wireless (e.g., Bluetooth-compatible) devices. Furthermore, in some embodiments, the wireless interface 216 (or a different communications interface of the one or more network interfaces 214) enables data communications with other WLAN-compatible devices and/or the server system 106 (via the one or more network(s) 104).

The memory 218 includes high-speed random-access memory, such as DRAM, SRAM, DDR RAM, or other random-access solid-state memory devices; and may include non-volatile memory, such as one or more magnetic disk storage devices, optical disk storage devices, flash memory devices, or other non-volatile solid-state storage devices. The memory 218 optionally includes one or more storage devices remotely located from the CPU(s) 202. The memory 218, or alternately, the non-volatile memory solid-state storage devices within the memory 218, includes a non-transitory computer-readable storage medium. In some embodiments, the memory 218 or the non-transitory computer-readable storage medium of the memory 218 stores the following programs, modules, and data structures, or a subset or superset thereof:

- an operating system 220 that includes procedures for handling various basic system services and for performing hardware-dependent tasks;

- network communication module(s) 222 for connecting the client device 102 to other computing devices connected to one or more network(s) 104 via the one or more network interface(s) 214 (wired or wireless);

- a user interface module 224 that receives commands and/or inputs from a user via the user interface 204 (e.g., from the input device(s) 212) and provides outputs via the user interface 204 (e.g., the output device(s) 206);

- a machine-learning (ML) component 226 that may include a plurality of agent modules 227 (e.g., agent building blocks and/or generated agents). In some embodiments, the ML component 226 works in conjunction with an assistant module at the server system 106 (e.g., the assistant module 316). In some embodiments, the ML component 226 includes the following modules (or sets of instructions), or a subset or superset thereof:

   ◦ one or more models 228 that engage with a user and/or perform specific tasks in furtherance of a user request or query. In some embodiments, the model(s) 228 include one or more large language models, such as GPT-3, GPT-4, BioGPT, and PaLM-2; and

   ◦ an interface module 231 that allows the model(s) 228 communicate with other applications, components, and devices (e.g., via an API or structured query). In some embodiments, the interface module 231 is, or includes, an agent (e.g., a task-specific orchestration), a task-specific orchestration creator application, one or more orchestration libraries (e.g., orchestration marketplaces) for selecting orchestrations for performing tasks as discussed herein;

- a web browser application 233 for accessing, viewing, and interacting with web sites;

- other applications 235, such as applications for word processing, calendaring, mapping, weather, stocks, time keeping, virtual digital assistant, presenting, number crunching (spreadsheets), drawing, instant messaging, e-mail, telephony, video conferencing, photo management, video management, a digital music player, a digital video player, 2D gaming, 3D (e.g., virtual reality) gaming, electronic book reader, and/or workout support; and

- one or more data modules 240 for managing the storage of and/or access to data such as medical data, clinical data, patient data, and user data. In some embodiments, the one or more data modules 240 include:

   ◦ one or more medical databases 242 for storing medical data (e.g., regarding therapies, drugs, treatments, patients, cohorts and/or diseases) and associated metadata;

   ◦ one or more user databases 244 for storing user data such as user preferences, user settings, and other metadata;

   ◦ one or more research databases 246 for storing research and conference data and associated metadata; and

◦ one or more entity databases 248 for storing entity-specific data (e.g., regarding research projects, interests, and products) and associated metadata.

In some embodiments, one or more agent modules 227 are configured to engage with a user in an integrated, conversational manner using natural language dialog, and/or invoke external services when appropriate to obtain information or perform various actions.

In some embodiments, the ML component 226 executes on hardware accelerators optimized for training and inference, including one or more GPUs and/or tensor processing units (TPUs) coupled to high-bandwidth memory and NVMe storage for fast model and embedding retrieval. The accelerators may be interconnected via high-speed fabrics (e.g., PCIe Gen5, NVLink, or InfiniBand) to support distributed inference, parameter sharding, and vector index operations, while collocated CPUs handle orchestration, preprocessing, and secure I/O.

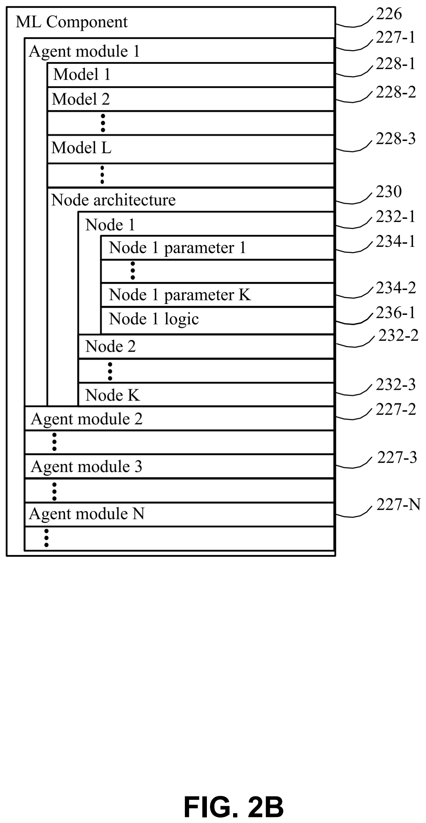

Referring to Figure 2B, in some embodiments, the platform 100 provides a plurality of agent modules 227 and a system for managing and deploying these agent modules 227, such as managing through various blocks (e.g., agent builder blocks) realized in the form of one or more nodes 232. In some embodiments, each respective agent module 227 is associated with a defined domain of information and/or a task-specific capability, which allows for retrieving a particular agent module 227 based on information determined from a prompt provided by a user and/or based on a selection of the agent module 227 by the user. In some embodiments, an agent module 227-1 is configured for a first specific task of generating a summary report of a patient's medical records, a second agent module 227-2 is configured for a second specific-task of guiding a patient through a care plan, a third agent module 227-3 is configured for a third specific-task of creating patient care guidelines based on a patient's health profile, a fourth agent module 227-4 is configured for a fourth specific-task of identifying patients requiring follow-up at a hospital, a fifth agent module 227-5 is configured for a fifth specific-task of identifying changes in a standard of care for a disease setting, a sixth agent module 227-6 is configured for a sixth specific-task of evaluating unstructured data associated with a patient to identify a cohort of similar patients, a seventh agent module 227-7 is configured for a seventh specific-task of phenotyping a subject, or a combination thereof. However, the present disclosure is not limited thereto. In some embodiments, the ML component 226 is at one or more client devices 102 and/or server system 106.

In some embodiments, each model 228 and/or agent module 227 provides a range of content and functionality that an end-user can engage with and/or configure for such engagement through one or more nodes 232 associated with the agent module 227, from a simple static response to sophisticated knowledge systems that facilitate automated conversations and data analysis leading to solutions and integrated transactions with external systems. Collectively, the one or more nodes 232 form some or all of a node architecture 230 associated with the agent module 227, which defines rules for traversing between nodes. In some embodiments, each respective agent 227 has a corresponding node architecture 230, which provides a one-to-one relationship between agent modules 227 and node architectures 230. In some embodiments, a respective agent module 227 supports the generation of additional agent modules 227 that engage with one or more models 228 and/or nodes 232 of a node architecture 230 of the respective agent module 227 or a different agent module 227. In some embodiments, a respective agent module 227 supports the selection of agent modules 227 in a library of agent modules, and defining flexible integrations of these agent module 227 into various system architectures. However, the present disclosure is not limited thereto.

In some embodiments, each model 228 and/or agent module 227 provides a defined scope for engaging in a workflow. Accordingly, in some embodiments, each model 228 and/or agent module 227 is configured to assist end users to either resolve a question and/or problem or to fulfill a specific request for retrieving information, such as through a conversational communications framework. Some embodiments provide an ability to create, manage, and administer agent modules 227 to make them available for use in creating, editing, or deleting agent modules 227 via a user interface, e.g., by using a user-interface-based agent module builder or the like.

Some embodiments provide a user-interface-based agent module designer to assist in the creation and editing of agent modules 227 and/or a workflow associated with a variety of agent modules 227 (the workflow is also sometimes also referred to as an assembly or orchestration). In some embodiments, this workflow is manifested as a node architecture that includes a plurality of interconnected nodes. In some embodiments, the agent module designer includes the ability to define the name of an agent module 227, create an agent module 227, edit an agent module 227, delete individual nodes 232 associated with an agent module 227, expand and/or collapse node 232 branches, the ability to see and edit the conditional logic for a node 232, and the ability to see node traversals (e.g., when one or more nodes 232 connect to a different node 232).

In some embodiments, a node 232 of an agent module 227 reflects one or more decision points within an agent module 227, such as one or more predetermined decision points. In some embodiments, an agent module 227 evaluates data (e.g., a prompt provided by a user at a client device 102, an output from a different agent module 227, etc.), such as graphical data from a client device 102 by parsing and/or evaluating the incoming data for recognized keywords, phrases, ground truth labels, etc. For example, based on detection of recognized features, an agent module 227 may process information associated with the data received from the client device 102 in a particular direction within the plurality of interconnected nodes 232, such as from a node 232-1 associated with an agent module 227-1 to a node 232-2 associated with the agent module 227-1 and/or from the node 232-1 associated with the agent module 227-1 to a node 232-2 associated with the agent module 227-1. Thus, in some embodiments, the use of one or more nodes 232 associated with a respective agent module 227 in a plurality of interconnected nodes 232 is similar to walking through a decision tree, with different nodes 232 associated with different agent modules 227, where each different agent module 227 evaluates information based on associated conditional logic to progress information in the plurality of interconnected nodes 232. However, the present disclosure is not limited thereto. In some embodiments, each node in the plurality of interconnected nodes 232 comprises conditional logic that can evaluate data, retrieve data, generate data, or a combination thereof, e.g., based on an evaluation of information inputted to the respective node 232. In some embodiments, each node in the plurality of interconnected nodes 232 takes some action, such as generating a message and/or sending information to another node 232 in the same agent module 227 as the respective node, or a different node 232 of another agent module 227, or the like.

In some embodiments, a corresponding node architecture 230 associated with one or more respective agent modules 227 defines conditional logic 236, at least in part, for performing a specific clinical task. For example, each respective node 232 may include corresponding logic 236, which defines a workflow for handling one or more tasks assigned to the respective node 232. In some embodiments, the conditional logic of the node architecture 230 is executed in accordance with a first order of a first set of interconnected nodes 232 from a plurality of nodes 232 based on the corresponding logic 236 of each node 232 in the set of interconnected nodes 232. Accordingly, the logic 236 allows for granular configuration of each respective node 232 that when collectively coupled through interconnected nodes of the node architecture 230, define a conditional logic of the node architecture. For example, the logic 236 may include one or more AND, OR, XOR, and/or NOT operations within the logic 236. As an example, a corresponding logic 236 requires presence of a first condition but not a second condition or third condition.

In some embodiments, the plurality of nodes includes one or more data source nodes 232 associated with a specific task of obtaining data elements from a remote data source (e.g., an external database 108). In some embodiments, the corresponding logic 236 allows for connecting to a corresponding database, e.g., by using an access token associated with the corresponding agent module 227, communicating at least a portion of the obtained data to one or more nodes 232, and/or execute one or more queries to identify/analyze such data. In some embodiments, each node architecture 230 includes at least one input node, which forms an initial terminal node in an order of nodes 232. In some embodiments, the node architecture includes a plurality of paths to traverse from an input to an output node, such as paths of branching trees. In some embodiments, each respective node 232 represents a computational process, such as a function, an input, an output, or the like, which is realized when data is applied to the node 232. Moreover, since each node is interconnected, such by an edge, to at least one other node 232, the output from one node 232 may be supplied as input to a different node 232 in order to form chains, or orders, or nodes in the node architecture 230.

In some embodiments, the memory 218 includes one or more modules not shown in Figures 2A and 2B. For example, the memory 218 may include one or more agent modules (e.g., a retriever component) that are distinct from the ML component 226. In some embodiments, the client device 102 includes one or more standalone agents (e.g., that execute and operate at the client device 102) and/or one or more dependent agents (e.g., that operate in conjunction with a component at a remote device, such as the server system 106). In some embodiments, one or more models are generated/trained at the server system 106 and deployed at the client device 102.

Although Figures 2A and 2B illustrate the client device 102 in accordance with some embodiments, Figures 2A and 2B are intended more as a functional description of the various features that may be present in a client device than as a structural schematic of the embodiments described herein. In practice, and as recognized by those of ordinary skill in the art, items shown separately could be combined and some items could be separated.

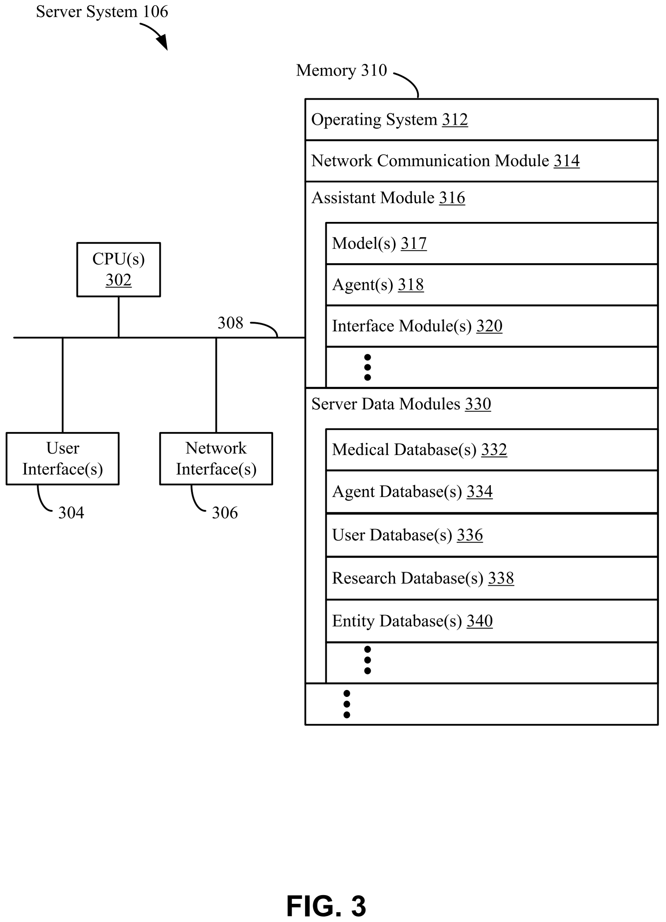

Figure 3 is a block diagram illustrating a server system 106 in accordance with some embodiments. In accordance with some embodiments, the server system 106 includes one or more CPUs 302, one or more user interfaces 304, one or more network interfaces 306, memory 310, and one or more communication buses 308 for interconnecting these components. In some embodiments, the server system 106 includes other types of control circuitry and/or processors (e.g., in addition to, or alternatively to the CPUs 302). For example, the server system 106 may include one or more GPUs or DPUs for machine learning tasks.

The memory 310 includes high-speed random-access memory, such as DRAM, SRAM, DDR RAM, or other random access solid-state memory devices; and may include non-volatile memory, such as one or more magnetic disk storage devices, optical disk storage devices, flash memory devices, or other non-volatile solid-state storage devices. The memory 310 optionally includes one or more storage devices remotely located from one or more CPUs 302. The memory 310, or, alternatively, the non-volatile solid-state memory device(s) within the memory 310, includes a non-transitory computer-readable storage medium. In some embodiments, the memory 310, or the non-transitory computer-readable storage medium of the memory 310, stores the following programs, modules and data structures, or a subset or superset thereof:

- an operating system 312 that includes procedures for handling various basic system services and for performing hardware-dependent tasks;

- a network communication module 314 that is used for connecting the server system 106 to other computing devices connected to one or more networks 104 via one or more network interfaces 306 (wired or wireless);

- an assistant module 316 that engages with a user (e.g., a remote user) in an integrated, conversational manner using natural language dialog, and invokes external services when appropriate to obtain information or perform various actions. In some embodiments, the assistant module 316 works in conjunction with an agent library at a client device 102 (e.g., the ML component 226). In some embodiments, the assistant module 316 includes the following modules (or sets of instructions), or a subset or superset thereof:

  ◦ one or more models 317 that are configured to intake data (e.g., numerical embeddings) and output an analysis of the intake data;

  ◦ one or more agents 318 that are configured to perform specific tasks or perform tasks within specific domains (e.g., any of the agents described herein, such as a retriever agent and a target population membership agent); and

  ◦ one or more interface modules 320 that allows the agent(s) 318 to communicate with other agents, applications, components, and devices (e.g., via an API or structured query); and

- one or more server data modules 330 for managing the storage of and/or access to data (e.g., clinical and user data). In some embodiments, the one or more server data modules 330 include:

  ◦ one or more medical databases 332 for storing medical data (e.g., regarding therapies, drugs, treatments, patients, cohorts, imaging, and/or diseases);

  ◦ one or more agent databases 334 for storing agent data such as settings, training, instructions, and other metadata;

  ◦ one or more user databases 336 for storing user data such as user preferences, user settings, and other metadata;

  ◦ one or more research databases 338 for storing research and conference data and associated metadata; and

  ◦ one or more entity databases 340 for storing entity-specific data (e.g., regarding research projects, interests, and products) and associated metadata.

In some embodiments, each model 228 and/or 317 includes a plurality of learned parameters (e.g., weights and biases) that are optimized during training to map inputs to outputs, as well as tunable hyperparameters (such as learning rate, batch size, optimizer type, number of layers/heads, hidden dimensions, dropout rates, and context window length) that govern training dynamics and generalization. Models may employ attention mechanisms, normalization layers, and activation functions (e.g., ReLU, GELU) and can be configured with modality-specific adapters, temperature and top-k/top-p decoding controls for generative tasks, and calibration routines that produce confidence scores or uncertainty estimates alongside outputs.

In some embodiments, the server system 106 includes web or Hypertext Transfer Protocol (HTTP) servers, File Transfer Protocol (FTP) servers, as well as web pages and applications implemented using Common Gateway Interface (CGI) script, PHP Hyper-text Preprocessor (PHP), Active Server Pages (ASP), Hyper Text Markup Language (HTML), Extensible Markup Language (XML), Java, JavaScript, Asynchronous JavaScript and XML (AJAX), XHP, Javelin, Wireless Universal Resource File (WURFL), and the like.

In some embodiments, the memory 310 includes one or more modules not shown in Figure 3. For example, the memory 310 may include one or more agent modules (e.g., a retriever component) that are distinct from the assistant module 316. In some embodiments, the server system 106 includes one or more standalone agents (e.g., that execute and operate at the server system 106) and/or one or more dependent agents (e.g., that operate in conjunction with a component at a remote device, such as a client device 102). In some embodiments, the memory 310 includes an agent library.

Although Figure 3 illustrates the server system 106 in accordance with some embodiments, Figure 3 is intended more as a functional description of the various features that may be present in a server system than as a structural schematic of the embodiments described herein. In practice, and as recognized by those of ordinary skill in the art, items shown separately could be combined and some items could be separated. For example, some items shown separately in Figure 3 could be implemented on single servers and single items could be implemented by one or more servers. In some embodiments, one or more of the databases shown in Figure 3 are stored on devices that are accessed by the server system 106 (e.g., the external database(s) 108). The actual number of servers used to implement the server system 106, and how features are allocated among them, will vary from one implementation to another and, optionally, depends in part on an amount of data traffic that the server system manages during peak usage periods as well as during average usage periods.

Each of the above identified modules stored in the memory 218 and 310 corresponds to a set of instructions for performing a function described herein. The above identified modules or programs (e.g., sets of instructions) need not be implemented as separate software programs, procedures, or modules, and thus various subsets of these modules may be combined or otherwise re-arranged in various embodiments. In some embodiments, the memory 218 and 310 optionally store a subset or superset of the respective modules and data structures identified above. Furthermore, the memory 218 and 310 optionally store additional modules and data structures not described above.

In some embodiments, client device 102 and/or the server system 106 includes custom hardware optimized for models (e.g., models 228 and 317). Example configurations employ accelerator boards with multi-die tensor cores and mixed-precision pipelines (e.g., FP8, BF16, FP16, INT8) backed by on-package high-bandwidth memory to sustain attention and matrix-multiply throughput during both training and inference. Multiple accelerator boards within a node can be coupled over a high-speed, low-latency fabric to enable tensor, pipeline, and expert parallelism for the models. In some embodiments, hardware-assisted collective operations (e.g., all-reduce, all-gather) and in-network aggregation operations are used. Dedicated DMA engines may be used to overlap compute with host-to-device transfers and NVMe-over-Fabrics access to sharded parameter stores, embedding/vector indices, and key/value caches.

A model architecture may integrate liquid-cooled accelerator trays, a spine-leaf network using 400G/800G links with congestion-aware routing, and/or disaggregated, memory-rich parameter servers (e.g., CXL-attached RAM pools) to host large (e.g., multi-terabyte) optimizer states and/or long-context KV tensors used by the models. Smart NICs and/or data processing units can offload token streaming, inference scheduling, and other operations (e.g., while providing in-line telemetry). Persistent storage tiers may combine NVMe SSDs with object storage for dataset lakes and artifact registries under an orchestration layer that supports elastic scaling and preemption. At the chip level, accelerators may include systolic/matrix engines (e.g., optimized for attention with fine-grained sparsity) and/or hardware partitioning (e.g., multi-instance modes) to multiplex concurrent inference jobs. These hardware features can collectively reduce energy per token, improve time-to-serve, and enable low-latency, high-throughput operation of the models in long-context, multi-modal workloads.

Figure 4 is a block diagram illustrating one or more system databases 400 in accordance with some embodiments. In some embodiments, at least a portion of the system database(s) 400 is optionally stored at a client device 102 (e.g., as the medical database(s) 242), the server system 106 (e.g., as the medical database(s) 332), and/or the external database(s) 108, which advantageously allows for an edge at and/or near the client device 102, such as via the communication network. However, the present disclosure is not limited thereto. In some embodiments, a single database stores all of the information shown in Figure 4. In some embodiments, the information is stored in a set of two or more databases.

In some embodiments, the system database(s) 400 includes subject and clinical datasets 402 and/or a non-patient specific knowledge database (KDB) 404. In some embodiments, the datasets 402 include, among other data, genome, transcriptome, epigenome, microbiome, clinical, stored alterations proteome, -omics, organoids, imaging and cohort and propensity data sets. For example, the cohort selection, searching, analytics, and research datasets may include data about patients and conditions, such as tumors of unknown origin (TUO) predictors, metastasis predictors, and survival analytics. As an example, the imaging datasets may include radiology imaging data, immunohistochemistry imaging data, positron emission tomography (PET) data, pathology imaging data, cardiology imaging data, neurology imaging data, and/or single-photon emission computed tomography (SPECT) imaging data. The pathology imaging data may include hematoxylin and eosin (H&E) and/or Immunohistochemistry (IHC) data. The cardiology imaging data may include electrocardiogram (ECG or EKG) data. The neurology imaging data may include electroencephalogram (EEG) data. The imaging datasets may include data regarding nodule identifiers, tracking, and/or longitudinal analytics. The clinical data may include curated, uncurated, electronic medical record (EMR), and/or EHR data. The uncurated data may include raw images of documents which can be OCRed and then fed to a model for structuring/summarizing. In some embodiments, the same model performs the OCR and structuring.

In some embodiments, the clinical data includes diagnostics, imaging, biopsy information, and other disease- and condition-related data. For example, for endocrinology diagnostics, the primary test used may be a blood test to measure hormone levels in the body, which can identify various endocrine disorders by checking for imbalances in hormones such as thyroid stimulating hormone (TSH), luteinizing hormone (LH), follicle stimulating hormone (FSH), testosterone, and others depending on the suspected condition. Additional tests such as ultrasounds, CT scans, or biopsies may be performed depending on the situation, e.g., to locate abnormalities in endocrine glands like the thyroid or adrenal glands. Blood tests for endocrinology diagnostics can be used to measure various hormones in the blood, allowing diagnosis of conditions like hypothyroidism, hyperthyroidism, diabetes, and adrenal insufficiency. Imaging tests such as ultrasounds, CT scans, or MRIs can be used to visualize the endocrine glands and identify abnormalities like nodules or tumors. A fine needle aspiration (FNA) biopsy may be performed to collect a tissue sample from a suspicious area in the thyroid gland for further analysis. Thyroid function tests may be used to measure TSH, T4, and T3 levels to assess thyroid function. Cortisol level tests may be used to check for adrenal gland issues. Glucose tolerance tests may be used to diagnose diabetes by monitoring blood sugar levels, e.g., after consuming a sugary drink. Prolactin tests may be used to check for prolactin levels associated with pituitary gland disorders. Calcium and parathyroid hormone (PTH) levels may be determined to assess parathyroid gland function. For each endocrinology-related test, the data relating to the test (e.g., diagnostics, imaging, and metadata (such as timing, location, etc.)) may be stored in the clinical data, and associated with a particular subject.

As another example, to diagnose diabetes, a doctor may use a blood test, such as the Hemoglobin A1c (A1C) test, which measures average blood sugar level over the course of two to three months. The A1C test provides a snapshot of a subject's average blood sugar over a period of time and does not require fasting. Other tests may be used, such as a fasting blood sugar test, an oral glucose tolerance test (OGTT), or a urine test, depending on the situation. The fasting blood sugar test measures a subject's blood sugar level after fasting for at least 8 hours. The OGTT involves the subject drinking a sugary liquid and then having their blood sugar levels checked at specific intervals. While not as accurate as blood tests, a urine test may be used in some situations to check for ketones, a sign of uncontrolled diabetes, particularly in type 1 diabetes. For each diabetes-related test, the data relating to the test may be stored in the clinical data, and associated with a particular subject.

As another example, to diagnose and/or assess depression a variety of tests and tools can be used, including questionnaires, physical exams, lab tests, and brain scans. For example, the Patient Health Questionnaire (PHQ-9) is a questionnaire that can help diagnose depression and assess its severity. The PHQ-2 is an initial screening tool for depression that can be used in all age groups. Other questionnaires include the Social Problem-Solving Inventory-Revised (SPSI-RTM), which is a self-report measure of social problem-solving strengths and weaknesses. The Edinburgh Postnatal Depression Scale (EPDS) is a 10-question scale that can be used to screen for depression in women who have recently given birth. In some situations, a doctor or other mental health professional may perform a physical exam and ask questions about a subject's health to diagnose/assess depression. A mental health professional may also use the criteria for depression listed in the Diagnostic and Statistical Manual of Mental Disorders (DSM-5). In some situations, lab tests are used to rule out other medical conditions that could be presenting as depression. These tests may include a complete blood count (CBC), thyroid-stimulating hormone (TSH), vitamin B-12, and the like. Additionally, a PET scan of the brain can compare brain activity during periods of depression with normal brain activity. A CT scan or MRI of the brain may be considered if organic brain syndrome or hypopituitarism is in the differential diagnosis. For each depression-related test, the data relating to the test may be stored in the clinical data, and associated with a particular subject.

As another example, there are different types of diagnostic tests that can be used to diagnose cardiovascular disease, including Electrocardiograms (ECG or EKG), stress tests, cardiac MRIs, cardiac positron emission tomography (PET) scans, invasive coronary angiographies, echocardiograms, blood tests, x-rays, cholesterol tests, and plasma ceramides tests. A doctor may use a combination of tests to diagnose a heart problem. For example, a doctor might use an echocardiogram, cardiac MRI, or a nuclear heart scan to take images of the heart during or after a stress test. For each test, the data relating to the test (including any comparisons, cross references, and conclusions based on multiple tests) may be stored in the clinical data, and associated with a particular subject.

In some embodiments, the KDB 404 includes separate sub-databases related to specific information types including, as shown, provider panels (e.g., information related to genetic panels supported by the service provider that operates the system), drug classes (e.g., drug class specific information (e.g., do drugs of a specific class work on pancreatic cancer, what drugs are considered to be included in a specific drug class, etc.)), specific genes, immuno results (e.g., information related to treatments based on specific immuno biomarker results), specific drugs, drug class-mutation interactions, mutation-drug interactions, provider methods (e.g., questions about processes performed by the service provider), clinical trials, immuno general, clinical conditions such as clinical diseases, term sheets (e.g., definitions of industry specific terms), provider coverage (e.g., information about provider tests and results), provider samples (e.g., information about types of samples that can be processed by the provider), knowledge (e.g., scripted questions and answers on various frequently asked questions that do not fall into other sub-databases), radiation (e.g., information related to suitable radiation treatments given specific cancer states), clinical guidelines (e.g., national guidelines related to classification of cancer states, accepted treatments, etc.) and clinical trials questions-answers (e.g., information related to locations and administrators of clinical trials. Organizing the KDB 404 into sub-databases may make it easier to manage those databases as information therein evolves over time and also enables addition of new sub-databases related to other defined information types. In some embodiments, the clinical datasets 402 and/or the KDB 404 is arranged in a different manner than is shown in Figure 4 (e.g., with different sub-databases and/or with a different organizational scheme).

In some embodiments, the data stored in the subject and clinical datasets 402 and/or the KDB 404 includes raw data, annotated data, and/or summarized data. In some embodiments, the raw data is input into one or more models to generate the annotated and/or summarized data. For example, a model may receive raw data, such as sequencing results, documents, and/or images, and extract/predict status information and/or summaries. In some embodiments, one or more models (e.g., one or more agents) are used to partition, annotate, summarize, and/or structure the data received from external sources (e.g., external databases and/or third parties). In some embodiments, the data stored in the subject and clinical datasets 402 and/or the KDB 404 is classified, grouped, cross-referenced, and/or otherwise related to other data using one or more models (and/or one or more agents). For example, a cohort may be identified based on EMR/EHR information from multiple subjects/patients. In some embodiments, an intake agent is used on data that is received to perform one or more of the actions described above. In some embodiments, different intake agents (e.g., data processing/pre-processing agents) are used for different modalities of data.

Advantageously, by utilizing multiple datasets associated with different domains of subject matter and/or applying a classification system to the datasets, the knowledge database provides a storage system for data, such as medical records and clinical documentation that one or more agent modules 227 can retrieve based on a task-specific requirement associated with a respective domain or classification. Moreover, in some embodiments, the knowledge database 404 allows for storing such data with deidentifying controls in order to allow for training on and/or analysis of the stored data without risk of leaking confidential and/or privileged information.

Considering the extensive volume of text contained within a real-world data (RWD) warehouse of EHRs, it becomes impractical to process the entirety of a patient's clinical notes within the context window of a model (e.g., an LLM). In some embodiments, this challenge is addressed by implementing a retrieval-augmented generative (RAG) approach to identify relevant portions of EHR text, e.g., relevant portions of unstructured clinical notes. A RAG approach proves to be more efficient and effective than providing the model with larger context windows. In some embodiments, RAG is a two-step process that involves retrieving relevant documents from a corpus (e.g., a large corpus with thousands or millions of documents) and then feeding the retrieved documents into a model to generate an analysis and response.

In some embodiments, the heterogeneous information described above is transformed into numerical embeddings for efficient retrieval and analysis. In some embodiments, unstructured text (e.g., clinical notes, guidelines, trial protocols), tabular fields (e.g., labs, therapies, outcomes), imaging-derived annotations, and/or molecular features are first normalized and segmented into modality-appropriate chunks, then passed through modality-specific encoders (or a shared cross-modal encoder with adapters) to produce (fixed-dimension) vectors in a shared latent space. These vectors capture semantic content and associated metadata context (e.g., timestamps, assay type, cohort attributes) via feature conditioning and are optionally augmented with additional information, such as ontology IDs and provenance tags. The resulting embeddings may be stored in a vector database that supports approximate nearest neighbor search, time-aware indexing, access control lists, and/or incremental upserts, thereby enabling fast similarity retrieval, temporally adaptive clustering, and downstream pipelines using the combined clinical, molecular, and imaging corpus.

In some embodiments, the ML component 226 and/or assistant module 316 use a retrieval-augmented generation (RAG) to perform operations described herein (e.g., requests to process zero-shot information). For example, the computing system may apply the RAG process to entire patient records, which allows for applying the entire patient records to the model 228 with excess computational burdens, instead of focusing solely on a specific type of clinical note. In some embodiments, the RAG process is used to analyze clinical mentions throughout a patient's entire record without the need for predefined sections of interest. However, the present disclosure is not limited thereto. In some embodiments, the RAG process utilizes one or more vector embeddings, such as a plurality of predetermined vector embeddings in which each predetermined vector embedding is associated with a corresponding text string, or snippet. Advantageously, this RAG approach can be more efficient and effective than providing a model (e.g., an LLM) with larger context windows.

In some embodiments, one or more of the models or agent modules use additional techniques to address an issue that RAG implementations can fail to obtain all of the needed information to fully answer a question (e.g., a user query). In such situations, another request (e.g., a new user query, and/or a modified version of the user query) can be automatically generated to cause more information to be obtained. An example technique includes applying a user query for information from a source dataset to a first RAG agent (e.g., from the ML component 226) to determine if there is enough information to generate an output based on the user query. The RAG agent can determine that there is enough information, that there is not enough information, or that the determination is not clear. In some embodiments, if the determination whether there is enough information is not clear, the computing system provides a query to a different task-specific orchestration (e.g., corresponding to a different agent module 227 of the ML component 226). That is, in some embodiments, the system determines that the RAG agent may not be the optimal instrumentation for resolving the user query.

In some embodiments, operations of one or more models and/or task-specific orchestrations of the system are adjusted to reduce/prevent negative consequences of retrieval-augmented generation. For example, for some inclusion/exclusion criteria for trial matches or care gap discovery, the queries have a relationship and can include a temporal question (e.g., "Is this medication administration currently administered as the first line of therapy?"). As another example, with a standard RAG retrieval approach, only documents relevant to medications may be retrieved. But the task-specific orchestration (e.g., the RAG agent) may not know if the medications were administered as part of the first or second line of therapy without the full context of the patient. In such situations, using a large context where most of the patient notes can be applied can provide the task-specific orchestration better context and more comprehensive information about the temporal relationship between events. Alternatively (e.g., to address the resource constraints of increasing the context window applied to the RAG agent), a different model (e.g., a full patient record LLM with a one-million-character context window) or agent can be used to resolve the user query in addition or alternatively to the RAG agent. For example, increasing the context window and/or performing additional operations alternative to directing a request to the RAG agent (e.g., to extract information) can increase performance of generating the output based on the user query for information.

In some embodiments, the techniques described herein are realized with a transformer-based architecture, e.g., configured to operate over heterogeneous, multimodal inputs and long temporal horizons. By leveraging attention mechanisms to fuse text, imaging-derived features, molecular profiles, and structured clinical signals into a unified representation, the transformer may serve as the engine for embedding generation, temporally-aware clustering, retrieval-augmented reasoning, and action ranking described herein.

A transformer model is a neural network that learns context and thus meaning by tracking relationships in sequential data like the words in this sentence. Transformer models can apply attention, or self-attention, to detect how distant data elements in a series influence and depend on each other. Using embeddings (e.g., word embeddings), transformers can pre-process text as numerical representations through the encoder and understand the context of words and phrases with similar meanings as well as other relationships between words such as parts of speech. The models can then apply this knowledge of the language through the decoder to produce a unique output.

A large language model (LLM) is a large deep learning model that is pre-trained on large amounts of data, for example, in the size range of terabytes or even pentabytes. An LLM may have billions or trillions of parameters. LLMs typically consist of dozens or even hundreds of transformer blocks stacked on top of each other. In a classic LLM, each LLM includes an encoder block that takes a sequence and processes it into a set of context-rich embeddings, and a decoder block that takes the encoder's output and generates the output sequence. However, some LLMs include transformer blocks that only include an encoder and some LLMs include transformer blocks that only include a decoder. The transformer architecture makes use of self-attention, residual connections, and normalization. LLMs, which include stacks of transformer blocks, therefore make use of these features as well. Whereas a transformer model has in the order of millions of parameters, a large language model is characterized by having at least 1 billion parameters. As is apparent to one of skill in the art, these values exist in a continuous stream, e.g., there may be LLMs with 100 million parameters, 50 transformer blocks, or other numbers of parameters that allow for the robust performance expected of LLMs.

Whereas a transformer model may have, for example, between 6 to 24 transformer blocks, an LLM typically has 80 or more transformer blocks.. Whereas a transformer model may be trained on domain-specific datasets that range in size between gigabytes and tens of gigabytes, an LLM is typically trained on more diverse datasets that are measured in terabytes or pentabytes.

Embeddings are representations of values or objects (e.g., text, images, and/or audio) that are used by machine learning models. Embeddings may be vectors generated to capture meaningful data about each object. An embedding may be a word embedding that represents a word (or phrase) and is used in text analysis. The word embedding may be in the form of a real-valued vector that encodes the meaning of the word in such a way that the words that are closer in the vector space are expected to be similar in meaning. In the case where words and phrases are one-hot encoded, an embedding is typically dimension reduced relative to the model input. For example, consider the case where a model has a vocabulary size of 50,000 words and/or phrases. Words and phrases in model input are one-hot encoded using this vocabulary and thus the input has a dimension of 50,000. In some models in accordance with the present disclosure, such high-dimensional input is dimension reduced relative to the original one-hot input. For instance, in one particular example the embedding maps the 50,000 word/phrase vocabulary to 768 dimensions. However, there is no absolute requirement that an embedding be dimension reduced relative to the input. For instance, in some embodiments the embedding captures input context and/or metadata, resulting in embeddings that are not dimension reduced or even dimension increased relative to the input.

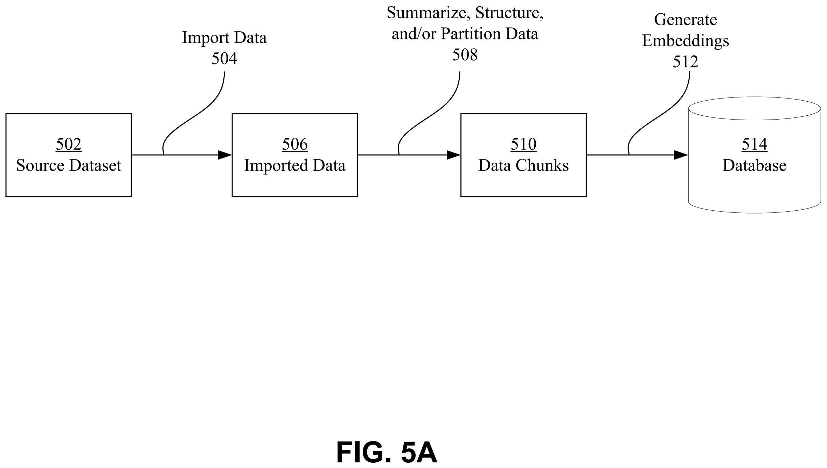

Figure 5A illustrates an example process for data vectorization in accordance with some embodiments. As shown in Figure 5A, a source dataset 502 is imported (504) as imported data 506. In some embodiments, the source dataset 502 includes one or more documents (e.g., one or more PDF documents, text documents, and/or other types of documents), one or more images, and/or other structured or unstructured data. In some embodiments, the source dataset 502 is obtained from one or more databases (e.g., the external database(s) 108). In some embodiments, the source dataset 502 is identified by a user for importation into the system (e.g., the platform 100). In some embodiments, the source dataset 502 includes medical, clinical, and/or patient data. In some embodiments, the source dataset 502 comprises research corpora curated around a specific conference, publication venue, corporation, key investigator, or other entity of interest. For example, the system may ingest an entire conference abstract booklet, a compendium of PubMed articles authored by a particular principal investigator, or filings and press releases associated with a pharmaceutical sponsor. These materials can be acquired via authenticated APIs, bulk downloads, web crawling subject to access policies, or user-provided uploads, and may include heterogeneous artifacts such as PDFs, slide decks, posters, supplementary tables, and figures. In some embodiments, a target collection is constructed based on a keyword search, a biomarker search, and/or other targeted searches. The platform can also construct targeted collections by querying external registries (e.g., for trials sponsored by a named company), scraping conference schedules to assemble session-level packets, subsetting by specific keywords or biomarkers, and/or filtering publication feeds by journal or special issue, thereby enabling entity-centric analyses and downstream vectorization of the assembled research documents.

In some embodiments, the source dataset 502 includes clinical notes. Some embodiments include preprocessing long clinical notes (e.g., greater than 100,000 words) to use with pre-trained models that have a word limit (e.g., 512 words) without having to throw away context. In some embodiments, the clinical notes are aggregated to episodes. An encounter includes an interaction between a patient and a healthcare provider that results in the logging of clinical notes into an EHR system. An episode includes a cluster of encounters representing a hospital stay. Typically, a single hospital stay is logged into multiple encounters. Some embodiments determine, for each patient, episode boundaries using one-dimensional clustering (e.g., kernel density estimation (KDE)) on encounter date. In some embodiments, notes between boundaries are aggregated together.

In accordance with some embodiments, the imported data 506 is de-identified (e.g., any personally identifiable information (PII) is removed). The imported data 506 is converted (508) into data chunks 510. In some embodiments, the conversion includes summarizing the imported data 506 (e.g., using one or more ML models). In some circumstances, text may be too large to feed directly into an ML model. Accordingly, some embodiments segment or split the text into roughly even snippets, e.g., taking sentence boundaries into account. Some embodiments rank and trim text according to a number of relevant words in each snippet. Some embodiments limit the number of snippets and/or words per snippets (e.g., a maximum size of 512 snippets of 256 words, totaling 131,072 words). In some embodiments, the conversion includes converting unstructured data into structured data (e.g., using one or more ML models). In some embodiments, the conversion includes partitioning the data (also sometimes called chunking or snippetizing). For example, the imported data may be converted to structured data then summarized and then the summary data may be partitioned to generate the data chunks 510. In some embodiments, the imported data 506 is summarized without being converted to structured data. In some embodiments, the imported data includes visual data that is annotated and/or characterized during the conversion process. Although Figure 5A illustrates an example in which data is segmented into smaller chunks prior to embedding, in some embodiments, tokens and/or embeddings are generated from the imported data without the segmentation. For example, tokens and/or embeddings of the imported data may be segmented instead of the imported data itself. In some embodiments, a first model is used to generate the tokens/embeddings and a second model is used to perform the segmentation. For example, the output of the first model may be input into the second model (or vice versa).

In some embodiments, the conversion includes identifying relevant portions of metadata (e.g., authors, sponsors, assay type, cohort attributes, timestamps, study design, and biomarker identifiers) from available metadata fields (optionally normalizing them using techniques such as schema harmonization and ontology mapping), and attaching the normalized metadata to each data chunk as annotations. Visual and tabular chunks can be annotated with modality-specific labels (e.g., imaging region of interest, magnification, stain type; table schema and units), while text chunks can be tagged with entity and relation markers, provenance, and confidence scores. The annotated chunks may then be processed by modality-specific encoders (or a shared cross-modal encoder with adapters) that condition on the attached metadata to generate (fixed-dimension) numerical embeddings in a shared latent space. These embeddings capture semantics and context (including temporal signals) and may be written to a vector database, e.g., with access control lists, ontology IDs, and time stamps to support approximate nearest neighbor search, time-aware indexing, and incremental upserts. In some embodiments, tokens and/or embeddings generated from the imported data are annotated. For example, updated tokens and/or embeddings may be generated by combining the initial tokens and/or embeddings with annotation data. In this way, tokens/embeddings may be annotated rather than (or in addition to) annotating input data itself. In some embodiments, an embedding process is performed on one or more tokens and/or one or more embeddings, e.g., to incorporate metadata and/or context data.

A set of (one or more) embeddings are generated (512) from the data chunks 510 and stored in a database 512 (e.g., a vector database). In some embodiments, the embeddings are used to train (e.g., fine tune) a machine-learning model (e.g., a model that is a component of a task-specific orchestration). In some embodiments, the embeddings are used to fine-tune downstream models and task-specific orchestrations, allowing for metadata-aware retrieval, clustering, and recommendation.

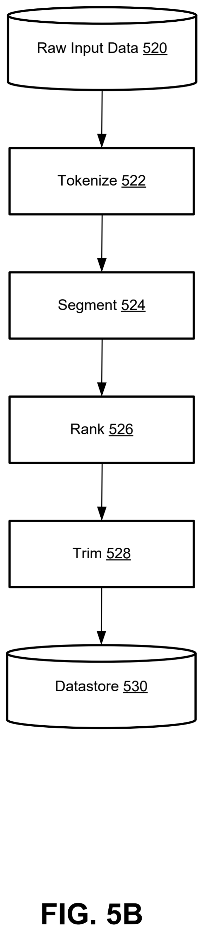

Figure 5B shows an example method of segmenting (splitting) raw input data, according to some embodiments. In the example of Figure 5B, a database 520 stores raw input data, which may have an arbitrary length. This raw data is tokenized (522) to produce a set of tokens. This set of tokens is segmented (524) into segments, e.g., with each segment having a predetermined number of tokens (e.g., 256). The segments are ranked (526) to obtain an ordered set of the segments. The ordered set is subsequently trimmed (528) to obtain a predetermined number of segments (e.g., 512 segments with 256 tokens in each segment) that may be stored in a datastore 530. The raw input data may be obtained from any number of sources. In some embodiments, regular expression filtering is used to split raw text. In some embodiments, particular punctuation marks are excluded from being identified as sentence boundaries. For example, the period at the end of the abbreviation 'Dr.' for doctor can be excluded (e.g., "dr. XX"). In some embodiments, an ML model is used to split raw text into sentences.

In some embodiments, the segment step is performed before the tokenize step. For example, the raw input data is segmented to obtain segments having a predetermined segment length. These segments are then tokenized. The tokenized segments are split to avoid long snippets. In this example, some segments are split into multiple segments. The resulting segments are ranked to obtain an ordered set of segments, which is subsequently trimmed to obtain a reduced number of snippets that may be stored in the datastore 530. Some embodiments split the raw input data into roughly even snippets of given size (e.g., 256 tokens). Some embodiments avoid cutting a snippet in the middle of a sentence, e.g., by first cutting text into sentences and then combining neighboring sentences to get roughly a same number of token snippets (e.g., 256 token snippets).

In some embodiments, the raw input data is sentencized to obtain a number of sentences, which are tokenized to obtain sets of tokens. Long snippets are split to obtain sets, where each set has a predetermined number of tokens (e.g., 256). Some embodiments generate a warning to alert a user regarding long snippets. In some embodiments, short snippets are merged to obtain a candidate set of snippets, which is ranked to obtain an ordered set and trimmed to obtain a trimmed set of snippets that is stored in the datastore 530.

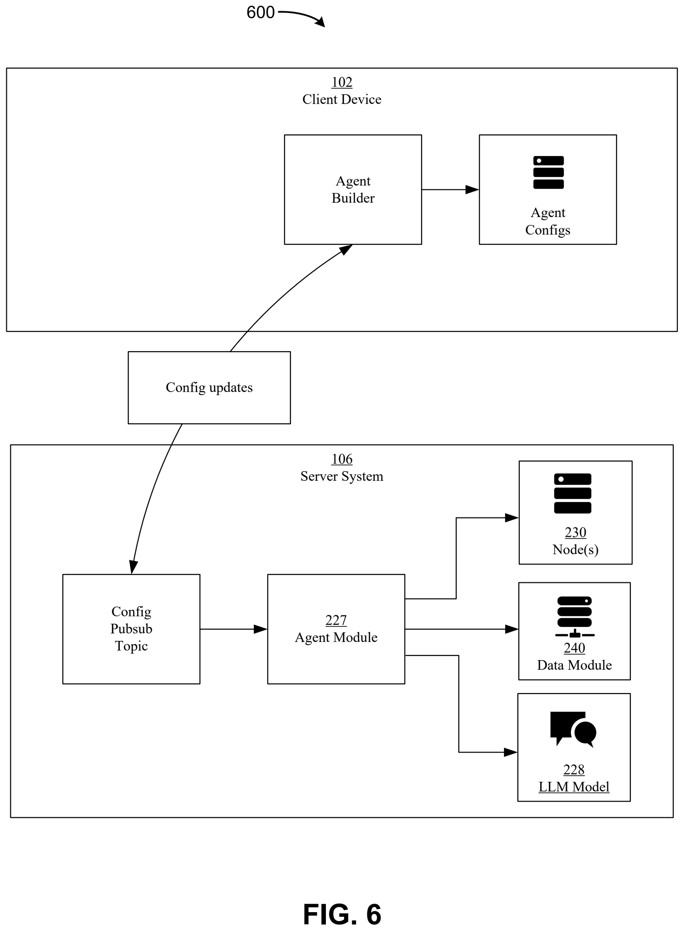

Figure 6 illustrates an example system architecture for deploying agents (e.g., agent modules 227) in accordance with some embodiments. The architecture 600 shown in Figure 6 includes an agent builder component in a control plane of a client device 102. The control plane may function as a supervisor of data, coordinating communication between different components and collecting data from a data plane (e.g., a working environment presented on a display of the client device 102). In some embodiments, the control plane resides above the data plane (e.g., above the working environments) and enforces rules for the data plane, which allows for partitioning the data plane to prevent unauthorized or unauthenticated control of the data plane from unsecure client devices, such as those unassociated with a portion of the data plane. However, the present disclosure is not limited thereto. In some embodiments, the agent builder hosts a user interface for configuring agent modules, such as by configuring the corresponding node architecture 230 associated with the agent module 227. In some embodiments, the agent builder component is communicatively coupled to an ML component 226 in the control plane that stores a plurality of agent modules 227, such as the agent module 227, and to an agent host (e.g., via a config pubsub component) in a working environment. The agent module 227 in the working environment may be communicatively coupled to an ML component 226 in the working environment, a document index (e.g., one or more data sources, such as knowledge database 404 and/or external databases 108), and a large language model (e.g., a model 228). In some embodiments, the agent library 226 includes a user interface and API for interacting with deployed agents. In some embodiments, the large language model in Figure 6 represents a node 232 (e.g., a task-specific agent) as described herein.

In some embodiments, the agent builder includes a frontend and a backend. In some embodiments, the agent builder frontend includes an access component (e.g., an administrative console, which may be a home user interface that a user is presented with upon providing access credentials to the application), an agent list (e.g., an agent library, which may include a plurality of task-specific orchestrations to which the user has access, e.g., based on the access credentials provided to the application), an agent builder component (e.g., either or both of the user interfaces 1812 and 1822 respectively, which may include a first representation of the node architecture 230 (e.g., a form-builder representation) and a second representation of the node architecture 230 (e.g., a workflow representation)), and/or a data source management component. In some embodiments, the agent builder backend includes a database layer, an API service, and/or a configuration publisher component. In some embodiments, the frontend and the backend of the agent builder are executed on separate electronic devices.

In some embodiments, the agent host includes a frontend and a backend. In some embodiments, the agent host frontend includes an access component, an agent list, an interaction console, and/or a document console. In some embodiments, the agent host backend includes a web socket for interactive user interfaces, a database layer, an API access to deployed agents, tools and/or custom chain implementations, a document loader, and/or a configuration subscription component. In some embodiments, the frontend and the backend of the agent host are executed on separate electronic devices.

In some embodiments, the agent builder component is configured to generate, deploy, and/or update one or more agent modules 227 and/or a corresponding node architecture 230 to one or more working environments (e.g., one or more workload planes). In some embodiments, each agent module 227 is associated with an agent type. In some embodiments, the agent type includes a type of model 228 and/or conditional logic 236, such as an implementation configuration. For example, an agent module 227 may include a language model associated with a first node 232 and a corresponding, type-specific logic that further associates the agent module 227, through the first node 232, with a particular domain, such as a first configuration implementation for applying the prompt to the model 228 if the prompt is associated with a first modality and a second configuration implementation if the prompt is associated with a second modality different from the first modality. In some embodiments, the logic 236 is specified in a corresponding agent module 227 configuration file, which advantageously allows for configuring the logic after applying various prompts to the agent module 227 and/or using multiple client devices (e.g., end users) to configure the logic 236. However, the present disclosure is not limited thereto.

In some embodiments, agent module types include a transform agent modules (e.g., performing functions such as data transformations, regular expressions, and string templating), authorization agent modules, language model agent modules (e.g., applying inputs to a large language model), data collection agent modules (e.g., RAG modules), super-agent modules (e.g., aware of other agent types and their capabilities and configured to instantiate and/or delegate to the appropriate agent modules), sequential agent modules (e.g., including multiple models and/or tools coupled together in a sequential fashion), tool-using agent modules, coding agent modules (e.g., configured to generate code in particular programming languages), and categorization agent modules (e.g., configured to determine an intent, domain, or other categorization for user inputs).

In some embodiments, a transform agent module comprises one or more machine-learning models configured to transform data (e.g., transform the data from a first modality to a second modality), e.g., prior to the transformed data being used by another agent. In some embodiments, the language model agent modules provide/store context information such as conversation history, user preferences, subject details, and the like. In some embodiments, the data collection agent modules are couplable to external data sources (e.g., the external service(s) 110 and/or the external database(s) 108). In some embodiments, a sequential agent module includes a recursive agent module (e.g., repeating and/or refining outputs until predetermined criteria are met). In some embodiments, a super-agent module is configured to compare available agent module types and recommend a particular agent module type for a particular situation/purpose. In some embodiments, a coding agent module is configured to generate code for new agent modules based on inputs (e.g., natural language inputs) from a user. In some embodiments, a categorization agent module is a component of a routing agent module. For example, the categorization agent module determines an intent/domain for an input and the routing agent module routes the input to a downstream component in accordance with the determined intent/domain. In some embodiments, a sequential agent module is a component of a routing agent module. For example, the routing agent module coordinates operation (e.g., data transmission and timing) of multiple components and/or modules. In some embodiments, each agent module is generated/provided with guardrails (e.g., enforcing privacy, security, data typing, etc.). In some embodiments, an agent module is configured to recognize whether data is protected health information (PHI) and take appropriate action. For example, an agent module may disable information sharing options when providing PHI.

In some embodiments, different agent module types are associated with (e.g., trained on, instructed on, and/or coupled to) different domains (e.g., different subjects, types of data, modalities of data, and/or classes of data) in a plurality of domains. For instance, in some embodiments, the plurality of domains forms an input space, which defines a universe of data associated with a variety of subject matters. In some embodiments, the input space defines an N-dimensional space of data obtained from a plurality of data sources, in which N is a positive integer, such as two, three, four, ten, etc. In some embodiments, each respective domain in the plurality of domains defines a partition classification or subset of data, such as one or more specific data sets of system databases 400 of Figure 4. However, the present disclosure is not limited thereto.

As a non-limiting example, consider a first input space associated with a plurality of medical records, in which each medical record in the plurality of medical records includes a plurality of text data and a plurality of graphical data associated with a corresponding patient. Accordingly, a plurality of domains collectively defined by information obtained from the plurality of medical records allows for classification of the information and training the agent module 227 of the information classified domain, such as a first domain associated with a statin drug class and a second domain associated with a glucagon-like peptide (GPL) agonist drug class. As a non-limiting example, an agent module 227-1 is associated with a first domain for identifying a patient cohort, an agent module 227-2 is associated a second domain for identifying research related to a patient cohort, an agent module 227-3 associated with a third domain for identifying research gaps in a set of research, an agent module 227-4 associated with a fourth domain for generating research report information (e.g., an outline, a data visualization, and/or other research report information), an agent module 227-5 associated with a fifth domain for identifying research insights, and/or an agent module 227-6 associated with a sixth domain for comparing research insights with previous insights in the topic area.

An example agent type is a database-interfacing agent module (e.g., an agent module 227) associated with one or more data source nodes 232. An example database-interfacing agent may be an adverse effects agent that has access to an FDA label database and is configured to interpret adverse effect information from the database. The configuration of the tool-using agent may include a custom prompt for the model 228 and one or more data sources that the agent database-interfacing module may access and/or use.

Another example agent type is a custom-chain agent module (e.g., a super-agent module) that takes an input prompt, analyzes the prompt (e.g., parsing the prompt into one or commands and/or a plurality of tokens), and transmits information from the parsed prompt (e.g., commands and/or tokens) to a model 228 or other component, such as a node 232 of the custom-chain agent module or a different node 232 of a different agent module 227). For example, an agent module 227 may obtain data from different databases (e.g., external databases 108, knowledge database 404, etc.), in which the data is obtained in a variety of different formats and/or structures, such as unstructured text, structured text, tables, charts, graphical data, and/or the like. In some embodiments, the agent module 227 reformats and/or restructures the data obtained from the databases for application to the model 228 and/or a different agent module 227. In some embodiments, the agent module 227 evaluates and/or obtains an optimal set of parameters for inputting data to the model 228 and/or a different agent module 227 and/or translates the data obtained from the databases based on the optimal set of parameters. In some embodiments, the obtained data is restructured into a homogenous dataset (e.g., different hospitals may use different codes for the same procedure, such is homogenized by the agent module 227 into a uniform coding). The configuration of the custom-chain agent module 227 may include a sequence of nodes 232 associated with the custom-chain agent module 227 and/or other nodes 232 associated with other agent modules 227 to be used by the custom-chain agent module 227 and/or definitions of corresponding chain objects. In this way, an agent module 227 may be considered a configuration of a particular agent type for a particular task through a plurality of interconnected nodes 232 that form a node architecture 230 of the agent module 227 (e.g., represented as a database object). Accordingly, the super-agent module 227 allows for dissecting complex evaluations and logics into a reasoning path through the plurality of interconnected nodes 232, which makes arriving at an accurate and precise response computationally less burdensome. In some embodiments, the agent modules 227 are accessible via an interaction console and/or an application programming interface (API).

In some embodiments, one or more parts of the agent configuration are stored in a separate versioning table (e.g., linked by agent ID). In this way, an agent configuration may be edited without affecting a deployed agent version. In an example scenario, a user configures an agent in the console and then deploys it to one or more environments (e.g., workload planes and/or control planes). For this scenario, the agent configuration is stored in the control plane (e.g., as shown in Figure 6). As shown in Figure 6, the agents themselves execute in the appropriate working environments, and working environments do not have access to the control plane. The agent builder in the control plane is configured to push configurations into the various environments (e.g., via the config pubsub component shown in Figure 6). In some embodiments, when an agent configuration is changed or an agent version is deployed, the agent builder informs the agent host in each environment so that the updated agent can be deployed. This may be via a pubsub message to the agent-config topic or via a simple HTTP request.

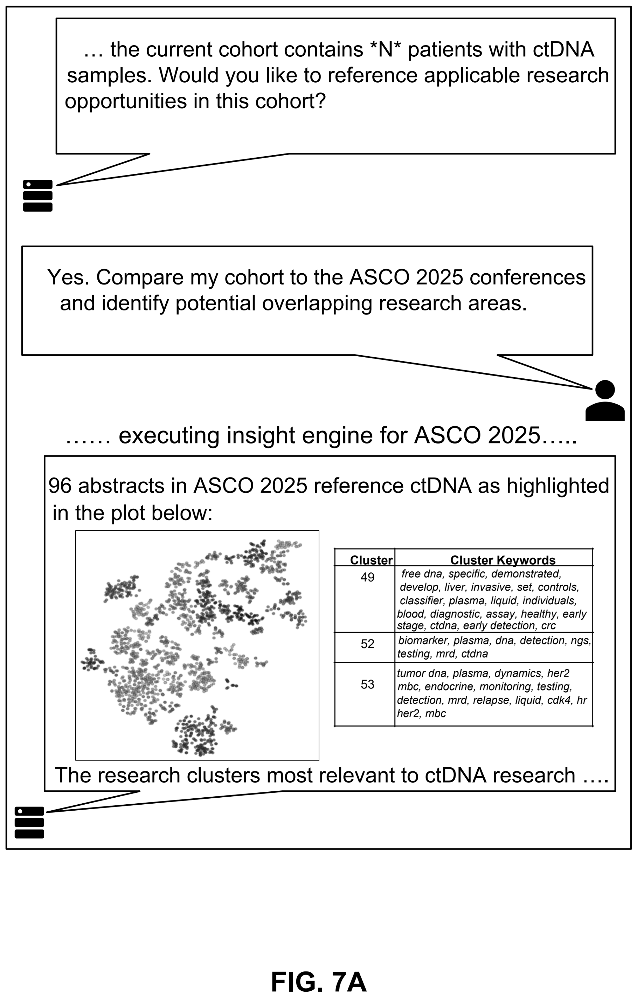

The architecture 600 allows for flexibility in supporting a variety of deployment strategies for each respective agent module 227. For example, some end-users, e.g., those using agent modules 227 interactively and without engineering support, expect to operate their agent modules 227 entirely within a production working environment. In some embodiments, the administrator, such as creator, of an agent module 227 is able to choose a deployment style suitable for their application, such as by restricting the agent module 227 to one or more domains, one or more databases 108, one or more services 110, or a combination thereof. For example, a first user may wish to employ a user interface that includes one or more user interface elements described with respect to the application (e.g., the user interfaces illustrated in Figures 7A-7B) by directly embedding the components within a web page, and a second user may wish to interact with an API that is configured to receive user requests and provide responses in the form of data structures, which the second user may integrate into different user interface elements not associated with the application.

In some embodiments, users of an agent builder user interface in the control plane are provided with a production access token that can also make requests to the production agent host. In some embodiments, an integrated user interface is presented to a user that shows both the agent builder having a plurality of input features visualized through a representation and the interaction console without concerning the users with the differences between the control plane and the working environments. For example, for users who want to evaluate agent modules 227 in a lower environment, a link may be provided to open that agent module 227 in a new tab or frame of an application. In some embodiments, a request to authenticate is presented and an access token is obtained by the agent module 227 for that environment. In some embodiments, the user interface includes an indication of which environment is currently active.

In some embodiments, an end-to-end model is trained across multiple domains (e.g., to eliminate handoffs between domain-specific agents). The model ingests heterogeneous inputs spanning text, images, tabular clinical fields, molecular profiles, and device signals, and learns shared representations via cross-modal encoders with lightweight adapters that specialize to each domain while preserving a common latent space. Domain priors (e.g., oncology guidelines, assay schemas, imaging ontologies) may be injected through multi-task objectives and contrastive alignment so that the model simultaneously optimizes for retrieval, normalization, clustering, and action ranking. In some embodiments, a unified conditioning vector encodes domain context, user/institution constraints, and temporal scope, allowing the same network to adapt its behavior when operating over different subject matters without architecture changes. Some embodiments include training under a joint loss that combines semantic consistency, metadata prediction, temporal stability, and recommendation quality. The end-to-end model produces embeddings, cluster assignments, and ranked action proposals in a single forward pass, which can reduce latency, reduce error propagation, and improve robustness across domains.

In some embodiments, the data module 240 (e.g., document index) shown in Figure 6 includes one or more of: a static corpus, a dynamic corpus, an embedding model (e.g., a model 228), a chunking strategy, a storage back-end, a data classifier (e.g., public, internal, or secret), and/or a visibility setting (e.g., private, public, or restricted by role). In some embodiments, the data module maintains an index of data that may be ephemeral or permanent. In some embodiments, data elements associated with data files (e.g., documents) are evaluated via a chunking process, embeddings are generated for the chunks generated from the chunking process, and the embeddings are inserted into a database. In some embodiments, the data module 240 includes a set of retrieval parameters (e.g., for a number of documents to retrieve and/or a similarity measure). In some embodiments, the data module 240 corresponds to a set of databases (e.g., medical databases), such as the database(s) 400 in Figure 4. In some embodiments, a parameter associated with a node 232 of a respective agent module 227 and/or model 228 includes selecting one or more document indices to retrieve from via the data module 240. In some embodiments, embeddings are created and siloed for future use. In some embodiments, each embedding is associated with one or more access control lists (ACLs).

Tools are a mechanism by which modules can integrate with other components and with the outside world. In some embodiments, tools are made available to the modules as agent builder blocks. Some tools may be general-purpose, and others may be custom for a particular integration. Different agent module types may have different access to tools: for example, a langchain agent may be configured with a set of available tools, and the model may be configured to choose when and how to use them, whereas a langchain chain may follow a fixed sequence of steps. In some embodiments, an agent configuration defines when and how tools are invoked. As an example, a tool may be configured with a fixed base URL so that the agent cannot make authentication requests to some other service. In some embodiments, a tool is configured to use an end-user's access token to authenticate, rather than granting an access role to the agent's machine user. In some embodiments, a tool is restricted to certain endpoints and/or methods (e.g., only GET requests) so that the tool is restricted from performing admin tasks on behalf of a user who lacks admin privileges (e.g., write permissions).

In some embodiments, a tool has parameters that are specified when configuring the agent modules and/or parameters (e.g., the parameters 234) that can be specified at invocation time by the agent module itself. An example tool is an authentication request tool configured to fetch an internal URL using a user's access token. The authentication request tool may include the following parameters: name, description, base URL, and/or input parameters (e.g., specifiable by the agent). For example, an example authentication request tool may have an order identifier as an input parameter. Another example tool is an external request tool that fetches an external URL. The parameters for the external request tool may include name, description, base URL, and/or input parameters. Another example tool is an email tool that sends an email. The parameters for the email tool may include destination, subject, and/or body. Other example tools include a verification/validation tool to which information received from an external request are passed for validation prior to other processing. For example, an incoming message is confirmed against expected content and/or parsed/mined to ensure no malicious prompt injection is involved before being eligible for further analysis and response.

Example task-specific agents modules include (i) an agent module configured to send emails summarizing which customers are facing issues with orders and/or identifying retraining opportunities, (ii) an agent module configured to generate data tables, JSON schema, and other data translations, (iii) an agent module configured to find orders within a group of clients that have particular flags and/or provide a summary by client, flag, etc. (e.g., with timestamp for order creation timing), (iv) an agent module for identifying behavioral changes in ordering habits and adjust orders accordingly (e.g., increase delays and/or cancel orders) and sending notifications, (v) an agent module for generating inclusion/exclusion criteria from a protocol document, generating structured queries (e.g., SQL queries) from a structured list, and/or generate specifications (e.g., YAML specifications) from structured lists of inclusion/exclusion criteria, (vi) an agent module for answering questions about particular trials based on information in the protocol and/or other trial materials or documentation, (vii) an agent module for identifying patient cohorts based on researcher information, (viii) an agent module for identifying research related to a patient cohort, (ix) an agent module for identifying research gaps from a set of research documents, (x) an agent module for determining insights from research information, and (xi) an agent for generating research publications, such as outlines, abstracts, data visualizations, posters, and the like.

As an example, an agent module 227 configured to identify and/or evaluate adverse effects receives a user query regarding adverse effects associated with a particular drug. In this example, the agent module 227 parses the query in order to identify the drug name from the query and applies the drug name to one or more nodes 232 in order to obtain a set of adverse effects associated with the drug. In this example, the agent module 227 provides a response with a description of the set of adverse effects.

In some embodiments, a type or classification of agent module 227 is selected for a specific task based on an analysis of a set of different types or classifications. In some embodiments, the analysis includes comparing label-dependent spectra from the output of a pretrained model 228. For example, the comparison may be performed using a Jensen-Shannon (JS) divergence of a principal component analysis (PCA) decomposed output spectra. In some circumstances, models 228 that are better suited for a downstream task have a larger JS divergence. JS divergence is described in Menendez et al., 1997, "The Jensen-Shannon divergence," Journal of the Franklin Institute 334(2), pp. 307-314, which is hereby incorporated by reference in its entirety for all purposes. Additionally, some models 228 have greater information capacity at intermediate layers. The greater information capacity may be determined by measuring the dimensionality of the PCA reduced spectra coming from the output of the layer. In some embodiments, a decomposed spectra selector of pretrained models is configured to perform the above analysis.

One of ordinary skill in the art will appreciate there is a large number of pretrained and fine-tuned deep language models (DLMs) available. However, the performance of each model for a downstream fine-tuning task can vary greatly. Therefore, a process (e.g., a heuristic) for model selection can save time and energy, compared to training several models and choosing the most performant one afterwards.

In some circumstances, models that are better fit for the downstream task are better at separating data according to the label of each respective datapoint. This can be seen by examining the label dependent statistics in the output of the task dependent output head. When downstream training has not occurred, this can still be done by examining the label dependent spectra of the data coming from the output of the pretrained model. A useful metric for determining the label-dependent spectra separation is the JS divergence. Often the spectra is multidimensional, so the JS divergence can be calculated and summed along the dimensions of the spectra. This can be problematic because high dimensional outputs have an innate advantage simply because of the larger number of dimensions contributing to the sum. Not only does naive JS divergence favor higher dimensional outputs, it also does not account for intra-output correlations.

To circumvent this issue, the spectra can be decomposed into its first N principal components necessary to account for 99% cumulative explained variance ratio, where N is a positive integer. In some embodiments, N is 1, 2, 3, 4, 5, 6, 7, 8, 9, 10, between 10 and 20, between 20 and 50, between 10 and 100, or between 1 and 1000. Pretrained DLMs with higher PCA-reduced JS divergence can lead to better downstream classification performance because they have an innate edge in discriminating label dependent data. For example, there is a correlation between the PCA-reduced JS divergence and the macroscopic F1 of the DLM against the test data. Thus, in some scenarios, the choice of pretrained model has a large impact on the final performance.

The macroscopic F1 score for the DLM against the test data may be derived from precision and recall of the DLM. Precision is the accuracy of positive predictions made by the DLM. It can be considered the ratio of true positive (TP) predictions to the total number of positive predictions (true positive + false positive). Recall (also sometimes called sensitivity or true positive rate) measures the ability of the DLM to correctly identify positive instances. It can be considered the ratio of true positive predictions to the total number of actual positive instances (true positive + false negative). The F1 score can then be calculated as the harmonic mean of precision and recall:

$$F1 = \frac{2 \; x \; precision \; x \; recall}{precision + recall}$$

Equation 1 – F1 Score for DLMs

The macroscopic F1 score can be obtained by calculating the F1 score for each class separately and then averaging the F1 scores across all classes. It treats each class equally and is useful when there are imbalanced datasets with varying class distributions. In this way, the macroscopic F1 score provides a more comprehensive evaluation of the DLM's performance by considering its ability to classify all classes correctly, not just the majority class.

The correlation between the PCA-reduced JS divergence and the macroscopic F1 of the test data holds for an image modality as well. In the computer vision domain and modality, greater PCA-reduced JS divergence of the model output spectra indicates better downstream performance. Additionally, in some scenarios, the layer that yields the highest PCA dimension leads to the strongest results. For example, a full example model when trained yielded a macroscopic test F1 score of 0.76, and the same model with layers only up to layer 8 (e.g., maximum PCA dimension) yielded a macroscopic test F1 score of 0.86, which is considerably better.

In some embodiments, an analysis of the PCA-reduced JS divergence is used for (i) selecting the appropriate agent module 227 and/or model 228 for a specific task (e.g., selecting the appropriate agent module), (ii) identifying the appropriate dimensionality of the agent module 227 and/or model 228 (e.g., reduced dimensionality), (iii) optimizing one or more node parameters of the agent module 227 and/or model 228 for best use (e.g., layer selection), (iv) optimizing one or more inputs of the agent module 227 and/or model 228 (e.g., which combination of inputs provide the best early divergence), (v) creating embeddings to identify if combinations of the agent module 227 and/or model 228 that are beneficial, (vi) pruning the agent modules 227 and/or models 228 deterministically, or (vii) a combination thereof. For example, optimizing the agent module 227 and/or model 228 configuration for best use may include selecting the node and/or layer of the model with highest dimension after reduction.

As an example scenario, a user obtains a set of labeled data with which to train a classifier. The user may split the data into train, validation, and test data sets. A set of pretrained agent modules 227 and/or models 228 are identified by a first agent module 227 that may fit to the task (e.g., transformers, convolutional neural networks, and/or recurrent neural networks). Each of the agent modules 227 and/or models 228 may be run over the validation set of data and the spectra from the last layer of the pretrained model and/or agent module may be examined (e.g., before entering the classification head). This yields a tensor of shape (N, D) where N is the number of examples in the validation set, and D is the dimensionality of the output from the last layer of the pretrained model and/or agent. To remove linear dependence, a 99% PCA reduction may be applied on the output that yields a new tensor of shape (N, D_pca). The JS divergence may be calculated between the class labels (e.g., in a one-vs-rest fashion for each component and sum). The pretrained agent modules 227 and/or models 228 that yield the highest summed JS divergence may then be selected. The selected agent modules 227 and/or models 228 may correspond to a particular type or classification. In some embodiments, agent modules 227 and/or models 22 in the set of pretrained models has 2, 3, 4, 5, 6, 7, 8, 9, 10, between, 10 and 20, between 20 and 30, or more than 30 nodes and/or layers.

Additionally, a portion of an agent module 227 and/or model 228 may be identified as useful for a particular dataset and/or domain of information, such as a first agent for use in a genome domain, a second agent module for use in a microbiome domain, a third agent for use in an imaging domain, a fourth agent module for use in a drug domain, and the like. The spectra from the $0^{th}$ element of each hidden layer may be obtained and a 99% PCA reduction performed and then the dimension may be recorded. For example, the node and/or layer of the agent module 227 and/or model 228 with the largest PCA-reduced dimension may be selected and all nodes and/or layers following the selection may be discarded. The output of the selection may be fed to the classification head (and agent module 227 and/or model 228 fine-tuning can then be performed).

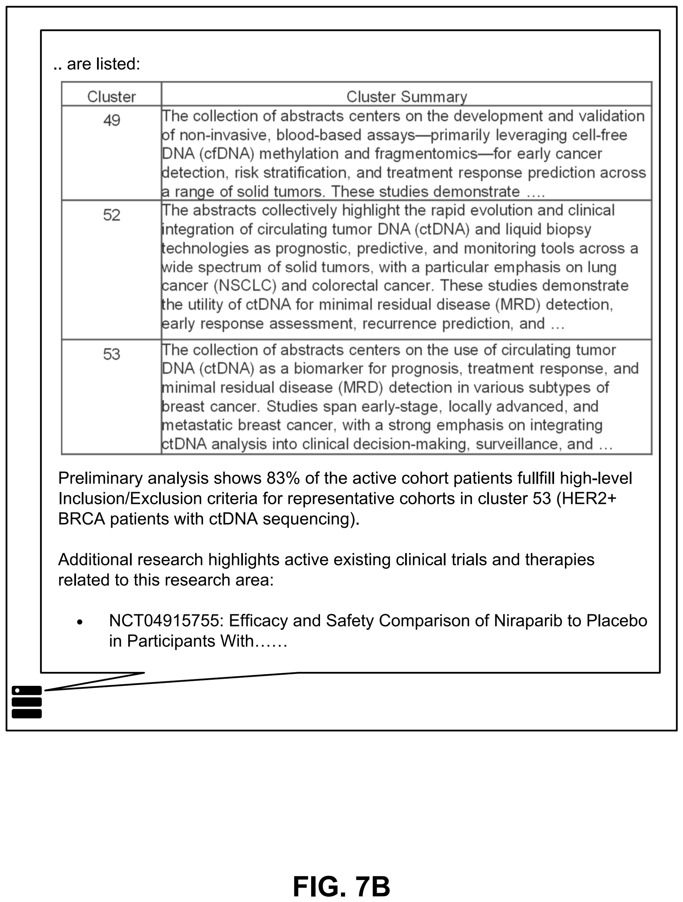

Figures 7A and 7B illustrate example user interfaces for interacting with a digital assistant in accordance with some embodiments. Figure 7A illustrates an example user interface configured to surface research insights based on an entity's current cohort and a time- and subject-matter-constrained ingestion of external documents. In the example shown, the interface indicates that the current cohort contains a number of patients having circulating tumor DNA (ctDNA) samples and prompts the user to reference applicable research opportunities for that cohort. The user enters a natural language request to compare the cohort to materials from a specified conference cycle (ASCO 2025) and to identify overlapping research areas. In the example of Figure 7A, the system initiates an insight workflow for the specified constraint and reports abstracts from the ASCO 2025 corpus that reference ctDNA. In this example, the interface presents a visualization panel of clustered abstracts, with cluster identifiers and representative keyword sets. In the illustrated example, clusters labeled "49," "52," and "53" are highlighted as most relevant to ctDNA research. In accordance with some embodiments, each highlighted cluster is accompanied by a compact keyword summary. The visualization example in Figure 7A conveys how the system's clustering organizes conference abstracts into semantically coherent groups and allows the user to preview which clusters align to the entity's cohort attributes, thereby guiding downstream action generation and ranking.

Figure 7B presents a tabular summary view corresponding to the clusters identified in Figure 7A, along with preliminary cohort alignment and related follow-up items, in accordance with some embodiments. The table lists cluster identifiers (e.g., 49, 52, 53) and provides narrative cluster summaries that describe prevailing themes within each group of abstracts. For example, the summary for cluster 49 indicates development and validation of non-invasive, blood-based assays (e.g., leveraging cell-free DNA methylation and fragmentomics) for early cancer detection, risk stratification, and treatment response prediction across multiple solid tumors. The summary for cluster 52 highlights integration of ctDNA and liquid biopsy technologies as prognostic, predictive, and monitoring tools across solid tumors, with emphasis on non-small cell lung cancer and colorectal cancer, and utility in minimal residual disease detection and early response assessment. The summary for cluster 53 describes use of circulating tumor DNA as a biomarker for prognosis, treatment response, and minimal residual disease in breast cancer subtypes, including locally advanced and metastatic disease, with focus on clinical decision-making and surveillance.

In addition to the table, the example interface of Figure 7B also provides an example preliminary cohort analysis indicating the proportion of active cohort patients that satisfy high-level inclusion/exclusion criteria representative of a selected cluster (e.g., cluster 53, HER2-positive BRCA patients with ctDNA sequencing). In accordance with some embodiments, the view further lists related clinical trials or therapies aligned to the summarized research area, such as a trial identified by its NCT number, to facilitate immediate follow-up actions (e.g., literature synthesis, cohort feasibility queries, and trial landscape comparisons). Figures 7A and 7B demonstrates how cluster-level summaries, cohort overlap metrics, and provenance-linked trial references may be presented in a structured format that supports action identification, ranking, and notification workflows. The cohort overlap may be determined based on cluster summary similarities, genomics, EHR information, and/or imaging data. For example, similarity may be determined based on one or more modalities and/or datasets. Further details regarding these aspects are discussed later in this application (e.g., see aspect E7).

As shown in Figures 7A and 7B, the digital (AI) assistant may be used to identify research gaps and candidate hypotheses to pursue. In some embodiments, the digital assistant is communicatively coupled to and/or trained on an embedding/vector database. For example, the digital assistant can obtain a constraint from the user (as illustrated in Figure 7A) indicating a period of time and a type of subject matter (e.g., a recent conference cycle within a specified oncology indication) and, using the constraint, ingest a set of research documents and corresponding metadata that were not previously included in a database corresponding to the digital assistant. The digital assistant may generate numerical embeddings for the newly ingested documents, update similarity clusters to incorporate the new embeddings, and derive metadata-aware cluster summaries that expose prevailing themes and their temporal trajectories. The digital assistant may obtain entity information for a user or institution, including approved workflows and available datasets, and identify potential actions by linking the updated clusters to the entity's capabilities, such as proposing targeted literature syntheses, cohort queries, trial landscape comparisons, or feasibility analyses. Potential actions may be generated/identified, and the digital assistant may formulate corresponding testable hypotheses (e.g., biomarker and outcome associations or underexplored subpopulation effects). The actions may be ranked by multi-criteria scoring. The scoring may account for one or more of expected impact, regulatory feasibility, data availability within the entity's datasets, and estimated effort/cost. In some embodiments, the assistant provides a notification indicating top-ranked gaps and hypotheses with supporting provenance, enabling the user to advance from discovery to validation within an integrated workflow.

As discussed above, in some embodiments, the digital assistant is a digital research assistant. In some embodiments, the digital research assistant is configured to integrate with existing workflows, such as JupyterLab with Python and RStudio with R, thereby enhancing user experience and productivity. In some embodiments, the digital assistant is configured to interact with the user to provide information and instructions for pursuing a research topic or follow-up action. For example, the digital research assistant may leverage AI capabilities to identify and analyze cohorts of African American patients who are often underrepresented in clinical trials. The digital research assistant allows researchers to focus on specific genetic markers such as epidermal growth factor receptor (EGFR) mutations and clinical characteristics unique to this group, thereby ensuring that the research is both inclusive and comprehensive. In some embodiments, identifying a cohort involves a single, detailed query from the user or involve a multi-step process of fine-tuning the query from the user to include additional details based on the response(s) from and to the digital research assistant. In some embodiments, identifying a cohort involves querying and analyzing the cohorts of clinical trials to better identify representation and characteristics for research.

As an example, the digital assistant may identify a cohort of African American patients with Stage III non-small cell lung cancer (NSCLC) who have specific genetic alterations such as ALK rearrangements. By analyzing this cohort, researchers can better understand how these genetic factors influence treatment outcomes and tailor their research to address these unique needs. Additionally, researchers and the digital research assistant can generate high-quality drafts of publications and posters that highlight these findings, such as a detailed figure showing the survival rates of African American patients with ALK-positive NSCLC compared to other ethnic groups. This may involve agents and/or analytic models trained, tuned, and/or prompted to (1) generate outcome analytics; (2) generate abstract, poster, or publication reviews for specific conferences in recent years to identify trends and outlines for specific research subject matter; and/or (3) orchestrate the drafting based on the identified cohort, associated analytics, including any derived by agent or model, and populating the respective data and imagery into a respective abstract, poster, publication or the like based on the outline of the research subject matter. In some embodiments, a single model implements multiple steps of the step-wise process in a single iteration, such as drafting the research publication materials based on a conference and the gathered data in a single step rather than first generating a template and then filling the template.

As another example, the digital research assistant may allow users to explore the impact of socioeconomic factors on lung cancer treatment outcomes. For instance, the digital research assistant can identify a cohort of patients from low-income backgrounds who have limited access to healthcare and analyze how these factors affect their response to immunotherapy. By integrating data from external sources, the digital research assistant can uncover new research opportunities that address these disparities and generate actionable insights to improve patient care. This process may involve one or more agents for (i) cohort generation, (ii) external database querying, and (iii) performing analytics and inferences on the combined results of multiple agents. In some embodiments, a single agent may approach the research query in reverse, and first identify disparities across a specified field of research based on cohorts and report cohorts with disparities to the user for further action.

As another example, the digital research assistant may identify gaps in existing research, such as the lack of studies on the impact of environmental factors on lung cancer incidence in urban African American communities. By querying external data sources and integrating multimodal data, the digital research assistant can indicate studies that investigate the correlation between air pollution exposure and lung cancer risk in these populations. This allows researchers to focus on uncovering insights that can drive meaningful improvements in health outcomes for all patients, particularly those from underserved communities. This process may involve one or more agents for (i) data transformation (e.g., using techniques such as image recognition, text recognition, and fuzzy matching), (ii) concept matching (e.g., using techniques such as categorization, fuzzy matching, template matching, and nearest neighbor matching), and (iii) deficiency identification (e.g., using techniques such as contradiction identification, gap identification, and next steps identification).

By providing intelligent cohort suggestions, automating the creation of research artifacts, and identifying new research opportunities, the digital research assistant allows users to conduct more inclusive and impactful studies that address the unique needs of a community. Thus, the digital research assistant can significantly accelerate the research process for researchers, leading to a reduction in the time required to draft research publications and posters. This acceleration results in a higher volume of high-quality research outputs, improved identification of relevant patient cohorts and enhanced discovery of new research opportunities, thereby driving more impactful scientific discoveries and improved patient outcomes. For example, by automating the generation of drafts for publications and posters, the digital research assistant can free up valuable time for researchers, allowing them to focus on high-impact activities such as data analysis and hypothesis generation. Reducing the time and effort required for drafting research documents optimizes the use of resources, enabling researchers to produce more outputs with the same or fewer resources.

From a technical standpoint, the described processes yield measurable system-level improvements. Embedding heterogeneous inputs into a shared latent space and maintaining temporally adaptive similarity clusters increases recall and precision over keyword baselines, enabling stable retrieval as terminology evolves. Metadata-aware chunking and ontology normalization reduce schema drift and cross-vendor variance, improving match quality and lowering false positives in cohort and trial linking. Online cluster updates and multi-criteria action ranking shorten feedback loops by supporting stream ingestion and incremental upserts, which reduces end-to-insight latency. Provenance tagging and uncertainty calibration enhance auditability and trust by exposing source citations and confidence indicators alongside recommendations. Finally, stateful personalization and role-based access controls minimize irrelevant notifications and redundant computation, lowering cognitive load while reducing compute and I/O overhead through targeted retrieval and in-context execution.

Multiple workflows may be used to traverse a user through their research, e.g., from a hypothesis stage to an analytics stage then to a publication stage. For example, a first example workflow assists a user who knows the characteristics of a cohort that they want to research, but needs assistance in clearing the technical hurdles of crafting a database query for the desired cohort and confirming their research hypothesis via analytics on the cohort for specific criteria. A set of agents may tune the cohort characteristics into a query, then build a code base for performing and reporting the analytics back to the user (e.g., before reiterating through the process or advancing to a next workflow of reporting the findings).

A second example workflow assists a user who has a specific hypothesis of research in mind but does not know which cohort to analyze to perform analytics on to evaluate the hypothesis, such as a pharmaceutical researcher who wants to find off-label cohorts that may respond well to a specific treatment. A set of agents may generate/identify a cohort of all subjects having a favorable treatment and those not having the treatment to identify populations within the cohort which overlap on characteristics between those who respond favorably and those who have not taken the treatment. The researcher may select all of the resulting populations within the cohort for further analytics or select a specific population and craft a cohort search query for them (e.g., by hand or via an agent) before advancing to reporting the findings (e.g., using a second set of agents).

A third example workflow assists a user who neither knows what they wish to research nor which cohort of patients on which to research to mine existing clinical trials and/or research abstracts, posters, publications or the like for trends which may be investigated in a new domain or for gaps. A set of agents may summarize research within a time period and field for consumption by the user, or iterate through multiple rounds of summarization until the user has settled on a hypothesis of interest, before (i) mapping that hypothesis to a specific cohort and evaluating the hypothesis via analytics, (ii) advancing to reporting on the existence of the region of interest and an area for further research due to population prevalence and the identified gap, and/or (iii) reporting on the region of interest as applied to the specific cohort and evidenced in analytics on the hypothesis.

By creating/maintaining a robust collection of agents capable of working from any starting point between raw data, analytics, and the review of available data, many different workflows are supported to assist any user with their research objectives. Automated creation (e.g., figure creation, abstract creation, poster creation, and/or publication creation) and intelligent cohort identification as described herein improves consistency and accuracy in research outputs, reducing the likelihood of errors and enhancing the overall quality of publications and posters. Additionally, integrating multimodal data sources provides comprehensive insights, enabling researchers to make more informed decisions and produce more robust and impactful studies.

In some embodiments, the digital assistant is composed of an end-to-end model (e.g., trained on multiple disciplines and modalities). In some embodiments, the digital research assistant is composed of a set of task-specific orchestrations. For example, using a complex network of agents (e.g., AI agents) in a multiple layered network to streamline and accelerate research processes. As discussed previously, the digital research assistant can identify discovery question using a network of task-specific orchestrations (e.g., agents). An example, discovery process uses four (4) steps. First, the digital research assistant utilizes a platform (e.g., the platform 100) to integrate with a set of databases (e.g., the database(s) 400), to identify and access patient cohorts relevant to a research hypothesis. Next, the digital research assistant applies statistical analytics to determine significant findings and generate visual graphic options. Third, the digital assistant compares and associates those findings to established peer-viewed literature. Fourth, the digital assistant collates the information, graphs, and associated peer referenced information to generate drafts for research abstract, posters and manuscript publications, thereby facilitating the retrieval of information into a streamlined process.

Multiple workflows may be used to prepare a publication stage of research, e.g., upon the completion of one or more of the workflows above, or upon request by a user able to identify or provide a requisite starting base for the publication stage. A first example workflow assists a user with generating a template (e.g., outline) to follow for a publication. A set of agents may identify, and/or suggest, a type of publication for the user for selection and then generate a corresponding template. The set of agents may allow the user to fill the template themselves, and/or advance to another workflow to generate data that populates the template and/or walk the user through the steps of generating the data to populate the template. In another example, a set of agents may first identify, or suggest, a conference type to which the researcher may be interested in presenting, e.g., before generating a more tailored template based on the trends, standards, and/or guidelines of the respective conference. In some embodiments, the conference type is identified before identifying or suggesting a type of publication to the user for selection (and before generating and/or filling a template for the publication). In another example, a set of agents may ingest the provided materials from the user to identify one or more conferences and eligible publication types for that conference based on the materials available (e.g., before identifying and/or selecting a type of publication). In some embodiments, the one or more conferences and eligible publication types are identified before generating and/or filling a template for the publication.

Various sets of the agents described herein may be used to generate publication templates (e.g., for all eligible types of publications based on the available information and without first requesting which one a user prefers). Various sets of the agents described herein may be used to parse available information from previous workflows and/or supplied materials to identify missing information needed to support and generate a more detailed type of publications, e.g., before walking the user through addressing the missing information and generating the publication template or draft.

Various sets of agents may identify when available information is sufficient to generate a particular type of publication (e.g., a poster) but not sufficient to generate another type of publication (e.g., an abstract or paper). Various sets of agents may select an appropriate representation of imaging (e.g., imaging type and/or content) to be included in the publication and/or suggest imaging to the user along with reasoning for why each imaging should be included to the respective publication. In this way, the agent(s) enable a user to select between using tables or charts or advanced modeling and analytics to display their research findings in the publication.

In some embodiments, an end-to-end model performs the above tasks without delegating to separate agents. The model ingests heterogeneous inputs, learns shared representations via various encoders, and identifies information gaps, proposes eligible publication types, selects appropriate visualizations, and drafts the targeted artifact. A unified conditioning vector may be used to encode user preferences, project context, institutional constraints, and/or target venue guidelines so the same network can adapt output structure and tone. Trained under a joint objective that combines semantic fidelity, metadata prediction, visualization suitability, and formatting compliance, the model may output a complete, provenance-linked draft with recommended figures and tables, along with confidence indicators and editable sections, thereby reducing latency, minimizing error propagation across tool handoffs, and improving consistency across publication workflows.

In some embodiments, the end-to-end model is provided with numerical vectors derived from the shared latent space together with a prompt tailored to elicit the desired output. In some embodiments, the prompt includes a constraint (e.g., a subject matter and/or time constraint). In some embodiments, the prompt encodes task intent, target artifact type, required sections, citation style, visualization preferences, and/or constraints corresponding to user or institutional policies. In some embodiments, the numerical vectors include cluster centroids, representative document embeddings, metadata conditioning vectors, and/or user or project embeddings. These numerical vectors may collectively ground the model's generation on the most relevant evidence. In some embodiments, the tailored prompt references the input data corresponding to the vectors and includes retrieval instructions, summarization granularity, hypothesis framing, and/or action ranking objectives so that the model aligns its decoding to the specified structure and tone. In some embodiments, the model uses the combined prompt and vector inputs to provide text output, assemble an evidence-linked draft, and/or generate figures and tables consistent with visualization preferences.

In some embodiments, a model (e.g., the end-to-end model) has access to, is trained on, and/or fine-tuned with multimodal data (e.g., the data illustrated in Figure 4). The multimodal data may include DNA data, RNA data, epigenetics data, organoids data, clinical imaging data, clinical EMRs, clinical notes, test results, cellular data, claims data, pathology data, and the like. In some embodiments, the multimodal data includes millions of de-identified records, imaging data, clinical data, and/or profiles. Clinical data may include demographics data (e.g., age, gender, race, ethnicity, etc.), diagnosis data (e.g., primary site, date of diagnosis, histology, etc.), treatments data (e.g., procedure details, medication details, etc.), outcomes data, claims data, and/or assessments data. In some embodiments, molecular and imaging data includes DNA sequencing data, RNA sequencing data, molecular pathology data, imaging data, and/or IO data.

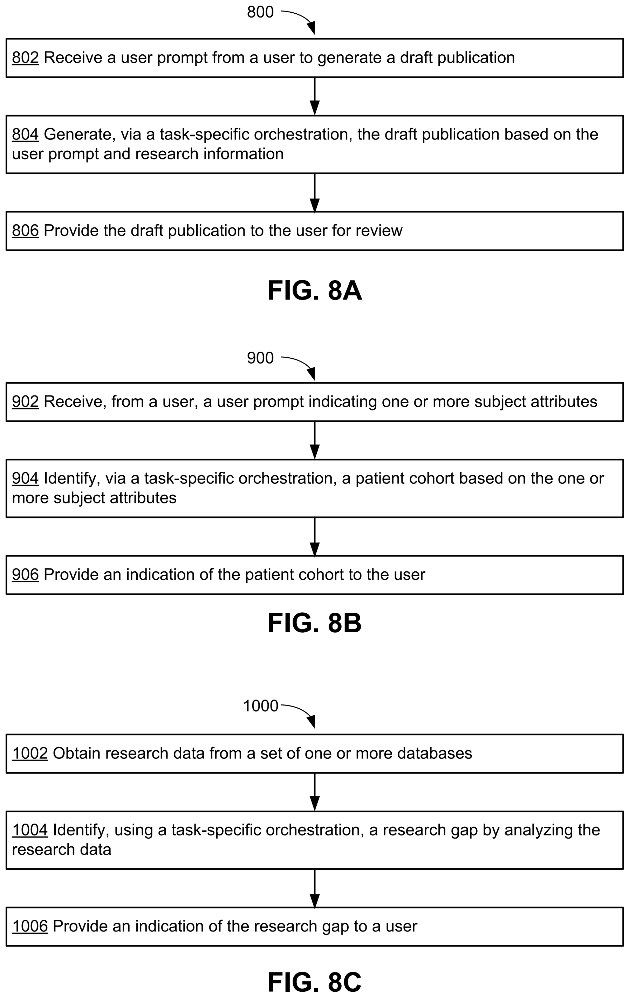

Figure 8A is a flow diagram illustrating a method 800 for generating draft publications in accordance with some embodiments. The method 800 is performed at a computing system (e.g., a client device, server system, and/or service platform) having one or more processors (e.g., the CPUs 202 and/or 302) and memory (e.g., the memory 218 and/or 310). In some embodiments, the memory stores one or more programs configured for execution by the one or more processors.

At least some of the operations shown in Figure 8A correspond to instructions stored in a computer memory or a computer-readable storage medium. In some embodiments, the computing system is the platform 100, the client device(s) 102, and/or the server system 106.

(A1) In one aspect, some embodiments include the method 800 being performed at a computing system. For example, the method 800 may be performed at an application of the client device 102 associated with the platform 100. In some embodiments, the computing system comprises a set of task-specific orchestrations. The computing system receives (802) a user prompt from a user to generate a draft publication. The computing system generates (804), via a task-specific orchestration, the draft publication based on the user prompt and research information. The computing system provides (806) the draft publication to the user for review. In some embodiments, the computing system causes the draft publication to be displayed to the user. In some embodiments, the computing system causes the draft publication to be displayed in a word processing application.

(A2) In some embodiments of A1, generating the draft publication comprises generating one or more data visualizations (e.g., imaging) based on the research information. For example, the one or more data visualizations may include a bar chart, a pie chart, a heatmap, a line chart, a scatter plot, an area chart, a histogram, a treemap, a boxplot, a graph, a word cloud, or the like. In some embodiments, the task-specific orchestration determines a type of data visualization to generate based on the underlying data, the type of publication, and/or one or more user preferences.

(A3) In some embodiments of A1 or A2, at least a portion of the research information is uploaded by the user in conjunction with providing the user prompt. For example, the user may upload test results along with a request to generate the draft publication. In some embodiments, the user includes research parameters and/or drafting parameters in the user prompt.

(A4) In some embodiments of any of A1-A3, at least a portion of the research information is obtained from a database. For example, the research information is obtained from a database maintained by the user. In some embodiments, the research information includes information from the user and information from one or more additional databases.

(A5) In some embodiments of any of A1-A4, the computing system identifies one or more key findings for the draft publication. In some embodiments, the key findings are identified based on a hypothesis provided by the user. In some embodiments, the key findings are identified based on abnormalities identified in the research data.

(A6) In some embodiments of A5, the one or more key findings are identified by the task-specific orchestration (e.g., an agent or agent module). In some embodiments, the one or more key findings are identified using a second task-specific orchestration. For example, the first task-specific orchestration may be configured (e.g., trained) to generate draft publications, and the second task-specific orchestration may be configured (e.g., trained) to identify key findings (e.g., abnormalities and results relevant to an identified hypothesis).

(A7) In some embodiments of A5 or A6, the computing system generates one or more insights by comparing the one or more key findings with peer-referenced information. For example, the first or second task-specific orchestrations may be configured to generate the insights. In some embodiments, a third task-specific orchestration may be configured to generate the insights.

(A8) In some embodiments of A7, the comparison is performed by the task-specific orchestration. In some embodiments, the comparison is performed by a second task-specific orchestration. For example, the second task-specific orchestration may be configured (e.g., trained) to compare results in the draft publication with peer-referenced information, e.g., to identify similarities and differences.

(A9) In some embodiments of any of A1-A8, the draft publication comprises a research paper. For example, the draft publication is generated to comply with research paper requirements (e.g., requirements regarding formatting, citations, and the like). In some embodiments, the draft publication comprises a portion of a research paper (e.g., an outline and/or an abstract).

(A10) In some embodiments of any of A1-A9, the draft publication comprises a poster. For example, the draft publication may include a research paper and corresponding poster.

(A11) In some embodiments of any of A1-A10, the task-specific orchestration comprises a machine-learning (ML) model. For example, the ML model is a transformer model, a large language model (LLM), or other type of long attention model.

(A12) In some embodiments of any of A1-A11, the user prompt comprises a natural language input. In some embodiments, the user prompt includes a natural language query from the user and context information.

Figure 8B is a flow diagram illustrating a method 900 for identifying patient cohorts in accordance with some embodiments. The method 900 is performed at a computing system (e.g., a client device, server system, and/or service platform) having one or more processors (e.g., the CPUs 202 and/or 302) and memory (e.g., the memory 218 and/or 310). In some embodiments, the memory stores one or more programs configured for execution by the one or more processors. At least some of the operations shown in Figure 8B correspond to instructions stored in a computer memory or a computer-readable storage medium. In some embodiments, the computing system is the platform 100, the client device(s) 102, and/or the server system 106.

(B1) In one aspect, some embodiments include the method 900 being performed at a computing system. For example, the method 900 may be performed at an application of the client device 102 associated with the platform 100. The computing system receives (902), from a user, a user prompt indicating one or more subject attributes. The computing system identifies (904), via a task-specific orchestration, a patient cohort based on the one or more subject attributes. The computing system provides (906) an indication of the patient cohort to the user. In some embodiments, the patient cohort is identified using a cohort builder.

(B2) In some embodiments of B1, the patient cohort is identified based on data from a set of one or more databases. In some embodiments, the patient cohort is identified based on characteristics identified by the user (e.g., demographics, categories, medications, conditions, and the like). In some embodiments, the patient cohort is identified based on information about the user (e.g., research fields, current projects, identified interests, and the like).

(B3) In some embodiments of B1 or B2, the data comprises multi-modal data. In some embodiments, the multi-modal data comprises one or more of: a structured text modality, an unstructured text modality, a tabular data modality, a data visualizations modality, an image modality, an audio modality, a video modality, a biological sequence modality, a natural language modality, and a source code modality. In some embodiments, the plurality of modalities includes a first modality for a first type of images (e.g., x-ray images) and a second modality for a second type of images (e.g., ultrasound images). In some embodiments, the plurality of modalities correspond to different parts of a patient record.

(B4) In some embodiments of any of B1-B3, the patient cohort is identified based on the one or more subject attributes and information about the user. For example, the patient cohort is identified based on patient attributes input by the user as well as context information about the user (e.g., research being performed by the user).

(B5) In some embodiments of B4, the information about the user is obtained from a user profile of the user. In some embodiments, the information about the user comprises information about past actions taken by the user. In some embodiments, the information about the user comprises information about research being performed by the user.

(B6) In some embodiments of any of B1-B5, the computing system provides one or more insights about the patient cohort based on information about the user. For example, the task-specific orchestration provides insights about how the patient cohort is related to research being performed by the user. In some embodiments, the insights include identifying research topics, research gaps, hypotheses, and/or populations of interest.

Figure 8C is a flow diagram illustrating a method 1000 for identifying research gaps in accordance with some embodiments. The method 1000 is performed at a computing system (e.g., a client device, server system, and/or service platform) having one or more processors (e.g., the CPUs 202 and/or 302) and memory (e.g., the memory 218 and/or 310). In some embodiments, the memory stores one or more programs configured for execution by the one or more processors. At least some of the operations shown in Figure 8C correspond to instructions stored in a computer memory or a computer-readable storage medium. In some embodiments, the computing system is the platform 100, the client device(s) 102, and/or the server system 106.

(C1) In one aspect, some embodiments include the method 1000 being performed at a computing system. For example, the method 1000 may be performed at an application of the client device 102 associated with the platform 100. The computing system obtains (1002) research data from a set of one or more databases. The computing system identifies (1004), using a task-specific orchestration, a research gap by analyzing the research data. The computing system provides (1006) an indication of the research gap to a user. For example, the computing system may identify new research opportunities by analyzing gaps in existing studies and external publications. In some embodiments, the one or more databases include a client (third-party) database. The database(s) may comprise a medical database, a patient database, and/or a treatment database (e.g., the database(s) 400). In some embodiments, the one or more databases comprise a client database, an external database, and/or a third-party database. In some embodiments, the research gap is identified by analyzing information about one or more patient cohorts.

(C2) In some embodiments of C1, providing the indication comprises providing one or more actionable insights to the user. For example, providing personalized cohort suggestions relevant to a researcher's needs. In some embodiments, providing one or more actionable insights to the user comprises providing further research suggestions, patient cohort search suggestions, and/or publication/conference suggestions.

(C3) In some embodiments of C1 or C2, the research data comprises one or more documents written in a first language and one or more second documents written in a second language. In some embodiments, the computing system is configured to provide results (e.g., the indication of the research gap) in the first language (e.g., based on a user preference and/or a language of a user input).

(C4) In some embodiments of any of C1-C3, the computing system translates the one or more second documents from the second language to the first language. For example, the task-specific orchestration may be used to translate the one or more second documents. In some embodiments, a second task-specific orchestration is used to translate the one or more second documents.

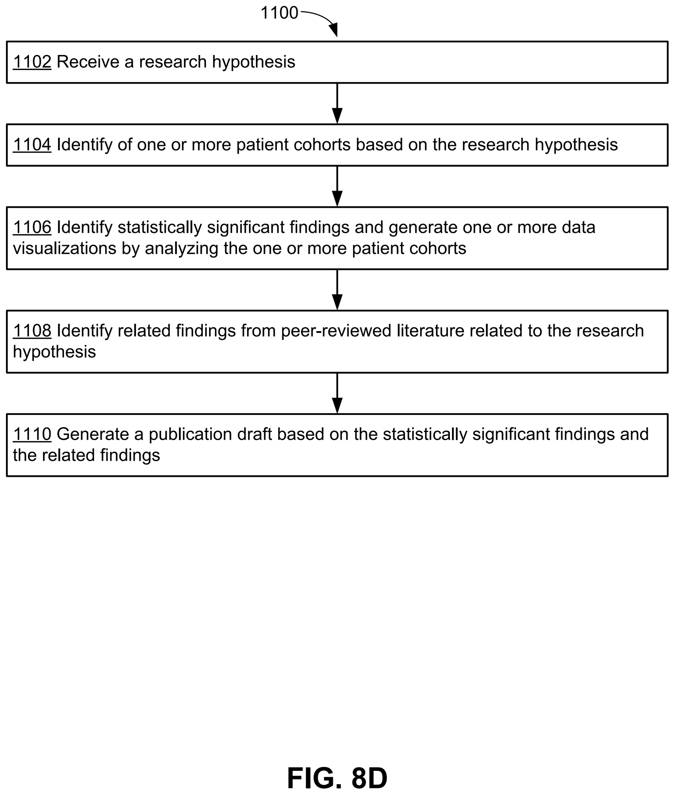

Figure 8D is a flow diagram illustrating a method 1100 for generating draft publications in accordance with some embodiments. The method 1100 is performed at a computing system (e.g., a client device, server system, and/or service platform) having one or more processors (e.g., the CPUs 202 and/or 302) and memory (e.g., the memory 218 and/or 310).

In some embodiments, the memory stores one or more programs configured for execution by the one or more processors. At least some of the operations shown in Figure 8D correspond to instructions stored in a computer memory or a computer-readable storage medium. In some embodiments, the computing system is the platform 100, the client device(s) 102, and/or the server system 106.

(D1) In one aspect, some embodiments include the method 1100 being performed at a computing system. For example, the method 1100 may be performed at an application of the client device 102 associated with the platform 100. In some embodiments, the computing system comprises a set of task-specific orchestrations. The computing system receives (1102) a research hypothesis. The computing system identifies (1104) one or more patient cohorts based on the research hypothesis. The computing system identifies (1106) statistically significant findings and generates one or more data visualizations by analyzing the patient cohorts. The computing system identifies (1108) related findings from peer-reviewed literature related to the research hypothesis. The computing system generates (1110) a publication draft based on the statistically significant findings and the related findings.

(D2) In some embodiments of D1, the research hypothesis is received from a user. For example, the research hypothesis is received as a user query or user prompt. In some embodiments, the research hypothesis is received by a task-specific orchestration (e.g., a super agent, a routing agent, or other type of agent).

(D3) In some embodiments of D1 or D2, the one or more patient cohorts are identified based on patient characteristics indicated by the research hypothesis. In some embodiments, the one or more patient cohorts are identified from a patient database. In some embodiments, the one or more patient cohorts are identified using a task-specific orchestration (e.g., a cohort agent).

(D4) In some embodiments of any of D1-D3, the statistically significant findings are identified using a task-specific orchestration. For example, the statistically significant findings are identified using an analysis agent.

(D5) In some embodiments of any of D1-D4, the one or more data visualizations are generated using a task-specific orchestration. For example, the one or more data visualizations are generated using a visualization agent.

(D6) In some embodiments of any of D1-D5, the related findings are identified using a task-specific orchestration. For example, the related findings are identified using research agent, a document analysis agent, or other type of agent. In some embodiments, the related findings are identified from a research database. As an example, the task-specific orchestration may analyze the peer-reviewed literature to determine novelty, utility, and impact of the statistically significant findings. In some embodiments, the task-specific orchestration retrieves and organizes the peer-reviewed literature.

(D7) In some embodiments of any of D1-D6, the publication draft is generated using a task-specific orchestration. In some embodiments, the task-specific orchestration collates data tables, graphs, and literature references to generate the publication draft. In some embodiments, generating the publication draft (also sometimes called a draft publication) includes generating an abstract, a poster, and/or a manuscript.

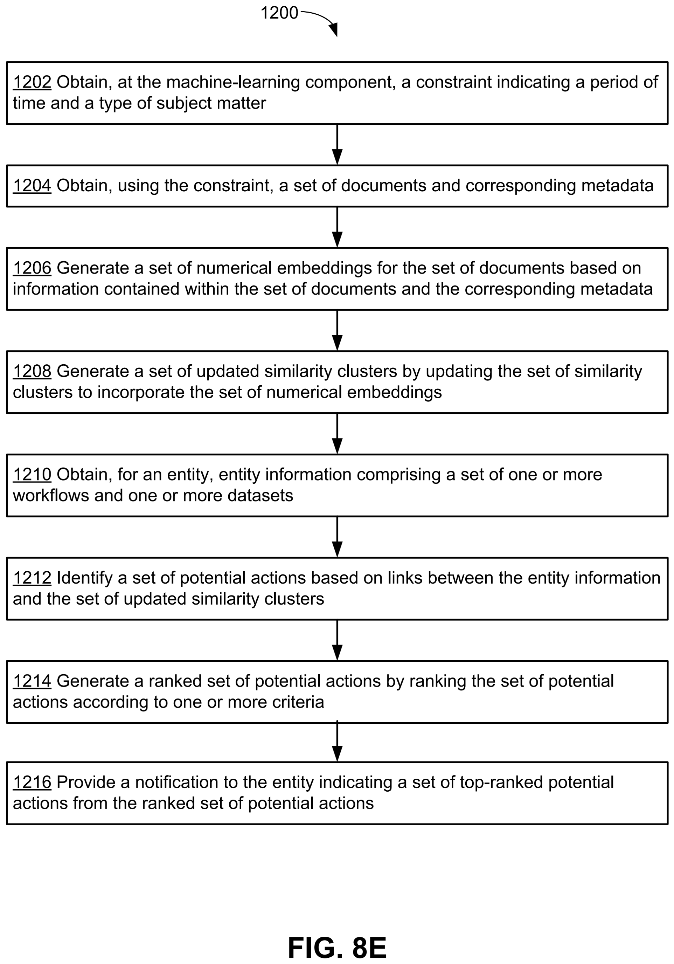

Figure 8E is a flow diagram illustrating a method 1200 for generating ranked sets of potential actions in accordance with some embodiments. The method 1200 is performed at a computing system (e.g., a client device, server system, and/or service platform) having one or more processors (e.g., the CPUs 202 and/or 302) and memory (e.g., the memory 218 and/or 310). In some embodiments, the memory stores one or more programs configured for execution by the one or more processors. At least some of the operations shown in Figure 8E correspond to instructions stored in a computer memory or a computer-readable storage medium. In some embodiments, the computing system is the platform 100, the client device(s) 102, and/or the server system 106.

(E1) In one aspect, some embodiments include the method 1200 being performed at a computing system. For example, the method 1200 may be performed at an application of the client device 102 associated with the platform 100. In some embodiments, the computing system comprises a machine-learning component that includes one or more machine-learning models. In some embodiments, the machine-learning component comprises a pre-trained transformer model. In some embodiments, the machine-learning component comprises a language model (e.g., an LLM). In some embodiments, the machine-learning component comprises a deep neural network. In some embodiments, the machine-learning component maintains versioned embeddings and cluster snapshots to compare changes between adjacent time windows. In some embodiments, the computing system comprises one or more natural language processing (NLP) components. For example, the computing system may include a tokenization, segmentation, and/or vectorization component. in some embodiments, the computing system includes any of the component described herein (e.g., the components described above with respect to Figure 5B).

The computing system obtains (1202), at the machine-learning component, a constraint indicating a period of time and a type of subject matter. The machine-learning component has knowledge of a plurality of numerical embeddings corresponding to a plurality of documents. The plurality of numerical embeddings relates to a set of similarity clusters. In some embodiments, the indicated period of time encompasses all time. In some embodiments, the indicated period of time comprises at least one of a start date and an end date. For example, the period of time may indicate a start date but not end date (e.g., indicating that the end date is the present date). As another example, the period of time may indicate an end date but not start date (e.g., indicating that all documents dated before the end date are to be considered). In some embodiments, the start date and/or end date is inferred from the constraint, context associated with the constraint, and/or other user inputs. For example, the constraint may include a natural language input from a user that indicates the period of time (e.g., language such as "last three years," "since the event," or "new publications"). In some embodiments, the start date is inferred based on a date of a prior search/update. For example, the term "new" in the constraint may be inferred as occurring in a time period after a previously-searched time period. In some embodiments, the plurality of numerical embeddings is generated by the machine-learning component. In some embodiments, the set of similarity clusters is generated by the machine-learning component (e.g., using a similarity metric and/or algorithm). For example, the constraint may specify a rolling thirty day window for oncology abstracts or a multi-year window for cardiovascular imaging studies. In some embodiments, the constraint indicates a specific conference cycle or a journal issue. The subject matter type may include biomarkers, therapies, trial phases, or cohort characteristics. The similarity metric may correspond to a cosine similarity or dot product over embeddings. The clustering algorithm may be HDBSCAN, Louvain community detection over a k-NN graph, or k-means tuned for temporal stability.

The computing system obtains (1204), using the constraint, a set of documents and corresponding metadata, where the set of documents is not included in the plurality of documents. By way of example, the set of documents may include conference abstract booklets in PDF, articles linked to a particular investigator, clinical trial records, payer policy bulletins, company filings, and/or preprints. In some embodiments, the documents are obtained via authenticated APIs, secure file transfer, and/or user uploads. In some embodiments, the corresponding metadata includes authors, sponsors, trial phase, assay type, cohort attributes, timestamps, locations, and identifiers such as NCT numbers.

The computing system generates (1206) a set of numerical embeddings for the set of documents based on information contained within the set of documents and the corresponding metadata. In some embodiments, embeddings are generated using domain specific text encoders such as BioBERT or PubMedBERT, image encoders for pathology or radiology, and tabular encoders for structured clinical fields with learned feature embeddings. In some embodiments, a multimodal encoder with adapters produces a unified vector for each chunk that conditions on attached metadata. In some embodiments, embeddings are augmented with ontology IDs, provenance tags, confidence scores, and/or time decay factors to support downstream temporal analyses.

The computing system generates (1208) a set of updated similarity clusters by updating the set of similarity clusters to incorporate the set of numerical embeddings. By way of example, updated similarity clusters may be formed with online clustering that supports split and merge operations as new evidence arrives. In some embodiments, a streaming k-NN graph (e.g., with periodic community detection) reassigns cluster memberships and ranks cluster centrality. In some embodiments, clusters retain temporal labels and drift measures to quantify emerging themes and waning topics across the indicated period.

The computing system obtains (1210), for an entity, entity information comprising a set of one or more workflows and one or more datasets. Example entities include organizations, persons (e.g., subject matter experts), and associations. In some embodiments, entity information is obtained and compared for multiple entities and respective notifications are provided to the multiple entities based on potential actions identified for each entity. In some embodiments, entity information includes approved workflows such as a cohort builder, trial matcher, analytics pipelines, and reporting templates, along with datasets like de-identified EHR records, genomics panels, imaging archives, and claims repositories. In some embodiments, the entity information encodes role-based permissions, compliance constraints, and/or active projects or grants. In some embodiments, the system compares multiple entities to identify shared opportunities and generates individualized notifications per entity.

The computing system identifies (1212) a set of potential actions based on links between the entity information and the set of updated similarity clusters. By way of example, potential actions may include targeted literature syntheses, cohort queries, trial landscape comparisons, feasibility analyses, hypothesis proposals, partner outreach, and follow up data acquisitions. In some embodiments, actions link clusters to institution specific tools, such as triggering a cohort query or drafting a trial eligibility summary. In some embodiments, actions are filtered by data availability, regulatory feasibility, user preferences, and estimated effort levels. In some embodiments, the potential actions include investigating a particular biomarker, investigating a treatment effect, creating a particular cohort, and/or designing a clinical trial.

The computing system generates (1214) a ranked set of potential actions by ranking the set of potential actions according to one or more criteria. In some embodiments, ranking criteria include expected impact, feasibility within available workflows, data coverage within the entity datasets, estimated effort and cost, time to result, dependency on external partners, and compliance risk. In some embodiments, rankings are defined/refined by user feedback with reinforcement learning or active learning. In some embodiments, the system generates effort and impact scores, rationales, and/or provenance links to assist the entity with evaluating the actions.

The computing system provides (1216) a notification to the entity indicating a set of top-ranked potential actions from the ranked set of potential actions. For example, providing the notification to the entity may comprise sending a message (e.g., an instant message, a text message, an email, or the like) to one or more persons of an organizational entity. By way of example, notifications may be delivered via email, instant message, mobile push, EHR inbox, or a dashboard card with actionable links that open cohort builders, trial matchers, or drafting tools. In some embodiments, notifications include confidence indicators, source citations, and/or one-click options (e.g., a one click option to dismiss, save, or execute the action). In some embodiments, the system batches low priority alerts, suppresses duplicates, and/or provides personalized notification formats to improve engagement and reduce cognitive burden. In some embodiments, the notification is interactive. In some embodiments, the notification includes an option to initiate an action of the set of top-ranked potential actions. In some embodiments, the notifications are interactive panes that present each top-ranked action with a compact rationale, side-by-side comparisons against alternative actions, and/or expandable provenance to review underlying documents. In some embodiments, users may review proposed cohort definitions or trial eligibility summaries inline, adjust parameters such as indication filters or biomarker thresholds, and/or preview estimated effort and impact scores prior to execution. In some embodiments, the notification includes quick actions to launch a cohort query, initiate a literature synthesis, trigger a trial landscape comparison, identify an action for an upcoming event (e.g., conference or talk), and/or route a task to a collaborator with role-based permissions. In some embodiments, users may bookmark or stack actions for later and request a reranking that updates recommendations based on newly provided constraints such as available datasets, timelines, or budget. In some embodiments, threaded feedback within the notification captures approvals, comments, and outcomes, which may be logged to refine future rankings and to provide an auditable trail linking each notification to downstream results.

(E2) In some embodiments of E1, the corresponding metadata is a subset of available metadata for the set of documents, and the corresponding metadata is identified from the available metadata using the constraint. In some embodiments, the metadata comprises one or more of author names, sponsoring companies, biomarker mentions, and therapy mentions. In some embodiments, the corresponding metadata includes trial phase indicators, assay types, cohort attributes, study design descriptors, and timestamps aligned to the constraint. In some embodiments, the constraint filters metadata to a named venue or sponsor portfolio to focus extraction on relevant fields. In some embodiments, the metadata is extracted using schema harmonization and ontology mapping to unify author identifiers, company names, biomarker synonyms, and therapy classes. In some embodiments, the metadata is enriched with provenance tags and confidence scores to support downstream ranking and auditability.

(E3) In some embodiments of E1 or E2, the set of documents comprises abstracts from one or more scientific conferences. In some embodiments, the set of documents comprise documents from one or more conferences, journal publications, and/or investigators. In some embodiments, the set of documents comprises at least one of a set of PDF documents, a set of Word documents, a set of images, a set of documents of other formats. In some embodiments, the set of documents corresponds to a particular entity (e.g., an investigator, a publisher, an organization, or other type of entity). In some embodiments, the set of documents corresponds to a particular event (e.g., a conference, a publication, or other type of event). In some embodiments, the set of documents are obtained by querying one or more databases for results meeting the constraint. Example databases include Internet databases, medical databases, publication databases, and organizational databases. In some embodiments, the abstracts are sourced from conferences within the constraint window. In some embodiments, the file formats include PDFs, DOCX files, CSV tables of abstracts, PNG poster images, and HTML pages captured from official sites. In some embodiments, the documents correspond to a named investigator with PubMed identifiers or a pharmaceutical organization with SEC filings and press releases. In some embodiments, the event correspondence targets a specific conference year or journal special issue tied to the subject matter type. In some embodiments, authenticated APIs and secure file transfer are used to ingest the documents that meet the constraint.

(E4) In some embodiments of any of E1-E3, the set of documents comprises a plurality of data types, and the machine-learning component comprises a multi-modal model. In some embodiments, the multi-modal model is a multi-modal language model (e.g., an LLM). In some embodiments, the plurality of data types includes unstructured text, imaging annotations, molecular profiles, tabular clinical outcomes, and device telemetry. In some embodiments, the multi-modal model uses cross modal encoders with modality specific adapters to generate embeddings in a shared latent space. In some embodiments, the multi-modal language model supports multilingual ingestion and maps ontology terms across vendors and sources. In some embodiments, the model fuses metadata conditioning signals such as sponsor, assay type, and cohort attributes to improve retrieval and clustering quality.

(E5) In some embodiments of any of E1-E4, updating the plurality of similarity clusters comprises one or more of: (i) incorporating the set of numerical embeddings into one or more existing clusters of the plurality of similarity clusters; (ii) forming one or more new clusters using the set of numerical embeddings; and (iii) splitting a cluster of the plurality of similarity clusters into a set of new clusters. In some embodiments, the updated clusters are used to identify trends and/or shifts in interest. For example, newly formed clusters may be used to identify current interests. As another example, differences between the previous clusters and updated clusters may be used to identify trends and shifts in interest. In some embodiments, updating the similarity clusters includes adaptive split and merge operations driven by streaming k nearest neighbor graphs and community detection. In some embodiments, the updated clusters are annotated with temporal labels and drift measures that quantify emerging themes and waning topics. In some embodiments, interest signals such as publication count, shared biomarkers, trial activity, and funding disclosures are computed per cluster, e.g., to prioritize current interests. In some embodiments, differential analysis between prior and updated clusters is used to identify trend shifts, novel subtopics, and consolidation of related themes.

(E6) In some embodiments of any of E1-E5, the method further comprises forming the plurality of similarity clusters by applying a clustering algorithm to the plurality of numerical embeddings. In some embodiments, similar embeddings are identified, updated, and linked to entity information. For example, similarities between embeddings may be identified (and updated) and these similarities can be visualized/conceptualized as embedding clusters. In some embodiments, forming the similarity clusters uses HDBSCAN for density based grouping, Louvain or Leiden for community detection over a k nearest neighbor graph, or k means configured for temporal stability. In some embodiments, the plurality of numerical embeddings is preprocessed with dimensionality reduction to improve cluster separability and computational efficiency. In some embodiments, similar embeddings are linked to entity information through shared ontology IDs, sponsor keys, or cohort attributes. In some embodiments, the embedding clusters are visualized with t-distributed stochastic neighbor embedding (t-SNE) or uniform manifold approximation and projection (UMAP) along with metadata overlays, e.g., to support expert review and validation.

(E7) In some embodiments of any of E1-E6, the method further comprises ranking the set of updated similarity clusters based one or more interest metrics. The interest metrics may include one or more of: publication count, shared biomarkers, and associated funding. In some embodiments, the cluster ranking is performed based on relevance to a particular entity, shared biomarkers, shared disease indicators, number of organizations working within the research space, amount research data for each cluster, public interest, and/or organizational attributes for the organizations working within the research space. In some embodiments, the ranking is based on clinical trials data (e.g., relevant clinical trials for a cluster indicates interest in the cluster topics). In some embodiments, the interest metrics include publication velocity, citation counts, and author centrality within the coauthorship graph. In some embodiments, the ranking incorporates grant disclosures, conference prominence, and media mentions to reflect public interest. In some embodiments, the relevance to a particular entity is computed using overlap between cluster keywords and the entity's active project tags. In some embodiments, the system generates a similarity matrix to quantify relatedness and overlap among clusters under multiple contexts. The matrix may comprise vectors whose components reflect context-specific similarity signals, including, for example, text embeddings from abstracts; molecular profiles (e.g., sequencing-derived gene and transcript signatures and clusters organized by biomarkers, diagnosis, or cancer type); structured EHR features (e.g., combinations and values across diagnostics, treatments, and outcomes along a subject's timeline); and treatment history trajectories. These context vectors may be used individually or in combination to produce a composite similarity measure. By integrating multi-context signals, the system improves cluster robustness and matches queries to clusters across modalities and data types (e.g., rather than relying solely on lexical overlap), thereby aligning people, data, and downstream workflows with the technical environment best suited to surface relevant outcomes. Similarities may be based on abstracts, molecular profiles, raw transcript results/signatures, biomarkers, diagnosis, cancer types, EHR entries, treatment history, and/or open ended referencing any means of clustering subjects, data, and/or their underlying intersections as the relate to precision medicine. In some embodiments, the clinical trials data includes trial phase, enrollment status, and biomarker eligibility criteria to signal maturation and focus. In some embodiments, the ranking outputs a composite score with separate sub scores for impact, feasibility, and novelty.

(E8) In some embodiments of any of E1-E7, the entity information indicates subject matter capabilities of the entity. In some embodiments, the entity information corresponds to one or more of: a previously-identified research topic, a prior publication, and use-case data. In some embodiments, the entity information corresponds to subject matter contributions generated by the entity. In some embodiments, identifying the set of potential actions comprises identifying a subject matter need (e.g., research gap, a hypothesis to be evaluated, etc.) from the set of updated clusters and identifying a subject matter capability of the entity. In some embodiments, the entity information includes approved workflows such as cohort builders, trial matchers, analytics pipelines, and reporting templates. In some embodiments, the entity information encodes role based permissions, compliance constraints, and data residency requirements. In some embodiments, the previously identified research topic is linked to a curated ontology of indications, biomarkers, and/or therapies. In some embodiments, the use case data includes target audiences and delivery formats for outputs such as posters, abstracts, and dossiers. In some embodiments, identifying the subject matter need includes detecting gaps where related clusters exhibit stronger evidence than the entity's cluster of interest.

(E9) In some embodiments of any of E1-E8, the method further comprises generating a cluster summary a cluster of the set of updated similarity clusters by selecting a set of keywords representing the cluster. In some embodiments, a cluster summary is generated using context information for the cluster (e.g., in addition to the keywords). In some embodiments, the cluster summary is presented to the entity. In some embodiments, the cluster summary is used to help identify the links between the entity information and the set of updated similarity clusters. In some embodiments, the cluster summary includes a short narrative synopsis and/or a list of representative documents with citations. In some embodiments, the keywords are selected using TF-IDF, guided key phrase extraction, and/or ontology term alignment. In some embodiments, the summary is presented through a dashboard element with links to cohort matchers and trial landscape views. In some embodiments, the summary includes temporal trajectories for top tokens and biomarker mentions. In some embodiments, the summary is used to derive rule-based linking between cluster topics and entity workflows. An example cluster summary is shown below in Table 1.

Table 1 - Example Cluster Summary

| Cluster Number | Cluster Keywords | Number of Abstracts | Number of Sponsors | Most Prolific Sponsor | Identified Potential Actions |
|---|---|---|---|---|---|
| 0 | resistant, castration, androgen, docetaxel, 177lu, arpi, enza, psa, adt, mcrpc, psma | 96 | 60 | Entity A (11 abstracts) | 3 |
| 1 | mutation, markers, showed, idh mutant, diffuse, glioblastoma, idh1, mutant, resection, mri, glioma, gbm, gliomas, idh | 23 | 4 | Entity B (2 abstracts) | 1 |
| 2 | like peptide, peptide, type diabetes, propensity score, hazard, receiving, ratios, matched, medications, loss, weight loss, groups, type, receptor agonists, weight, glp 1ras, 1ras, agonists, obesity, receptor | 14 | 1 | Entity C | 1 |
| 3 | standard, endpoint, brain metastases, temozolomide, radiotherapy, metastases, toxicity, csf, criteria, wbrt, glioblastoma, brain, tmz, gbm | 47 | 19 | Entity D (3 abstracts) | 3 |
| 4 | vaf, allogeneic, pre, hsct, gvhd, transplantation, hematopoietic, factors, myeloid, mds, sct, tp53, chronic, allo hct, allo, relapse, transplant, aml, hct | 28 | 1 | Entity C | 2 |

Table 1 indicates that cluster 0 addresses androgen axis resistance in prostate cancer with high activity as reflected by 96 abstracts and participation from 60 sponsors, with Entity A contributing (e.g., sponsoring) 11 abstracts, and the system identifies three potential actions such as a literature synthesis (e.g., on PSMA and 177Lu), a cohort feasibility query (e.g., for mCRPC patients on ARPIs), and a trial landscape comparison (e.g., for docetaxel plus ADT combinations). In Table 1, cluster 1 centers on diffuse glioma with IDH mutations and surgical resection imaging themes, includes 23 abstracts from 4 sponsors with Entity B contributing (e.g., sponsoring) 2 abstracts, and the system proposes one potential action (e.g., a hypothesis on IDH1 status and MRI response trajectories in GBM subtypes). In some embodiments, a summary in prose is provided for each cluster.

In some embodiments, a cluster summary is generated with a subset or superset of the fields shown in Table 1. For example, additional fields may include publication velocity, median cluster centrality, top ontology terms, clinical trial alignment scores, and grant disclosure counts. In some embodiments, a dashboard view of the summary links each cluster to representative documents. In some embodiments, the summary includes auto-generated keyphrases with associated confidence scores, filters for indication, biomarker, sponsor, trial phase, and the like. In some embodiments, cluster actions include outreach suggestions to entities ranked by overlap in biomarkers and trial portfolios, cohort feasibility toggles aligned to available EHR and genomics coverage, and draft eligibility summaries for top-aligned trials. In some embodiments, temporal trajectories for top tokens and biomarker mentions are indicated to highlight accelerating subtopics and waning areas, and the system flags clusters with rapid growth or consolidation for review. In some embodiments, rule-based linking maps cluster themes to institutional workflows such as cohort builders, trial matchers, and HEOR modeling templates, which reduces time to action and improves consistency across analyses. In some embodiments, the cluster keywords are identified using a frequency analysis (e.g., TF-IDF). In some embodiments, the cluster summaries include a summary of the set of documents (e.g., a conference summary, corpus summary, or other type of overall summary). In some embodiments, the cluster summaries include an indication of related areas of entity contribution, significant themes, relevant additional publications, and/or relevant ongoing research projects.

(E10) In some embodiments of any of E1-E9, the method further comprises identifying a set of entities linked to a potential action of the set of top-ranked potential actions. In some embodiments, indicating the set of top-ranked potential actions comprises indicating the respective sets of entities linked to the set of top-ranked potential actions. In some embodiments, information regarding the potential action is provided to an entity of the set of entities (e.g., a top ranked entity of the set of entities and/or an entity that has indicated it would like to receive this type of information). In some embodiments, the set of entities includes organizations, companies, associations, biopharma sponsors, health systems, academic centers, and/or advocacy groups. In some embodiments, the linkage is computed using shared biomarkers, overlapping trial portfolios, and/or coauthorship networks. In some embodiments, the indicated sets of entities include tiered priority levels and contact roles for outreach. In some embodiments, information regarding the potential action includes effort estimates, data requirements, and/or compliance notes. In some embodiments, delivery to an entity includes secure dashboard notifications and programmatic webhooks.

(E11) In some embodiments of E10, the set of entities is identified based on respective entity data for each entity in the set of entities. In some embodiments, the entities are identified based on identifying respective contributions and/or interests for each entity. In some embodiments, the set of entities is identified based on each entity's research funding, clinical trials, and/or provided medications. For example, an entity may be identified by identifying overlap between a patient cohort and/or clinical trial and a cluster of the set of updated similarity clusters. In some embodiments, the respective entity data includes organizational hierarchies, regional presence, and/or therapeutic area focus. In some embodiments, the contributions include authored publications, trial sponsorships, and/or released datasets. In some embodiments, the interests include stated strategic priorities and/or technology capabilities. In some embodiments, the identification includes scoring based on funding disclosures and active trials per indication. In some embodiments, the overlap is computed using cosine similarity between cohort embeddings and cluster centroids.

(E12) In some embodiments of any of E1-E11, the method further comprises correlating the set of updated similarity clusters with clinical trial data, where the set of potential actions is identified based on the clinical trial data. In some embodiments, respective numerical embeddings are generated for a plurality of clinical trials and the correlation is determined using a similarity measure/algorithm. In some embodiments, the clinical trial embeddings are generated from protocol texts, eligibility criteria, endpoints, and/or biomarker fields. In some embodiments, the similarity measure includes cosine distance in the shared latent space and neighborhood overlap within a k nearest neighbor graph. In some embodiments, the correlation outputs a ranked list of trials aligned to each cluster, e.g., with provenance links. In some embodiments, the potential actions include drafting eligibility summaries and proposing cohort queries tied to trial readiness. In some embodiments, the process includes periodic refreshes aligned to trial registry updates.

(E13) In some embodiments of any of E1-E12, the method further comprises correlating the set of updated similarity clusters with patient cohort data, wherein the set of potential actions is identified based on the patient cohort data. In some embodiments, respective numerical embeddings are generated for a plurality of patient cohorts and the correlation is determined using a similarity measure/algorithm. In some embodiments, the patient cohort embeddings are generated from de-identified EHR fields, genomics panels, imaging annotations, and/or outcomes. In some embodiments, the similarity measure includes dot product scoring and temporal decay to weigh recent signals more heavily. In some embodiments, the correlation identifies cohorts with sufficient data coverage for downstream analytics and feasibility studies. In some embodiments, the potential actions include hypothesis proposals and/or targeted recruitment strategies. In some embodiments, the approach supports role-based access controls and audit logs for cohort linkage operations.

(E14) In some embodiments of any of E1-E13, a potential action of the set of potential actions comprises a hypothesis to be evaluated. In some embodiments, the hypothesis corresponds to a biomarker of interest. In some embodiments, the hypothesis identifies a patient cohort for testing. In some embodiments, the hypothesis corresponds to an interesting follow-up question identified based on the set of documents. In some embodiments, the hypothesis specifies a biomarker outcome association such as EGFR mutation status linked to a response in a defined indication. In some embodiments, the hypothesis targets an underexplored subpopulation such as early-stage patients or a specific ethnic group. In some embodiments, the hypothesis identifies a cohort using inclusion and exclusion criteria aligned to trial readiness. In some embodiments, the hypothesis frames a follow-up question, e.g., derived from divergence between cluster themes and guideline recommendations.

(E15) In some embodiments of any of E1-E14, the method further comprises generating a visualization for at least one of: the set of documents, and the set of similarity clusters (e.g., as illustrated in Figures 7A and 7B). For example, a visualization may include indication of relevant biomarkers, therapies, contributors, keywords, and the like. In some embodiments, the visualization includes a cluster map with nodes annotated by biomarker frequency and/or sponsor participation. In some embodiments, the visualization includes timeline charts showing publication velocity and/or trial phase progression. In some embodiments, the visualization includes heatmaps of co-mentioned therapies and indications. In some embodiments, the visualization includes bar charts of contributor institutions and keyword salience.

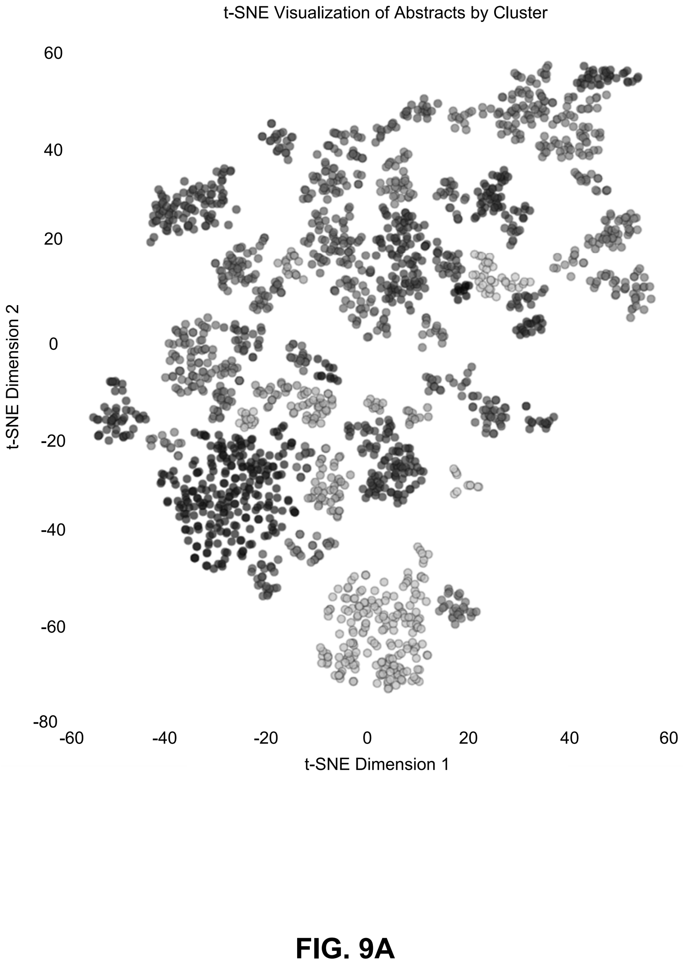

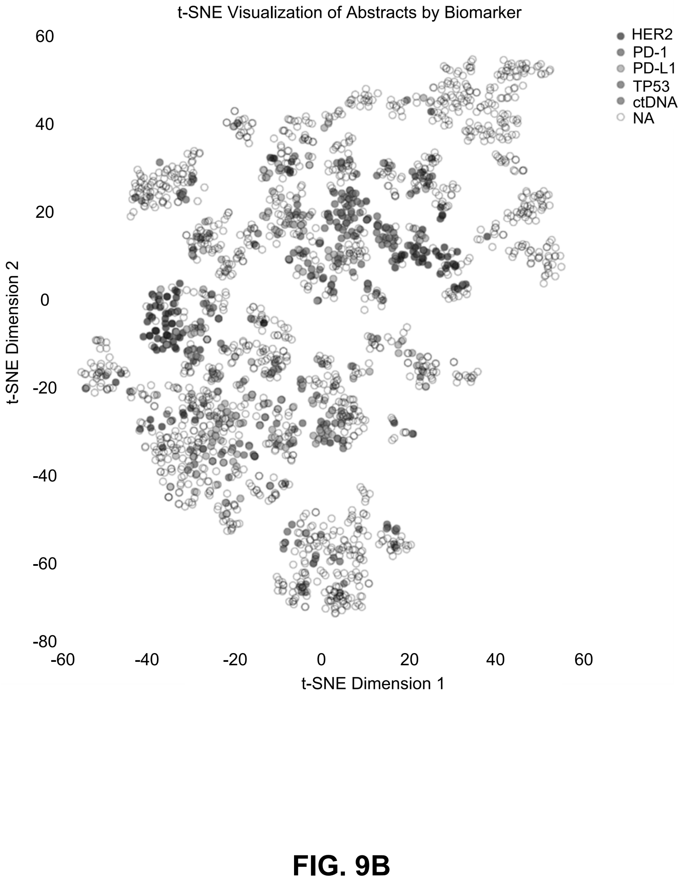

Figures 9A and 9B illustrate example document visualizations in accordance with some embodiments. Figure 9A illustrates a visualization of conference abstracts in a 2D space generated by t-SNE, with points shaded according to their assigned cluster. Figure 9B illustrates a visualization of conference abstracts in a 2D space generated by t-SNE, with points shaded according to the studied biomarker. In some embodiments, the visualization is interactive. For example, a user may hover over a point in the graph to view details about the particular abstract. In some embodiments, insights, potential actions, and/or summaries are generated with the visualization(s), e.g., to assist the user with sifting through the information and identifying key points. In some embodiments, additional visualizations are generated, such as timeline ribbons that track cluster publication velocity across conference years, heatmaps showing co-mention frequency of therapies and indications, bipartite graphs linking sponsors to biomarkers and trial phases, chord diagrams depicting cross-cluster term flows, and/or geographic maps aggregating study sites or author affiliations. In some embodiments, users are able to filter by sponsor, assay type, and/or trial phase, select a region of interest (e.g., to generate a reading list or do further analysis), and mark representative points (e.g., to compare abstracts using autogenerated summaries). In some embodiments, use cases include triaging which clusters merit rapid literature synthesis, identifying emerging biomarkers with accelerating activity, identifying partnership opportunities where a sponsor portfolio overlaps with institutional datasets, and guiding cohort feasibility by aligning cluster topics with available EHR and genomics coverage. The benefits of these visualizations include faster analysis of thousands of abstracts, reduced manual screening effort, earlier detection of trend shifts, and improved decision quality through context-rich overlays (e.g., tying each visualization element to actionable next steps and confidence indicators).

(E16) In some embodiments of any of E1-E15, the constraint is received as part of a user request. In some embodiments, the user request is a query that indicates the period of time and type of subject matter. In some embodiments, the user request specifies a rolling window, e.g., for oncology abstracts. In some embodiments, the user requests specifies an immunotherapy biomarker. In some embodiments, the user request includes a conference identifier and an indication tag to scope retrieval. In some embodiments, the user request provides filters for sponsor names and assay types to refine metadata extraction. In some embodiments, the user request includes refinements (e.g., language preferences) for targeted retrieval.

(E17) In some embodiments of any of E1-E16, the constraint is obtained as part of an automated workflow. In some embodiments, constraints are generated without explicit user requests (e.g., on a periodic basis or in response to identification of events and/or trends). In some embodiments, the automated workflow triggers periodic ingestion, e.g., aligned to conference schedules. In some embodiments, the automated workflow generates constraints when external registries report trial updates. In some embodiments, the automated workflow produces constraints in response to spikes in media mentions or funding disclosures. In some embodiments, the automated workflow schedules quarterly scans across predefined subject matter portfolios.

(E18) In some embodiments of any of E1-E17, the one or more criteria for ranking the set of potential actions comprises one or more of: (i) a criterion related to an assessed value of completing a corresponding potential action; (ii) a criterion related to an assessed capability of the entity to perform the corresponding potential action; and (iii) a criterion related to an assessed cost of the entity to perform the corresponding potential action. In some embodiments, at least a subset of the criteria are based on collaborations between the entity and one or more other entities (e.g., research partners). In some embodiments, the criteria are received from a user input. In some embodiments, the criteria are part of an automated workflow. For example, the criteria may include a criterion regarding having an existing partnership with another entity that is assessed to have an interest in the potential action. In some embodiments, the ranking criteria include expected clinical impact (e.g., scored against gaps and/or unmet needs). In some embodiments, the ranking criteria include feasibility scored using available datasets, approved workflows, and/or available resources. In some embodiments, the ranking criteria include a cost estimated from required analytics and/or partner dependencies. In some embodiments, the ranking criteria include collaboration signals such as coauthorship networks and existing partner agreements.

(E19) In some embodiments of any of E1-E18, the method further comprises, after generating the set of updated similarity clusters: (i) receiving a user query requesting information about the set of updated similarity clusters; and (ii) responsive to the user query, providing information from the set of documents. In some embodiments, the user query includes a second constraint, and the provided information is information from the set of documents that meets the second constraint. In some embodiments, the user query includes a request for a second ranked set of potential actions meeting one or more criteria. In some embodiments, the request for the second ranked set of potential actions includes identification of a second entity (different from the entity), and responsive to the request, second entity information is obtained for the second entity. In some embodiments, the user query is a query for information about subject matter trends and/or current interests. In some embodiments, the user query requests clusters within a second constraint such as a specific sponsor portfolio or time window. In some embodiments, the response provides citations and/or cluster summaries filtered to the second constraint. In some embodiments, the user query seeks a second ranked set of potential actions scoped to a second entity with different workflows and datasets. In some embodiments, the response includes individualized rankings for the second entity and trend analyses highlighting current interests.

Although Figures 8A-8E illustrate a number of logical stages in a particular order, stages which are not order dependent may be reordered and other stages may be combined or broken out. Some reordering or other groupings not specifically mentioned will be apparent to those of ordinary skill in the art, so the ordering and groupings presented herein are not exhaustive. Moreover, it should be recognized that the stages could be implemented in hardware, firmware, software, or any combination thereof.

In another aspect, some embodiments include a computing system including one or more processors and memory coupled to the one or more processors, the memory storing one or more programs configured to be executed by the one or more processors, the one or more programs including instructions for performing any of the methods described herein (e.g., A1-A12, B1-B6, C1-C4, D1-D7, and E1-E19 above). In another aspect, some embodiments include a non-transitory computer-readable storage medium storing one or more sets of instructions for execution by control circuitry of a computing system, the one or more sets of instructions including instructions for performing one or more of the methods described herein (e.g., A1-A12, B1-B6, C1-C4, D1-D7, and E1-E19 above).

As used herein, "biomarker" means any measurable, computable, or inferable feature that informs a biological state, condition, phenotype, disease, trait, exposure, response, safety event, outcome, or therapeutic effect in a subject, cohort, or population. Biomarkers include, without limitation: molecular features (e.g., genomic, transcriptomic, epigenomic, proteomic, metabolomic, microbiomic); imaging-derived features (e.g., radiomic/pathomic signatures and quantitative measurements); laboratory and physiological measurements (e.g., hematology, chemistry, vital signs, device telemetry); clinical attributes (e.g., diagnoses, stage, line of therapy, prior treatments, comorbidities, medications, performance status, social determinants); trial and care-context attributes (e.g., eligibility elements, endpoints, adverse events); real-world data features (e.g., from EHRs, claims, registries, and patient-reported outcomes); and clinico-genomic features that combine clinical and molecular information. A biomarker may be directly measured or derived (including via machine-learning models, embeddings, clustering outputs, risk scores, or composite indices), may be static or time-varying, single- or multi-modal, and categorical, ordinal, or continuous. The term expressly includes surrogate endpoints, prognostic and predictive markers, diagnostic indicators, safety and pharmacodynamic markers, and digital biomarkers.

Various types of models and algorithms may be used with the agents and ML components disclosed herein. In some embodiments, a model is a supervised machine learning algorithm. Nonlimiting examples of supervised learning algorithms include, but are not limited to, logistic regression, neural networks, support vector machines, Naive Bayes algorithms, nearest neighbor algorithms, random forest algorithms, decision tree algorithms, boosted trees algorithms, multinomial logistic regression algorithms, linear models, linear regression, Gradient Boosting, mixture models, hidden Markov models, Gaussian NB algorithms, linear discriminant analysis, or any combinations thereof. In some embodiments, a model is a multinomial classifier algorithm. In some embodiments, a model is a 2-stage stochastic gradient descent (SGD) model. In some embodiments, a model is a deep neural network (e.g., a deep-and-wide sample-level classifier).

In some embodiments, a model is, or includes, a neural network (e.g., a convolutional neural network and/or a residual neural network). Neural network algorithms, also known as artificial neural networks (ANNs), include convolutional and/or residual neural network algorithms (deep learning algorithms). Neural networks can be machine learning algorithms that may be trained to map an input data set to an output data set, where the neural network comprises an interconnected group of network nodes organized into multiple layers of network nodes. For example, the neural network architecture may comprise at least an input layer, one or more hidden layers, and an output layer. The neural network may comprise any total number of layers, and any number of hidden layers, where the hidden layers function as trainable feature extractors that allow mapping of a set of input data to an output value or set of output values. As used herein, a deep learning algorithm can be a neural network comprising a plurality of hidden layers, e.g., two or more hidden layers. Each layer of the neural network can comprise a number of network nodes (also sometimes referred to as neurons). A network node can receive input that comes either directly from the input data or the output of network nodes in previous layers, and perform a specific operation, e.g., a summation operation. In some embodiments, a connection from an input to a network node is associated with a parameter (e.g., a weight and/or weighting factor). In some embodiments, a network node sums up the products of all pairs of inputs, $x_i$, and their associated parameters. In some embodiments, the weighted sum is offset with a bias, b. In some embodiments, the output of a network node is gated using a threshold or activation function, f, which may be a linear or non-linear function. The activation function may be, for example, a rectified linear unit (ReLU) activation function, a Leaky ReLU activation function, or other function such as a saturating hyperbolic tangent, identity, binary step, logistic, arcTan, softsign, parametric rectified linear unit, exponential linear unit, softPlus, bent identity, softExponential, Sinusoid, Sine, Gaussian, or sigmoid function, or any combination thereof.

The weighting factors, bias values, and threshold values, or other computational parameters of the neural network, may be "taught" or "learned" in a training phase using one or more sets of training data. For example, the parameters may be trained using the input data from a training data set and a gradient descent or backward propagation method so that the output value(s) that the ANN computes are consistent with the examples included in the training data set. The parameters may be obtained from a back propagation neural network training process.

As an example, a variety of neural networks may be suitable for use in analyzing an image of an eye of a subject. Examples can include, but are not limited to, feedforward neural networks, radial basis function networks, recurrent neural networks, residual neural networks, convolutional neural networks, residual convolutional neural networks, and the like, or any combination thereof. In some embodiments, a machine-learning model uses a pretrained and/or transfer-learned ANN or deep learning architecture. Convolutional and/or residual neural networks can be used for analyzing an image of a subject in accordance with the present disclosure. Some embodiments use generative models, such as generative adversarial networks (GANs) and hidden Markov models. In a GAN, two neural networks compete against each other, with one generating samples and the other evaluating whether they are real or generated. A hidden Markov model is a generative model that has been successful in various sequence labeling tasks such as chunking, named entity recognition, POS tagging, and speech recognition.

A deep neural network model may include an input layer, a plurality of individually parameterized (e.g., weighted) convolutional layers, and an output scorer. The parameters (e.g., weights) of each of the convolutional layers as well as the input layer contribute to the plurality of parameters (e.g., weights) associated with the deep neural network model. In some embodiments, at least 100 parameters, at least 1000 parameters, at least 2000 parameters or at least 5000 parameters are associated with the deep neural network model. As such, deep neural network models require a computer to be used because they cannot be mentally solved. In other words, given an input to the model, the model output needs to be determined using a computer rather than mentally in such embodiments. See, for example, Krizhevsky et al., 2012, "Imagenet classification with deep convolutional neural networks," in Advances in Neural Information Processing Systems 2, Pereira, Burges, Bottou, Weinberger, eds., pp. 1097-1105, Curran Associates, Inc.; Zeiler, 2012 "ADADELTA: an adaptive learning rate method," CoRR, vol. abs/1212.5701; and Rumelhart et al., 1988, "Neurocomputing: Foundations of research," ch. Learning Representations by Back-propagating Errors, pp. 696-699, Cambridge, MA, USA: MIT Press, each of which is hereby incorporated by reference.

Neural network algorithms, including convolutional neural network algorithms, suitable for use as models are disclosed in, for example, Vincent et al., 2010, "Stacked denoising autoencoders: Learning useful representations in a deep network with a local denoising criterion," J Mach Learn Res 11, pp. 3371-3408; Larochelle et al., 2009, "Exploring strategies for training deep neural networks," J Mach Learn Res 10, pp. 1-40; and Hassoun, 1995, Fundamentals of Artificial Neural Networks, Massachusetts Institute of Technology, each of which is hereby incorporated by reference. Additional example neural networks suitable for use as models are disclosed in Duda et al., 2001, Pattern Classification, Second Edition, John Wiley & Sons, Inc., New York; and Hastie et al., 2001, The Elements of Statistical Learning, Springer-Verlag, New York, each of which is hereby incorporated by reference in its entirety. Additional example neural networks suitable for use as models are also described in Draghici, 2003, Data Analysis Tools for DNA Microarrays, Chapman & Hall/CRC; and Mount, 2001, Bioinformatics: sequence and genome analysis, Cold Spring Harbor Laboratory Press, Cold Spring Harbor, New York, each of which is hereby incorporated by reference in its entirety.

In some embodiments, a model is, or includes, a support vector machine (SVM). SVM algorithms suitable for use as models are described in, for example, Cristianini and Shawe-Taylor, 2000, "An Introduction to Support Vector Machines," Cambridge University Press, Cambridge; Boser et al., 1992, "A training algorithm for optimal margin classifiers," in Proceedings of the 5th Annual ACM Workshop on Computational Learning Theory, ACM Press, Pittsburgh, Pa., pp. 142-152; Vapnik, 1998, Statistical Learning Theory, Wiley, New York; Mount, 2001, Bioinformatics: sequence and genome analysis, Cold Spring Harbor Laboratory Press, Cold Spring Harbor, N.Y.; Duda, Pattern Classification, Second Edition, 2001, John Wiley & Sons, Inc., pp. 259, 262-265; and Hastie, 2001, The Elements of Statistical Learning, Springer, New York; and Furey et al., 2000, Bioinformatics 16, 906-914, each of which is hereby incorporated by reference in its entirety. When used for classification, SVMs separate a given set of binary labeled data with a hyper-plane that is maximally distant from the labeled data. For cases in which no linear separation is possible, SVMs can work in combination with the technique of 'kernels', which automatically realizes a non-linear mapping to a feature space. The hyper-plane found by the SVM in feature space can correspond to a non-linear decision boundary in the input space. In some embodiments, the plurality of parameters (e.g., weights) associated with the SVM define the hyper-plane. In some embodiments, the hyper-plane is defined by at least 10, at least 20, at least 50, or at least 100 parameters and the SVM model requires a computer to calculate because it cannot be mentally solved.

In some embodiments, a model is, or includes, a Naive Bayes algorithm. Naïve Bayes models suitable for use as models are disclosed, for example, in Ng et al., 2002, "On discriminative vs. generative classifiers: A comparison of logistic regression and naive Bayes," Advances in Neural Information Processing Systems, 14, which is hereby incorporated by reference. A Naive Bayes model is any model in a family of "probabilistic models" based on applying Bayes' theorem with strong (naïve) independence assumptions between the features. In some embodiments, they are coupled with Kernel density estimation. See, for example, Hastie et al., 2001, The elements of statistical learning : data mining, inference, and prediction, eds. Tibshirani and Friedman, Springer, New York, which is hereby incorporated by reference.

In some embodiments, a model is, or includes, a Boltzmann machine. A Boltzmann machine comprises a set of binary units that are connected through weighted connections. Boltzmann Machines may use directionless unsupervised generative deep learning network for recommended systems.

In some embodiments, a model is, or includes, a nearest neighbor algorithm. Nearest neighbor models can be memory-based and include no model to be fit. For nearest neighbors, given a query point x0 (a test subject), the k training points x(r), r, ... , k (here the training subjects) closest in distance to x0 are identified and then the point x0 is classified using the k nearest neighbors. Here, the distance to these neighbors is a function of the abundance values of the discriminating gene set. In some embodiments, Euclidean distance in feature space is used to determine distance as Typically, when the nearest neighbor algorithm is used, the abundance data used to compute the linear discriminant is standardized to have mean zero and variance 1. The nearest neighbor rule can be refined to address issues of unequal class priors, differential misclassification costs, and feature selection. Many of these refinements involve some form of weighted voting for the neighbors. For more information on nearest neighbor analysis, see Duda, Pattern Classification, Second Edition, 2001, John Wiley & Sons, Inc; and Hastie, 2001, The Elements of Statistical Learning, Springer, New York, each of which is hereby incorporated by reference.

As an example, a k-nearest neighbor model is a non-parametric machine learning method in which the input includes the k closest training examples in feature space. The output is a class membership. An object is classified by a plurality vote of its neighbors, with the object being assigned to the class most common among its k nearest neighbors (k is a positive integer, typically small). If k = 1, then the object is simply assigned to the class of that single nearest neighbor. See, Duda et al., 2001, Pattern Classification, Second Edition, John Wiley & Sons, which is hereby incorporated by reference. In some embodiments, the number of distance calculations needed to solve the k-nearest neighbor model is such that a computer is used to solve the model for a given input because it cannot be mentally performed.

In some embodiments, a model is, or includes, a decision tree. Decision trees suitable for use as models are described generally by Duda, 2001, Pattern Classification, John Wiley & Sons, Inc., New York, pp. 395-396, which is hereby incorporated by reference. Tree-based methods partition the feature space into a set of rectangles, and then fit a model (like a constant) in each one. In some embodiments, the decision tree is random forest regression. One specific algorithm that can be used is a classification and regression tree (CART). Other specific decision tree algorithms include, but are not limited to, ID3, C4.5, MART, and Random Forests. CART, ID3, and C4.5 are described in Duda, 2001, Pattern Classification, John Wiley & Sons, Inc., New York, pp. 396-408 and pp. 411-412, which is hereby incorporated by reference. CART, MART, and C4.5 are described in Hastie et al., 2001, The Elements of Statistical Learning, Springer-Verlag, New York, Chapter 9, which is hereby incorporated by reference in its entirety. Random Forests are described in Breiman, 1999, "Random Forests--Random Features," Technical Report 567, Statistics Department, U.C. Berkeley, September 1999, which is hereby incorporated by reference in its entirety. In some embodiments, the decision tree model includes at least 10, at least 20, at least 50, or at least 100 parameters (e.g., weights and/or decisions) and requires a computer to calculate because it cannot be mentally solved.

In some embodiments, a model uses a regression algorithm. A regression algorithm can be any type of regression. For example, the regression algorithm may be logistic regression. In some embodiments, the regression algorithm is logistic regression with lasso, L2 or elastic net regularization. In some embodiments, those extracted features that have a corresponding regression coefficient that fails to satisfy a threshold value are pruned (removed from) consideration. In some embodiments, a generalization of the logistic regression model that handles multicategory responses is used as the model. Logistic regression algorithms are disclosed in Agresti, An Introduction to Categorical Data Analysis, 1996, Chapter 5, pp. 103-144, John Wiley & Son, New York, which is hereby incorporated by reference. In some embodiments, the model makes use of a regression model disclosed in Hastie et al., 2001, The Elements of Statistical Learning, Springer-Verlag, New York. In some embodiments, the logistic regression model includes at least 10, at least 20, at least 50, at least 100, or at least 1000 parameters (e.g., weights) and requires a computer to calculate because it cannot be mentally solved.

Linear discriminant analysis (LDA), normal discriminant analysis (NDA), or discriminant function analysis can be a generalization of Fisher's linear discriminant, a method used in statistics, pattern recognition, and machine learning to find a linear combination of features that characterizes or separates two or more classes of objects or events. The resulting combination can be used as a model (e.g., a linear model) in some embodiments of the present disclosure.

In some embodiments, a model is a mixture model, such as that described in McLachlan et al., Bioinformatics 18(3):413-422, 2002. In some embodiments, in particular, those embodiments including a temporal component, a model is a hidden Markov model such as described by Schliep et al., 2003, Bioinformatics 19(1):i255-i263.

In some embodiments, a model is an unsupervised clustering model. In some embodiments, a model is a supervised clustering model. Clustering algorithms suitable for use as models are described, for example, at pages 211-256 of Duda and Hart, Pattern Classification and Scene Analysis, 1973, John Wiley & Sons, Inc., New York, (hereinafter "Duda 1973") which is hereby incorporated by reference in its entirety. The clustering problem can be described as one of finding natural groupings in a dataset. To identify natural groupings, two issues can be addressed. First, a way to measure similarity (or dissimilarity) between two samples can be determined. This metric (e.g., similarity measure) can be used to ensure that the samples in one cluster are more like one another than they are to samples in other clusters. Second, a mechanism for partitioning the data into clusters using the similarity measure can be determined. One way to begin a clustering investigation can be to define a distance function and to compute the matrix of distances between all pairs of samples in the training set. If distance is a good measure of similarity, then the distance between reference entities in the same cluster can be significantly less than the distance between the reference entities in different clusters. However, clustering may not use a distance metric. For example, a nonmetric similarity function $s(x, x')$ can be used to compare two vectors $x$ and $x'$. $s(x, x')$ can be a symmetric function whose value is large when $x$ and $x'$ are somehow "similar." Once a method for measuring "similarity" or "dissimilarity" between points in a dataset has been selected, clustering can use a criterion function that measures the clustering quality of any partition of the data. Partitions of the data set that extremize the criterion function can be used to cluster the data. Particular exemplary clustering techniques that can be used in the present disclosure can include, but are not limited to, hierarchical clustering (agglomerative clustering using a nearest-neighbor algorithm, farthest-neighbor algorithm, the average linkage algorithm, the centroid algorithm, or the sum-of-squares algorithm), k-means clustering, fuzzy k-means clustering algorithm, and Jarvis-Patrick clustering. In some embodiments, the clustering comprises unsupervised clustering (e.g., with no preconceived number of clusters and/or no predetermination of cluster assignments).

In some embodiments, an ensemble (e.g., two or more) of models is used. In some embodiments, a boosting technique such as AdaBoost is used in conjunction with many other types of learning algorithms to improve the performance of the model. In this approach, the output of any of the models disclosed herein, or their equivalents, is combined into a weighted sum that represents the final output of the boosted model. In some embodiments, the plurality of outputs from the models is combined using any measure of central tendency known in the art, including but not limited to a mean, median, mode, a weighted mean, weighted median, weighted mode, etc. In some embodiments, the plurality of outputs is combined using a voting method. In some embodiments, a respective model in the ensemble of models is weighted or unweighted.

In some embodiments, a model is a reinforcement learning model. In some embodiments, the reinforcement learning system comprises four main elements - an agent, a policy, a reward signal, and a value function, where the behavior of the agent is defined in terms of the policy. In some embodiments, the reinforcement learning system comprises a learning algorithm. In some implementations, the learning algorithm is an on-policy learning algorithm or an off-policy learning algorithm. On-Policy learning algorithms evaluate and improve the same policy which is being used to select the agent's actions. Off-Policy learning algorithms evaluate and improve policies that are different from the policy being used for action selection. Reinforcement learning is further described, for example, in Sutton RS, Barto AG, "Reinforcement learning: an introduction," IEEE Transactions on Neural Networks. 1998;9(5):1054-1054, which is hereby incorporated herein by reference in its entirety.

In some embodiments, a model is, or includes, an autoencoder. An autoencoder is a type of generative model used for unsupervised learning that learns a latent representation of the image and uses that to reconstruct the image. The autoencoder may be a variational autoencoder (VAE) that learns to generate new data samples that are similar to a training dataset.

In some embodiments, a model is, or includes, a transformer model. As described previously, a transformer model is a neural network that learns context and thus meaning by tracking relationships in sequential data like the words in this sentence. Transformer models are used to generate images and audio as well as text.

In some embodiments, a model is, or includes, a diffusion model. A diffusion model generates data points that are similar to the data points on which the model has been trained. In some embodiments, a model is, or includes, a probabilistic generative model, such as a Bayesian network in which the joint distribution between all of the model variables can be expressed as a function of their parents.

As used herein, the term "instruction" refers to an order given to a computer processor by a computer program. On a digital computer, in some embodiments, each instruction is a sequence of 0's and 1's that describes a physical operation the computer is to perform. Such instructions can include data transfer instructions and data manipulation instructions. In some embodiments, each instruction is a type of instruction in an instruction set that is recognized by a particular processor type used to carry out the instructions. Examples of instruction sets include, but are not limited to, Reduced Instruction Set Computer (RISC), Complex Instruction Set Computer (CISC), Minimal Instruction Set Computers (MISC), Very Long Instruction Word (VLIW), Explicitly Parallel Instruction Computing (EPIC), and One Instruction Set Computer (OISC).

As used herein, the term "parameter" refers to any coefficient or, similarly, any value of an internal or external element (e.g., a weight and/or a hyperparameter) in an algorithm, model, regressor, and/or classifier that can affect (e.g., modify, tailor, and/or adjust) one or more inputs, outputs, and/or functions in the algorithm, model, regressor and/or classifier. For example, in some embodiments, a parameter refers to any coefficient, weight, and/or hyperparameter that can be used to control, modify, tailor, and/or adjust the behavior, learning, and/or performance of an algorithm, model, regressor, and/or classifier. In some instances, a parameter is used to increase or decrease the influence of an input (e.g., a feature) to an algorithm, model, regressor, and/or classifier. As a nonlimiting example, in some embodiments, a parameter is used to increase or decrease the influence of a node (e.g., of a neural network), where the node includes one or more activation functions. Assignment of parameters to specific inputs, outputs, and/or functions is not limited to any one paradigm for a given algorithm, model, regressor, and/or classifier but can be used in any suitable algorithm, model, regressor, and/or classifier architecture for a desired performance. In some embodiments, a parameter has a fixed value. In some embodiments, a value of a parameter is manually and/or automatically adjustable. In some embodiments, a value of a parameter is modified by a validation and/or training process for an algorithm, model, regressor, and/or classifier (e.g., by error minimization and/or backpropagation methods). In some embodiments, an algorithm, model, regressor, and/or classifier of the present disclosure includes a plurality of parameters. As such, the algorithms, models, regressors, and/or classifiers of the present disclosure cannot be mentally performed. In some embodiments, the algorithms, models, regressors, and/or classifier of the present disclosure operate in a k-dimensional space, where k is a positive integer of 5 or greater (e.g., 5, 6, 7, 8, 9, 10, etc.). As such, the algorithms, models, regressors, and/or classifiers of the present disclosure cannot be mentally performed.

In some embodiments, the methods described herein include inputting information into a model comprising a plurality of parameters, where the model applies the plurality parameters to the information through a plurality of instructions to generate an output from the model.

In some embodiments, an algorithm, model, regressor, and/or classifier of the present disclosure comprises a plurality of parameters. In some embodiments the plurality of parameters is n parameters, where: $n \geq 2$; $n \geq 5$; $n \geq 10$; $n \geq 25$; $n \geq 40$; $n \geq 50$; $n \geq 75$; $n \geq 100$; $n \geq 125$; $n \geq 150$; $n \geq 200$; $n \geq 225$; $n \geq 250$; $n \geq 350$; $n \geq 500$; $n \geq 600$; $n \geq 750$; $n \geq 1,000$; $n \geq 2,000$; $n \geq 4,000$; $n \geq 5,000$; $n \geq 7,500$; $n \geq 10,000$; $n \geq 20,000$; $n \geq 40,000$; $n \geq 75,000$; $n \geq 100,000$; $n \geq 200,000$; $n \geq 500,000$, $n \geq 1 \times 10^6$, $n \geq 5 \times 10^6$, or $n \geq 1 \times 10^7$. In some embodiments n is between 10,000 and $1 \times 10^7$, between 100,000 and $5 \times 10^6$, or between 500,000 and $1 \times 10^6$. In some embodiments, the plurality of parameters is at least 1000 parameters, at least 5000 parameters, at least 10,000 parameters is at least 50,000 parameters, at least 100,000 parameters, at least 250,000 parameters, at least 500,000 parameters, at least 1 million parameters, at least 5 million parameters, at least 10 million parameters, at least 25 million parameters, at least 50 million parameters, at least 100 million parameters, at least 250 million parameters, at least 500 million parameters, at least 1 billion parameters, or more parameters.

In some embodiments, the plurality of instructions is at least 1000 instructions, at least 5000 instructions, at least 10,000 instructions is at least 50,000 instructions, at least 100,000 instructions, at least 250,000 instructions, at least 500,000 instructions, at least 1 million instructions, at least 5 million instructions, at least 10 million instructions, at least 25 million instructions, at least 50 million instructions, at least 100 million instructions, at least 250 million instructions, at least 500 million instructions, at least 1 billion instructions, or more instructions.

It will be understood that, although the terms "first," "second," etc. may be used herein to describe various elements, these elements should not be limited by these terms. These terms are only used to distinguish one element from another.

The terminology used herein is for the purpose of describing particular embodiments only and is not intended to be limiting of the claims. As used in the description of the embodiments and the appended claims, the singular forms "a," "an" and "the" are intended to include the plural forms as well, unless the context clearly indicates otherwise. It will also be understood that the term "and/or" as used herein refers to and encompasses any and all possible combinations of one or more of the associated listed items. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

As used herein, the term "set" refers to a group of one or more objects. As used herein, the terms "request," "prompt," and "query" are used interchangeable unless expressly stated otherwise. As used herein, the term "model" refers to a machine learning model or algorithm. In some embodiments, the model is a task-specific model (e.g., a task-specific machine-learning model). As used herein, the term "task-specific" refers to a component that is specifically configured to perform a single task or a subset of tasks (e.g., a single class of tasks). In some embodiments, the model is an unsupervised learning algorithm. One example of an unsupervised learning algorithm is cluster analysis.

As used herein, the term "if" can be construed to mean "when" or "upon" or "in response to determining" or "in accordance with a determination" or "in response to detecting" that a stated condition precedent is true, depending on the context. Similarly, the phrase "if it is determined [that a stated condition precedent is true]" or "if [a stated condition precedent is true]" or "when [a stated condition precedent is true]" can be construed to mean "upon determining" or "in response to determining" or "in accordance with a determination" or "upon detecting" or "in response to detecting" that the stated condition precedent is true, depending on the context.

The foregoing description, for purposes of explanation, has been described with reference to specific embodiments. However, the illustrative discussions above are not intended to be exhaustive or limit the claims to the precise forms disclosed. Many modifications and variations are possible in view of the above teachings. The embodiments were chosen and described in order to best explain principles of operation and practical applications, to thereby enable others skilled in the art.

The following items constitute embodiments of the invention:

1. A method performed at a machine-learning component that comprises one or more machine-learning models, the method comprising:

obtaining, at the machine-learning component, a constraint indicating a period of time and a type of subject matter, wherein the machine-learning component has knowledge of a plurality of numerical embeddings corresponding a plurality of documents, and wherein the plurality of numerical embeddings relates to a set of similarity clusters;
obtaining, using the constraint, a set of documents and corresponding metadata, wherein at least a subset of the set of documents is not included in the plurality of documents;
generating a set of numerical embeddings for the set of documents based on information contained within the set of documents and the corresponding metadata;

generating a set of updated similarity clusters by updating the set of similarity clusters to incorporate the set of numerical embeddings;

obtaining, for an entity, entity information comprising a set of one or more workflows and one or more datasets;

identifying a set of potential actions based on links between the entity information and the set of updated similarity clusters;

generating a ranked set of potential actions by ranking the set of potential actions according to one or more criteria; and

providing a notification to the entity indicating a set of top-ranked potential actions from the ranked set of potential actions.

2. The method of item 1, wherein the corresponding metadata is a subset of available metadata for the set of documents, and wherein the corresponding metadata is identified from the available metadata using the constraint.

3. The method of item 1, wherein the set of documents comprises abstracts from one or more scientific conferences.

4. The method of item 1, wherein the set of documents comprises a plurality of data types, and wherein the machine-learning component is configured to ingest multiple modalities of input data.

5. The method of item 1, further comprising forming the set of similarity clusters by applying a clustering algorithm to the plurality of numerical embeddings.

6. The method of item 1, wherein the set of updated similarity clusters are formed by applying a clustering algorithm to the plurality of numerical embeddings and the set of numerical embeddings.

7. The method of item 1, further comprising ranking the set of updated similarity clusters based on one or more interest metrics.

8. The method of item 1, wherein the entity information indicates subject matter capabilities of the entity.

9. The method of item 1, further comprising generating a cluster summary for a cluster of the set of updated similarity clusters by selecting a set of keywords representing the cluster, wherein the links between the entity information and the set of updated similarity clusters includes links between the entity information and the cluster summary.

10. The method of item 1, further comprising identifying a set of entities linked to a potential action of the set of top-ranked potential actions.

11. The method of item 10, wherein the set of entities is identified based on respective entity data for each entity in the set of entities.

12. The method of item 1, further comprising correlating the set of updated similarity clusters with clinical trial data, wherein the set of potential actions is identified based on the clinical trial data.

13. The method of item 1, further comprising correlating the set of updated similarity clusters with patient cohort data, wherein the set of potential actions is identified based on the patient cohort data.

14. The method of item 1, wherein a potential action of the set of potential actions comprises a hypothesis to be tested.

15. The method of item 1, further comprising generating a visualization for at least one of: the set of documents, and the set of similarity clusters.

16. The method of item 1, wherein the constraint is received as part of a user request.

17. The method of item 1, wherein the constraint is obtained as part of an automated workflow.

18. The method of item 1, wherein the one or more criteria for ranking the set of potential actions comprises one or more of:

a criterion related to an assessed value of completing a corresponding potential action;

a criterion related to an assessed capability of the entity to perform the corresponding potential action; and
a criterion related to an assessed cost of the entity to perform the corresponding potential action.

19. The method of item 1, further comprising, after generating the set of updated similarity clusters:

receiving a user query requesting information about the set of updated similarity clusters; and
responsive to the user query, providing information from the set of documents.

20. A computing system, comprising:

one or more processors;
memory; and
one or more programs, wherein the one or more programs are stored in the memory and configured to be executed by the one or more processors, the one or more programs including instructions for:

obtaining, at a machine-learning component, a constraint indicating a period of time and a type of subject matter, wherein the machine-learning component has knowledge of a plurality of numerical embeddings corresponding a plurality of documents, and wherein the plurality of numerical embeddings relates to a set of similarity clusters;
obtaining, using the constraint, a set of documents and corresponding metadata, wherein at least a subset of the set of documents is not included in the plurality of documents;
generating a set of numerical embeddings for the set of documents based on information contained within the set of documents and the corresponding metadata;
generating a set of updated similarity clusters by updating the set of similarity clusters to incorporate the set of numerical embeddings;
obtaining, for an entity, entity information comprising a set of one or more workflows and one or more datasets;
identifying a set of potential actions based on links between the entity information and the set of updated similarity clusters;
generating a ranked set of potential actions by ranking the set of potential actions according to one or more criteria; and

providing a notification to the entity indicating a set of top-ranked potential actions from the ranked set of potential actions.

## Claims

1.  **A method** performed at a machine-learning component that comprises one or more machine-learning models, the method comprising:

obtaining, at the machine-learning component, a constraint indicating a period of time and a type of subject matter, wherein the machine-learning component has knowledge of a plurality of numerical embeddings corresponding a plurality of documents, and wherein the plurality of numerical embeddings relates to a set of similarity clusters;
obtaining, using the constraint, a set of documents and corresponding metadata, wherein at least a subset of the set of documents is not included in the plurality of documents;
generating a set of numerical embeddings for the set of documents based on information contained within the set of documents and the corresponding metadata;
generating a set of updated similarity clusters by updating the set of similarity clusters to incorporate the set of numerical embeddings;
obtaining, for an entity, entity information comprising a set of one or more workflows and one or more datasets;
identifying a set of potential actions based on links between the entity information and the set of updated similarity clusters;
generating a ranked set of potential actions by ranking the set of potential actions according to one or more criteria; and
providing a notification to the entity indicating a set of top-ranked potential actions from the ranked set of potential actions.

2. The method of claim 1, wherein the corresponding metadata is a subset of available metadata for the set of documents, and wherein the corresponding metadata is identified from the available metadata using the constraint.

3. The method of claim 1, wherein the set of documents comprises a plurality of data types, and wherein the machine-learning component is configured to ingest multiple modalities of input data.

4. The method of claim 1, further comprising forming the set of similarity clusters by applying a clustering algorithm to the plurality of numerical embeddings.

5. The method of claim 1, wherein the set of updated similarity clusters are formed by applying a clustering algorithm to the plurality of numerical embeddings and the set of numerical embeddings.

6. The method of claim 1, further comprising ranking the set of updated similarity clusters based on one or more interest metrics.

7. The method of claim 1, wherein the entity information indicates subject matter capabilities of the entity.

8. The method of claim 1, further comprising identifying a set of entities linked to a potential action of the set of top-ranked potential actions, wherein the set of entities is identified based on respective entity data for each entity in the set of entities.

9. The method of claim 1, further comprising correlating the set of updated similarity clusters with clinical trial data, wherein the set of potential actions is identified based on the clinical trial data.

10. The method of claim 1, further comprising correlating the set of updated similarity clusters with patient cohort data, wherein the set of potential actions is identified based on the patient cohort data.

11. **The method** of claim 1, wherein the constraint is received as part of a user request or as part of an automated workflow.

12. The method of claim 1, wherein the one or more criteria for ranking the set of potential actions comprises one or more of:

    a criterion related to an assessed value of completing a corresponding potential action;
    a criterion related to an assessed capability of the entity to perform the corresponding potential action; and
    a criterion related to an assessed cost of the entity to perform the corresponding potential action.

13. The method of claim 1, further comprising, after generating the set of updated similarity clusters:

    receiving a user query requesting information about the set of updated similarity clusters; and
    responsive to the user query, providing information from the set of documents.

14. A computing system, comprising:

    one or more processors;
    memory; and
    one or more programs, wherein the one or more programs are stored in the memory and configured to be executed by the one or more processors, the one or more programs including instructions to perform or cause performance of the methods of any one of claims 1-13.

15. A computer-readable storage medium including executable instructions that, when executed by one or more processors, cause the one or more processors to perform or cause performance of the methods of any one of claims 1-13.

Platform
100

Client device(s)
102

Network(s) 104

Server System
106

External Service(s)
110

External Database(s)
108

**FIG. 1**

EP 4 752 754 A1

Client Device
102

Memory 218

214

Network
Interface(s)

Wireless
216

CPU(s)
202

217

204

User Interface

Output Device(s) 206

Input Device(s)
212

Operating System 220

Network Communication Module(s) 222

User Interface Module 224

ML Component 226

Language Model(s) 228

Interface Module 231

Web Browser Application 233

Other Applications 235

Data Modules 240

Medical Database(s) 242

User Database(s) 244

Research Database(s) 246

Entity Database(s) 248

# FIG. 2A

ML Component — 226

Agent module 1 — 227-1

Model 1 — 228-1

Model 2 — 228-2

⋮

Model L — 228-3

⋮

Node architecture — 230

Node 1 — 232-1

Node 1 parameter 1 — 234-1

⋮

Node 1 parameter K — 234-2

Node 1 logic — 236-1

Node 2 — 232-2

⋮

Node K — 232-3

Agent module 2 — 227-2

⋮

Agent module 3 — 227-3

⋮

Agent module N — 227-N

⋮

# FIG. 2B

Server System 106

Memory 310

| Operating System 312 |
| Network Communication Module 314 |

Assistant Module 316

| Model(s) 317 |
| Agent(s) 318 |
| Interface Module(s) 320 |
| ⋮ |

Server Data Modules 330

| Medical Database(s) 332 |
| Agent Database(s) 334 |
| User Database(s) 336 |
| Research Database(s) 338 |
| Entity Database(s) 340 |
| ⋮ |

⋮

CPU(s)
302

308

User
Interface(s)

304

Network
Interface(s)

306

**FIG. 3**

**System Database(s) 400**

**Subject/Clinical Dataset(s) 402**

**Genome**
- Somatic DNA
- Germline DNA
- cfDNA
- cfRNA

**Transcriptome**
- RNA

**Epigenome**
- DNA Methylation

**Microbiome**
- Virology
- Immunology

**Clinical**
- Curated
- EMR/EHR
- Uncurated

**Stored Alterations**
- Isoforms, SNPs, etc.

**Proteome**
- Protein Synthesis

**Omics**
- Metabolome
- Other 'Omes

**Organoids**
- Treatments

**Images**
- PDL1
- IHC
- Radiology

**Pathology**
- H&E | IHC

**Cardiology**
- ECG | EKG

**Neurology**
- EEG

Cohort Selection, Searching, Analytics, and Research

**Knowledge Database (KDB) 404**
- Provider Panels
- Provider Samples
- Drug Class
- Knowledge
- Gene
- Radiation
- Immuno Result
- Clinical Guidelines
- Drug
- Clinical Trials Questions - Answers
- Drug Class – Mutation Interactions
- Term Sheet
- Mutation – Drug Interactions
- Provider Coverage
- Provider Methods
- Clinical Trials DB
- Immuno General
- Clinical Conditions

**FIG. 4**

**FIG. 5A**

Raw Input Data 520

Tokenize 522

Segment 524

Rank 526

Trim 528

Datastore 530

# FIG. 5B

600

**102**
Client Device

Agent
Builder

Agent
Configs

Config updates

**106**
Server System

Config
Pubsub
Topic

**227**
Agent Module

**230**
Node(s)

**240**
Data Module

**228**
LLM Model

# FIG. 6

… the current cohort contains *N* patients with ctDNA samples. Would you like to reference applicable research opportunities in this cohort?

Yes. Compare my cohort to the ASCO 2025 conferences and identify potential overlapping research areas.

…… executing insight engine for ASCO 2025…..

96 abstracts in ASCO 2025 reference ctDNA as highlighted in the plot below:

| Cluster | Cluster Keywords |
|---|---|
| 49 | *free dna, specific, demonstrated, develop, liver, invasive, set, controls, classifier, plasma, liquid, individuals, blood, diagnostic, assay, healthy, early stage, ctdna, early detection, crc* |
| 52 | *biomarker, plasma, dna, detection, ngs, testing, mrd, ctdna* |
| 53 | *tumor dna, plasma, dynamics, her2 mbc, endocrine, monitoring, testing, detection, mrd, relapse, liquid, cdk4, hr her2, mbc* |

The research clusters most relevant to ctDNA research ….

# FIG. 7A

.. are listed:

| Cluster | Cluster Summary |
|---|---|
| 49 | The collection of abstracts centers on the development and validation of non-invasive, blood-based assays—primarily leveraging cell-free DNA (cfDNA) methylation and fragmentomics—for early cancer detection, risk stratification, and treatment response prediction across a range of solid tumors. These studies demonstrate …. |
| 52 | The abstracts collectively highlight the rapid evolution and clinical integration of circulating tumor DNA (ctDNA) and liquid biopsy technologies as prognostic, predictive, and monitoring tools across a wide spectrum of solid tumors, with a particular emphasis on lung cancer (NSCLC) and colorectal cancer. These studies demonstrate the utility of ctDNA for minimal residual disease (MRD) detection, early response assessment, recurrence prediction, and … |
| 53 | The collection of abstracts centers on the use of circulating tumor DNA (ctDNA) as a biomarker for prognosis, treatment response, and minimal residual disease (MRD) detection in various subtypes of breast cancer. Studies span early-stage, locally advanced, and metastatic breast cancer, with a strong emphasis on integrating ctDNA analysis into clinical decision-making, surveillance, and … |

Preliminary analysis shows 83% of the active cohort patients fullfill high-level Inclusion/Exclusion criteria for representative cohorts in cluster 53 (HER2+ BRCA patients with ctDNA sequencing).

Additional research highlights active existing clinical trials and therapies related to this research area:

- NCT04915755: Efficacy and Safety Comparison of Niraparib to Placebo in Participants With……

# FIG. 7B

800

| 802 Receive a user prompt from a user to generate a draft publication |

↓

| 804 Generate, via a task-specific orchestration, the draft publication based on the user prompt and research information |

↓

| 806 Provide the draft publication to the user for review |

# FIG. 8A

900

| 902 Receive, from a user, a user prompt indicating one or more subject attributes |

↓

| 904 Identify, via a task-specific orchestration, a patient cohort based on the one or more subject attributes |

↓

| 906 Provide an indication of the patient cohort to the user |

# FIG. 8B

1000

| 1002 Obtain research data from a set of one or more databases |

↓

| 1004 Identify, using a task-specific orchestration, a research gap by analyzing the research data |

↓

| 1006 Provide an indication of the research gap to a user |

# FIG. 8C

1100 —

| 1102 Receive a research hypothesis |

↓

| 1104 Identify of one or more patient cohorts based on the research hypothesis |

↓

| 1106 Identify statistically significant findings and generate one or more data visualizations by analyzing the one or more patient cohorts |

↓

| 1108 Identify related findings from peer-reviewed literature related to the research hypothesis |

↓

| 1110 Generate a publication draft based on the statistically significant findings and the related findings |

# FIG. 8D

1200

1202 Obtain, at the machine-learning component, a constraint indicating a period of time and a type of subject matter

1204 Obtain, using the constraint, a set of documents and corresponding metadata

1206 Generate a set of numerical embeddings for the set of documents based on information contained within the set of documents and the corresponding metadata

1208 Generate a set of updated similarity clusters by updating the set of similarity clusters to incorporate the set of numerical embeddings

1210 Obtain, for an entity, entity information comprising a set of one or more workflows and one or more datasets

1212 Identify a set of potential actions based on links between the entity information and the set of updated similarity clusters

1214 Generate a ranked set of potential actions by ranking the set of potential actions according to one or more criteria

1216 Provide a notification to the entity indicating a set of top-ranked potential actions from the ranked set of potential actions

# FIG. 8E

t-SNE Visualization of Abstracts by Cluster

**FIG. 9A**

FIG. 9B

Europäisches
Patentamt

European
Patent Office

Office européen
des brevets

# EUROPEAN SEARCH REPORT

**Application Number**

EP 25 21 8816

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2023/059742 A1 (NEUMORA THERAPEUTICS INC [US]) 13 April 2023 (2023-04-13) * paragraph [0002] - paragraph [0012] * ----- | 1-15 | INV. G06F16/906 |
| | | | **TECHNICAL FIELDS SEARCHED (IPC)** |
| | | | G06F G16H |

The present search report has been drawn up for all claims

| Place of search | Date of completion of the search | Examiner |
|---|---|---|
| Munich | 30 March 2026 | Hauck, Rainer |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

    ...................................................................................
& : member of the same patent family, corresponding document

EPO FORM 1503 03.82 (P04C01)

# EP 4 752 754 A1

## ANNEX TO THE EUROPEAN SEARCH REPORT
## ON EUROPEAN PATENT APPLICATION NO.

EP 25 21 8816

This annex lists the patent family members relating to the patent documents cited in the above-mentioned European search report.
The members are as contained in the European Patent Office EDP file on
The European Patent Office is in no way liable for these particulars which are merely given for the purpose of information.

30-03-2026

| Patent document cited in search report | | Publication date | Patent family member(s) | | Publication date |
|---|---|---|---|---|---|
| WO 2023059742 | A1 | 13-04-2023 | EP | 4413498 A1 | 14-08-2024 |
| | | | EP | 4413499 A1 | 14-08-2024 |
| | | | EP | 4413500 A1 | 14-08-2024 |
| | | | US | 2023104158 A1 | 06-04-2023 |
| | | | US | 2023105659 A1 | 06-04-2023 |
| | | | US | 2023107415 A1 | 06-04-2023 |
| | | | US | 2023109108 A1 | 06-04-2023 |
| | | | US | 2023260634 A1 | 17-08-2023 |
| | | | US | 2023343446 A1 | 26-10-2023 |
| | | | US | 2024021298 A1 | 18-01-2024 |
| | | | US | 2024136058 A1 | 25-04-2024 |
| | | | WO | 2023059742 A1 | 13-04-2023 |
| | | | WO | 2023059743 A1 | 13-04-2023 |
| | | | WO | 2023059744 A1 | 13-04-2023 |
| | | | WO | 2023059746 A1 | 13-04-2023 |

EPO FORM P0459

For more details about this annex : see Official Journal of the European Patent Office, No. 12/82

**EP 4 752 754 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63725998

**Non-patent literature cited in the description**

- **MENENDEZ et al.** The Jensen-Shannon divergence. *Journal of the Franklin Institute*, 1997, vol. 334 (2), 307-314
- Imagenet classification with deep convolutional neural networks. **KRIZHEVSKY et al.** Advances in Neural Information Processing Systems. Curran Associates, Inc., 2012, vol. 2, 1097-1105
- **ZEILER**. ADADELTA: an adaptive learning rate method. *CoRR*, 2012, vol. abs/1212.5701
- Neurocomputing: Foundations of research. **RUMEL-HART et al.** Learning Representations by Back-propagating Errors. MIT Press, 1988, 696-699
- **VINCENT et al.** Stacked denoising autoencoders: Learning useful representations in a deep network with a local denoising criterion. *J Mach Learn Res*, 2010, vol. 11, 3371-3408
- **LAROCHELLE et al.** Exploring strategies for training deep neural networks. *J Mach Learn Res*, 2009, vol. 10, 1-40
- **HASSOUN**. *Fundamentals of Artificial Neural Networks*, 1995
- **DUDA et al.** Pattern Classification. John Wiley & Sons, Inc., 2001
- **HASTIE et al.** The Elements of Statistical Learning. Springer-Verlag, 2001
- **DRAGHICI**. Data Analysis Tools for DNA Micro-arrays. Chapman & Hall/CRC, 2003
- **MOUNT**. Bioinformatics: sequence and genome analysis. Cold Spring Harbor Laboratory Press, 2001
- **CRISTIANINI** ; **SHAWE-TAYLOR**. An Introduction to Support Vector Machines. Cambridge University Press, 2000
- A training algorithm for optimal margin classifiers. **BOSER et al.** Proceedings of the 5th Annual ACM Workshop on Computational Learning Theory. ACM Press, 1992, 142-152

- **VAPNIK**. Statistical Learning Theory. Wiley, 1998
- **DUDA**. Pattern Classification. John Wiley & Sons, Inc., 2001, 259, 262-265
- **HASTIE**. The Elements of Statistical Learning. Springer, 2001
- **FUREY et al.** *Bioinformatics*, 2000, vol. 16, 906-914
- **NG et al.** On discriminative vs. generative classifiers: A comparison of logistic regression and naive Bayes. *Advances in Neural Information Processing Systems*, 2002, vol. 14
- **HASTIE et al.** The elements of statistical learning : data mining, inference, and prediction. Springer, 2001
- **DUDA**. Pattern Classification. John Wiley & Sons, Inc, 2001
- **DUDA et al.** Pattern Classification. John Wiley & Sons, 2001
- **DUDA**. Pattern Classification. John Wiley & Sons, Inc., 2001, 395-396
- **DUDA**. Pattern Classification. John Wiley & Sons, Inc., 2001, 396-408, 411-412
- **BREIMAN**. Random Forests--Random Features. *Technical Report*, September 1999, vol. 567
- **AGRESTI**. An Introduction to Categorical Data Analysis. John Wiley & Son, 1996, 103-144
- **MCLACHLAN et al.** *Bioinformatics*, 2002, vol. 18 (3), 413-422
- **SCHLIEP et al.** *Bioinformatics*, 2003, vol. 19 (1), 255-263
- **DUDA** ; **HART**. Pattern Classification and Scene Analysis. John Wiley & Sons, Inc., 1973
- **SUTTON RS** ; **BARTO AG**. Reinforcement learning: an introduction. *IEEE Transactions on Neural Networks.*, 1998, vol. 9 (5), 1054-1054